(19) Europäisches Patentamt
European Patent Office
Office européen des brevets

(11) EP 2 634 174 B1

(12) EUROPEAN PATENT SPECIFICATION

(45) Date of publication and mention of the grant of the patent:
**17.05.2017 Bulletin 2017/20**

(21) Application number: **13157132.5**

(22) Date of filing: **28.02.2013**

(51) Int Cl.:
***C07D 213/74*** (2006.01) ***C07D 401/06*** (2006.01)
***C07D 405/06*** (2006.01) ***C07D 417/06*** (2006.01)
***A01N 43/40*** (2006.01)

(54) **Nitrogen-containing heterocyclic derivate having 2-imino group and pest control agent including the same**

Stickstoffhaltiges heterocyclisches Derivat mit 2-Imino-Gruppe sowie Schädlingsbekämpfungsmittel damit

Dérivé hétérocyclique contenant de l'azote ayant un groupe 2-imino et agent antiparasitaire comprenant celui-ci

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.02.2012 JP 2012043743**

(43) Date of publication of application:
**04.09.2013 Bulletin 2013/36**

(73) Proprietor: **Meiji Seika Pharma Co., Ltd.**
**Tokyo 104-8002 (JP)**

(72) Inventors:
- **Kagabu, Shinzo**
  **Japan, Tokyo 501-1193 (JP)**
- **Mitomi, Masaaki**
  **Kanagawa, Kanagawa 222-8567 (JP)**
- **Kitsuda, Shigeki**
  **Kanagawa, Kanagawa 222-8567 (JP)**
- **Horikoshi, Ryo**
  **Kanagawa, Kanagawa 222-8567 (JP)**
- **Onozaki, Yasumichi**
  **Kanagawa, Kanagawa 222-8567 (JP)**
- **Nakamura, Satoshi**
  **Kanagawa, Kanagawa 222-8567 (JP)**

(74) Representative: **Cohausz & Florack**
**Patent- & Rechtsanwälte**
**Partnerschaftsgesellschaft mbB**
**Bleichstraße 14**
**40211 Düsseldorf (DE)**

(56) References cited:
**EP-A1- 0 639 569**
**EP-A1- 1 820 504**
**EP-A2- 0 268 915**
**EP-A2- 0 432 600**
**WO-A1-2010/019547**
**WO-A1-2012/029672**
**WO-A1-2013/031671**
**GB-A- 1 571 826**
**JP-A- H0 578 323**
**JP-A- S52 113 970**
**US-A- 3 694 451**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 634 174 B1

## Description

BACKGROUND OF THE INVENTION

1. Field of the Invention

The present invention relates to a nitrogen-containing heterocyclic derivative having a 2-imino group, and a novel pest control agent using the same.

2. Description of Related Art

Although numerous pest control agents have been discovered so far, novel drugs are still required in view of the problem of reduction in drug sensitivity, long-term efficacy, safety during the use thereof and the like.
In particular, for rice cultivation in East Asia and Southeast Asia, damage by planthoppers that have developed drug resistance against main insecticides including Neonicotinoids represented by imidacloprid is materializing and a specific medicine against planthoppers that have developed drug resistance is expected.
European Patent Application Laid-Open No. 432600 discloses a plurality of compounds having the same ring structure as a compound represented by Formula (I), but the compounds are used as herbicides and there is no description about pest control.
European Patent Application Laid-Open No. 268915 discloses the structural formula of N-[1-((6-chloropyridin-3-yl)methyl)pyridin-2(1H)-ylide ne]-2,2,2-trifluoroacetamide, but there is no description about the pest control by compounds having other structures.
Japanese Patent Application Laid-Open No. 5-78323 discloses a compound similar to the compound represented by Formula (I), but the compound has a structure different from the compound of the present invention, in that R in Formula (I) is an acyl group having a benzene ring, an acyl group having a hetero ring, an alkylsulfonyl group, an alkylaminocarbonyl group and an alkylaminothiocarbonyl group. The biological activity thereof is an insecticidal activity, but the compound of the present invention does not suggest significantly high activity against a wide range of insect species.
International Publication No. 2006/051704 discloses a compound similar to Formula (I) of the present invention, but fails to specifically disclose the compound represented by Formula (I) and does not suggest the content of the present invention about pest control activity.
European Patent Application Laid-Open No. 259738 discloses a plurality of compounds having a ring structure similar to that of a compound represented by Formula (I), but fails to disclose or suggest a compound having a trifluoroacetic acid imino structure.

SUMMARY OF THE INVENTION

The present invention is contrived to provide a novel pest control agent to solve problems which chemicals in the related art have, such as reduction in drug sensitivity, long-term efficacy, safety during the use thereof and the like in the field of pest control.
One of the important problems in the present invention is to provide a chemical which has excellent pest control effects against Nilaparvata lugens, Sogatella furcifera and Laodelphax striatella, which has recently become major pests in the field of rice, exhibits high activity even against drug-resistant planthoppers, reduces the chance that workers are exposed to the chemical during the use thereof such as soil treatment, seed treatment, nursery box treatment and may be safely used.
In order to solve the problems, the present inventors have intensively studied, and as a result, have found that a nitrogen-containing heterocyclic derivative having a 2-imino group, which is represented by Chemical Formula (I), has excellent activity as a pest control agent.
That is, according to the present invention, provided is a pest-control agent containing a nitrogen-containing heterocyclic derivative having a 2-imino group, which is represented by the following Formula (I), or salts thereof

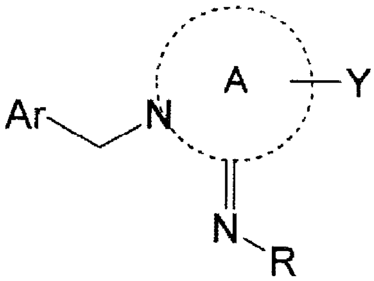

Formula (I),

in the formula Ar represents a phenyl group which may be substituted with any of a halogen atom, a C1 to C4 alkyl group which may be substituted with a halogen atom, a C1 to C4 alkyloxy group which may be substituted with a halogen atom, a hydroxyl group, a cyano group, a nitro group; a 5- to 6-membered heterocycle which may be substituted with any of a halogen atom, a C1 to C4 alkyl group which may be substituted with a halogen atom, a C1 to C4 alkyloxy group which may be substituted with a halogen atom, a hydroxyl group, a cyano group, and a nitro group; or a 4- to 10-membered heterocycloalkyl group,
the ring represented by the following Formula:

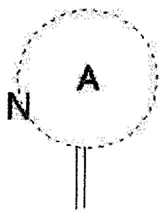

represents any one of rings represented by the following Formulae (A-1) to (A-40):

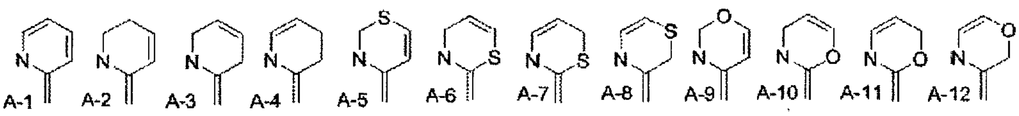

A-1 A-2 A-3 A-4 A-5 A-6 A-7 A-8 A-9 A-10 A-11 A-12

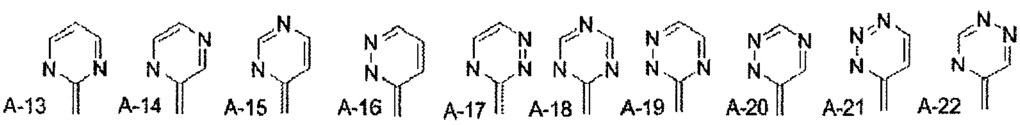

A-13 A-14 A-15 A-16 A-17 A-18 A-19 A-20 A-21 A-22

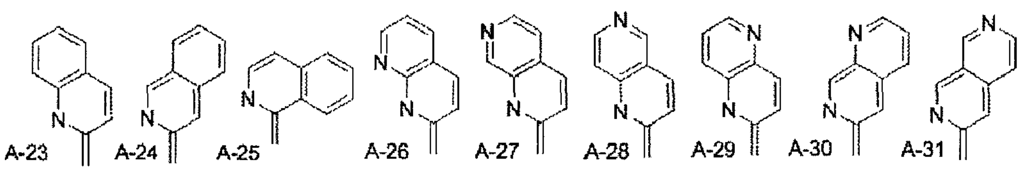

A-23 A-24 A-25 A-26 A-27 A-28 A-29 A-30 A-31

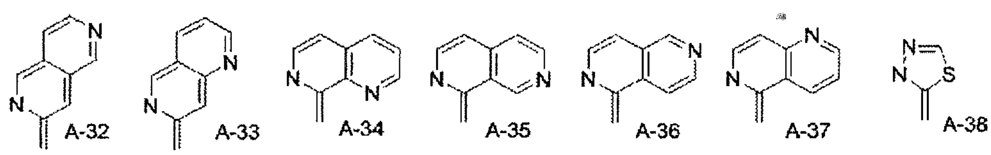

A-32 A-33 A-34 A-35 A-36 A-37 A-38

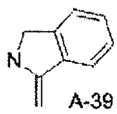 A-39 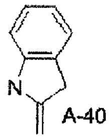 A-40,

Y represents a hydrogen atom, a halogen atom, a hydroxyl group, a C1 to C6 alkyl group which may be substituted with a halogen atom, a C1 to C6 alkyloxy group which may be substituted with a halogen atom, a cyano group, or a nitro group, and
R represents a group represented by the following Formula (c)

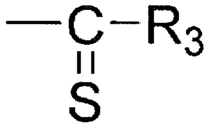 (c)

R3 represents a C1 to C6 alkyl group which may be substituted with a halogen atom.

In addition, the compound indicated in tables 1 and 2 below are not included.

Table 1-1

| 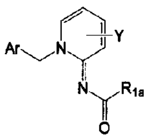 | | | | | |
|---|---|---|---|---|---|
| Compound No. | Ar | R1a | Y | $^1$H-NMR (CDCl3, δ, ppm) | IR (KBr, ν, $cm^{-1}$) or MS |
| P212 | 6-chloro-3-pyridyl | CF3 | H | 5.57 (2H, s), 6.92 (1H, td), 7.31 (1H, d), 7.80 (1H, td), 7.87 (1H, dd), 7.99 (1H, dd), 8.48 (2H, m) | m/z = 316 (M+H) |
| P213 | 2-chloro-5-thiazolyl | CF3 | H | 5.61 (2H, s), 6.93 (1H, dd), 7.68(1H, s), 7.83 (1H, td), 7.97 (1H, d), 8.53 (1H, d) | m/z = 322 (M+H) |
| P214 | 6-chloro-3-pyridyl | OCH3 | H | 3.74 (3H, s), 5.40 (2H, s), 6.45 (1H, td), 7.29 (1H, d), 7.46 (2H, m), 7.73 (1H, dd), 8.12 (1H, dd), 8.40 (1H, d) | m/z = 278 (M+H) |
| P215 | 6-chloro-3-pyridyl | CF3 | 5-Cl | 5.53 (2H, s), 7.34 (1H, d), 7.71 (1H, dd), 7.87 (1H, dd), 7.94 (1H, s), 8.49 (1H, d), 8.55 (1H, s) | m/z = 350 (M+H) |
| P216 | 6-chloro-3-pyridyl | CF3 | 5-F | 5.54 (2H, s), 7.34 (1H, d) , 7.70 (1H, m) , 7.80 (1H, m), 7.88 (1H, dd), 8.48 (1H, d), 8.64 (1H, m) | m/z = 334 (M+H) |
| P217 | 6-chloro-3-pyridyl | CF3 | 4-Cl | 5.49 (2H, s), 6.85 (1H, dd), 7.35 (1H, d), 7.76 (1H, dd), 7.85 (1H, dd), 8.44 (1H, d), 8.62 (1H, s) | m/z = 350 (M+H) |
| P218 | 2-chloro-5-thiazolyl | CF3 | 5-Cl | 5.56 (2H, s), 7.68 (1H, s), 7.74 (1H, dd), 7.84 (1H, d), 8.58 (1H, d) | m/z = 356 (M+H) |

Table 1-2

| | | | | | |
|---|---|---|---|---|---|
| P219 | 2-chloro-5-thiazolyl | CF3 | 5-F | 5.60 (2H, s), 7.69 (1H, s), 7.72 (1H, td), 7.86 (1H, m), 8.67 (1H, m) | m/z = 340 (M+H) |

(continued)

| | | | | | |
|---|---|---|---|---|---|
| P220 | 2-chloro-5-thiazolyl | CF3 | 4-Cl | 5.58 (2H, s), 6.90 (1H, d), 7.67 (1H, s), 7.90 (1H, d), 8.61 (1H, s) | m/z = 356 (M+H) |
| P221 | 6-chloro-3-pyridyl | CF3 | 3-Me | 2.31 (3H, s), 5.50 (2H, s), 6.98 (1H, m), 7.34 (1H, d), 7.73 (1H, dd), 7.77 (2H, m), 8.42 (1H, d) | m/z = 330 (M+H) |
| P222 | 6-chloro-3-pyridyl | CF3 | 4-Me | 2.40 (3H, S), 5.49 (2H, s), 6.70 (1H, dd), 7.32 (1H, d), 7.70 (1H, d), 7.86 (1H, dd), 8.37 (1H, s), 8.43 (1H, d) | m/z = 330 (M+H) |
| P223 | 6-chloro-3-pyridyl | CF3 | 5-Me | 2.29 (3H, s), 5.52 (2H, s), 7.32 (1H, d), 7.62 (1H, s), 7.65 (1H, dd), 7.88 (1H, dd), 8.46 (1H, d), 8.50 (1H, d) | m/z = 330 (M+H) |
| P224 | phenyl | CF3 | H | 5.58 (2H, s), 6.81 (1H, m), 7.37 (4H, m), 7.77 (2H, m), 8.50 (1H, d) | m/z = 281 (M+H) |
| P225 | 4-chlorophe nyl | CF3 | H | 5.52 (2H, s), 6.85 (1H, m), 7.30 (2H, d), 7.36 (2H, d), 7.75 (1H, td), 7.84 (1H, d), 8.47 (1H, d) | m/z = 315 (M+H) |
| P226 | 3-pyridyl | CF3 | H | 5.57 (2H, s), 6.86 (1H, m), 7.26-7.35 (2H, m), 7.78 (1H, td), 7.86 (1H, m), 8.63 (2H, m), 8.67 (1H, d) | m/z = 282 (M+H) |
| P227 | 6-chloro-5-fluoro-3-pyridyl | CF3 | H | 5.54 (2H, s), 6.89 (1H, td), 7.76 (1H, dd), 7.80 (1H, td), 7.85 (1H, d), 8.29 (1H, d), 8.57 (1H, d) | m/z = 334 (M+H) |

Table 1-3

| | | | | | |
|---|---|---|---|---|---|
| P228 | 6-trifluoro methyl-3-pyridyl | CF3 | H | 5.62 (2H, s), 6.90 (1H, t), 7.69 (1H, d), 7.81 (1H, t), 7.88 (1H, d), 8.06 (1H, d), 8.56 (1H, d), 8.78 (1H, s) | m/z = 350 (M+H) |
| P229 | 6-fluoro-3-pyridyl | CF3 | H | 5.56 (2H, s), 6.89 (1H, td), 6.94 (1H, d), 7.79 (1H, td), 7.87 (1H, d), 8.03 (1H, m), 8.31 (1H, s), 8.54 (1H, d) | m/z = 300 (M+H) |
| P230 | 5,6-dichlor o-3-pyridyl | CF3 | H | 5.49 (2H, s), 6.89 (1H, t), 7.79-7.90 (2H, m) , 8.04 (1H, d) , 8.37 (1H, d), 8.56 (1H, m) | m/z = 350 (M+H) |

Table 2-1

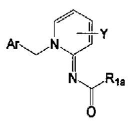

| Compound No. | Ar | R1a | Y | $^1$H-NMR (CDCl3, $\delta$, ppm) | IR (KBr, v, cm$^{-1}$) or MS |
|---|---|---|---|---|---|
| P231 | 6-bromo-3-pyridyl | CF3 | H | 5.52 (2H, s), 6.88 (1H, t), 7.48 (1H, 7.78 (2H, m), 7.84 (1H, d), 8.44 (1H, d), 8.53 (1H, d) | m/z = 360 (M+H) |
| P232 | 6-chloro-3 -pyridyl | CF3 | 4-F | 5.52 (2H, s), 6.71 (1H, m), 7.35 (1H, d), 7.86 (1H, dd), 7.94 (1H, m), 8.33 (1H, dd), 8.44 (1H, d) | m/z = 334 (M+H) |
| P233 | 6-chloro-3 -pyridyl | CF3 | 3-F | 5.53 (2H, s), 6.74 (1H, m), 7.33 (1H, d), 7.87 (1H, dd), 8.07 (1H, m), 8.29 (1H, dd), 8.45 (1H, d) | m/z = 334 (M+H) |
| P234 | 6-chloro-3 -pyridyl | CHCl 2 | H | 5.54 (2H, s), 6.02 (1H, s), 6.77 (1H, t), 7.32 (1H, m), 7.69 (1H, m), 7.77 (1H, d), 7.89 (1H, m), 8.42 (1H, m), 8.49 (1H, s) | m/z = 330 (M+H) |

(continued)

| Compound No. | Ar | R1a | Y | $^1$H-NMR (CDCl3, δ, ppm) | IR (KBr, ν, cm$^{-1}$) or MS |
|---|---|---|---|---|---|
| P235 | 6-chloro-3 -pyridyl | CCl3 | H | 5.59 (2H, s), 6.86 (1H, t), 7.32 (1H, 7.78 (1H, td), 7.91 (2H, m), 8.43 (1H, d), 8.50 (1H, d) | m/z = 364 (M+H) |
| P236 | 6-chloro-3 -pyridyl | CH2C l | H | 4.17 (2H, s), 5.46 (2H, s), 6.64 (1H, td), 7.31 (1H, d), 7.60 (1H, td), 7.64 (1H, dd), 7.80 (1H, dd), 8.32 (1H, d), 8.45 (1H, d) | m/z = 296 (M+H) |

[Table 2-2]

| | | | | | |
|---|---|---|---|---|---|
| P238 | 6-chloro-3 -pyridyl | CHF2 | H | 5.52 (2H, s), 5.90 (1H, t), 6.79 (1H, td), 7.33 (1H, d), 7.71 (1H, m), 7.77 (1H, dd), 7.85 (1H, dd), 8.45 (1H, d), 8.50 (1H, d) | m/z = 298 (M+H) |
| P239 | 6-chloro-3 -pyridyl | CF2C l | H | 5.56 (2H, s), 6.92 (1H, t), 7.33 (1H, d), 7.82 (1H, m), 7.91 (1H, dd), 8.02 (1H, d), 8.45 (1H, d), 8.48 (1H, d) | m/z = 332 (M+H) |
| P240 | 6-chloro-3 -pyridyl | CHCl Br | H | 5.53 (1H, d), 5.58 (1H, d), 6.06 (1H, s), 6.76 (1H, td), 7.32 (1H, d), 7.69 (1H, m), 7.70 (1H, m), 7.90 (1H, dd), 8.40 (1H, d), 8.50 (1H, d) | m/z = 374 (M+H) |
| P241 | 6-chloro-3 -pyridyl | CHBr 2 | H | 5.56 (2H, s), 5.99 (1H, s), 6.78 (1H, td), 7.33 (1H, d), 7.69 (1H, td), 7.76 (1H, dd), 7.93 (1H, dd), 8.39 (1H, d), 8.50 (1H, d) | m/z = 418 (M+H) |
| P242 | 6-chloro-3 -pyridyl | CF2C F 3 | H | 5.56 (2H, s), 6.90 (1H, td), 7.32 (1H, d), 7.79 (2H, m), 7.84 (1H, d), 8.43 (1H, d) , 8.56 (1H, d) | m/z = 366 (M+H) |
| P243 | 2-chloro-5 -pyrimidin yl | CF3 | H | 5.54 (2H, s), 6.98 (1H, m), 7.87 (1H, m), 8.18 (1H, m), 8.48 (1H, m), 8.83 (2H, m) | m/z = 317 (M+H) |
| P244 | 6-chloro-3 -pyridyl | CH2B r | H | 4.17 (2H, s), 5.46 (2H, s), 6.63 (1H, td), 7.31 (1H, d), 7.60 (1H, td), 7.65 (1H, dd), 7.80 (1H, dd), 8.32 (1H, d), 8.47 (1H, d) | |

Preferred embodiments of the invention are listed below: (2) a nitrogen-containing heterocyclic derivative or a salt thereof having a 2-imino group represented by Formula (I) in (1), wherein Ar in Formula (I) is a 6-chloro-3-pyridyl group, a 6-chloro-5-fluoro-3-pyridyl group, a 6-fluoro-3-pyridyl group, a 6-bromo-3-pyridyl group, a 2-chloro-5-pyrimidinyl group, a 2-chloro-5-thiazolyl group, or a 5-chloro-2-pyradinyl group,

(3) a nitrogen-containing heterocyclic derivative or a salt thereof having a 2-imino group indicated in either (1) or (2) in the above, wherein A in the formula (I) is equivalent to formula (A-1) in (1), Y being a hydrogen atom, a halogen atom, or a cyano group,
(4) a nitrogen-containing heterocyclic derivative or a salt thereof having a 2-imino group indicated in (1) in the above, the 2-imino group being N-[1-((6-chloropyridin-3-yl)methyl)pyridin-2(1H)-ylide ne]-2,2,2-trifluoroethanthioamide,
(5) a pest control agent including a nitrogen-containing heterocyclic derivative having a 2-imino group, which is described in any one of (1) to (4) or salts thereof,
(6) a non-medical method for controlling pests, using a nitrogen-containing heterocyclic derivative having a 2-imino group, which is described in any one of (1) to (4) or salts thereof, or a pest control agent described in (5),
(7) a method for controlling pests, including: treating seeds, roots, tubers, bulbs and rhizomes of plants, soil, a nutrient solution in nutrient solution culture, a solid medium in nutrient solution culture or a simple body that grows plants with a nitrogen-containing heterocyclic derivative having a 2-imino group, which is described in any one of (1) to (4) or salts thereof, or a pest control agent described in (5) to penetrate and migrate the compound into the plants,
(8) a method described in (6) or (7), in which the pest is an agricultural and horticultural pest,
(9) a method described in (6), in which the pest is an animal parasitic pest,
(10) a method described in (6) to (9), in which the pest is a drug-resistant pest,
(11) a method for preparing a compound represented by Formula (I-3) [in the formula, Ar, A, Y and R3 have the

same meaning as those defined as Formula (I) in (1)],

[Chemical Formula 9]

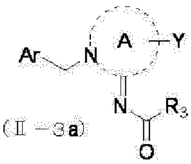

(II-3a)

in which a reaction of converting an oxygen atom in the compound represented by Formula (II-3a) [in the formula, Ar, A, Y and R3 have the same meaning as those defined as Formula (I) in (1)] or

[Chemical Formula 10]

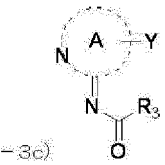

(II-3c)

Formula (II-3c) [in the formula, A, Y and R3 have the same meaning as those defined as Formula (I) in (1)]

[Chemical Formula 11]

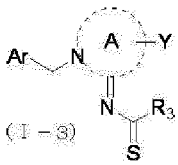

(I-3)

into a sulfur atom is performed, and

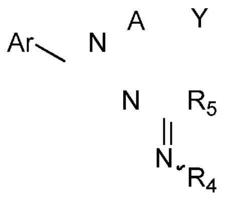

(I-4),

wherein a compound represented by Formula (II-4a):

[Chemical Formula 13]

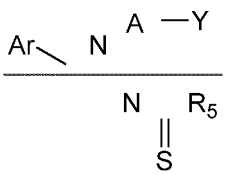

It is possible to effectively perform pest control against cabbage moths, Spodoptera litura, aphids, planthoppers,

leafhoppers, thrips and other numerous pests by using the nitrogen-containing heterocyclic derivative having a 2-imino group of the present invention.

DETAILED DESCRIPTION OF EMBODIMENTS

In a nitrogen-containing heterocyclic derivative having a 2-imino group denoted in the formula (I) provided in the present invention, examples of a substituent that may be substituted with "a phenyl group which may be substituted" and "a 5- to 6-membered heterocycle which may be substituted", which are represented by Ar, include a halogen atom, a C1 to C4 alkyl group which may be substituted with a halogen atom, a C1 to C4 alkyloxy group which may be substituted with a halogen atom, a hydroxyl group, a cyano group, a nitro group and the like, preferably a halogen atom, a trifluoromethyl group and a cyano group, and particularly preferably a halogen atom.

Specific examples of the "a phenyl group which may be substituted" represented by Ar of a nitrogen-containing heterocyclic derivative compound having a 2-imino group represented by Formula (I) include a phenyl group and a 3-cyano phenyl group.

"A 5- to 6-membered heterocycle which may be substituted", represented by Ar of a nitrogen-containing heterocyclic derivative compound having a 2-imino group represented by Formula (I) represents an aromatic 5- to 6-membered heterocycle including one or two of a heteroatom such as an oxygen atom, a sulfur atom or a nitrogen atom, specific examples thereof include a pyridine ring, a pyrazine ring, a pyrimidine ring, a pyridazine ring, a thiazole ring, an oxazole ring and the like, and preferable aspects thereof include a 6-chloro-3-pyridyl group, a 6-chloro-5-fluoro-3-pyridyl group, a 6-bromo-3-pyridyl group, a 6-fluoro-3-pyridyl group, a 6-trifluoromethyl-3-pyridyl group, a 6-chloro-3-pyridazinyl group, a 5-chloro-2-pyrazinyl group, a 2-chloro-5-pyrimidinyl group, a 2-chloro-5-thiazolyl group, a 2-chloro-4-pyridyl group, and more preferably a 6-chloro-3-pyridyl group, a 6-fluoro-3-pyridyl group, a 6-chloro-5-fluoro-3-pyridyl group, a 6-bromo-3-pyridyl group and a 2-chloro-5-pyrimidinyl group.

Specific examples of "a 4- to 10-membered heterocycloalkyl group" represented by Ar of a nitrogen-containing hetero ring derivative having a 2-imino group represented by Formula (I) include a 2-tetrahydrofuranyl group, a 3-tetrahydrofuranyl group and the like and preferably a 3-tetrahydrofuranyl group.

"A heterocycle having a 5- to 10-membered unsaturated bond including one or more nitrogen atoms", which A of a nitrogen-containing heterocyclic derivative having a 2-imino group, which is represented by Formula (I), represents, means

[Chemical Formula 14]

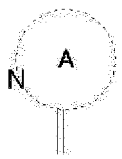

in Formula (I), but represents any one ring represented by the following Formulas A-1 to A-40. In each formula, the end of a double bond is the substitution position of a nitrogen atom.

[Chemical Formula 15]

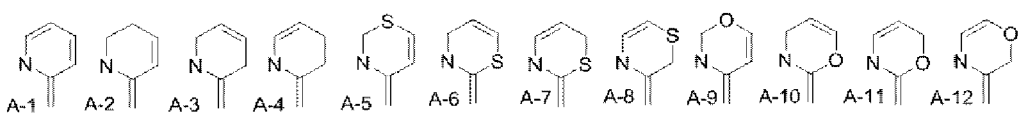

A-1 A-2 A-3 A-4 A-5 A-6 A-7 A-8 A-9 A-10 A-11 A-12

[Chemical Formula 16]

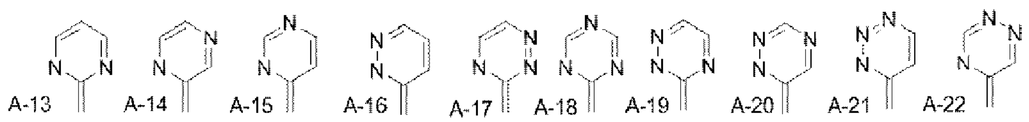

A-13 A-14 A-15 A-16 A-17 A-18 A-19 A-20 A-21 A-22

[Chemical Formula 17]

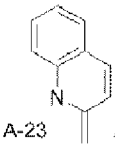 A-23 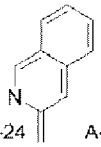 A-24 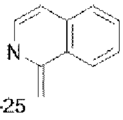 A-25 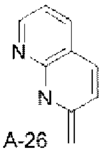 A-26 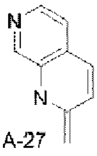 A-27 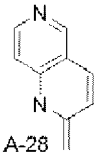 A-28 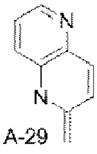 A-29 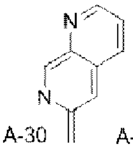 A-30 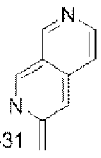 A-31

[Chemical Formula 18]

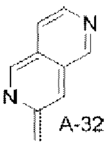 A-32 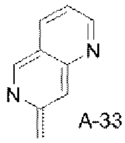 A-33 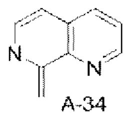 A-34 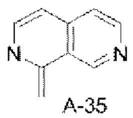 A-35 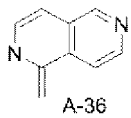 A-36 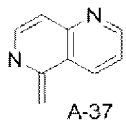 A-37 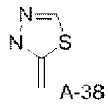 A-38

[Chemical Formula 19]

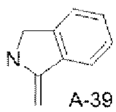 A-39 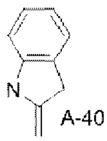 A-40

The ring is preferably the ring of Formulas A-1, A-13, A-14, A-15, A-16, A-23, A-25, A-38 and A-39 and more preferably the ring of Formula A-1.

"A C1 to C6 alkyl group which may be substituted with a halogen atom", which Y of the nitrogen-containing heterocyclic derivative having a 2-imino group, which is represented by Formula (I), represents, is a C1 to C6 alkyl group, which is chained, branched, cyclic or combination thereof, and the upper limit of the number of halogen atoms which may be substituted is the number of hydrogen atoms which the alkyl group has. When a branched or cyclic alkyl group is included, it is obvious that the number of carbons is 3 or more.

Specific examples of "a C1 to C6 alkyloxy group which may be substituted with a halogen atom" which Y represents include a methoxy group, an ethoxy group, a trifluoromethyloxy group and a difluoromethyloxy group.

A preferred aspect of Y is preferably a hydrogen atom or a halogen atom and more preferably a hydrogen atom.

In Formula (I), when R represents a Formula (a) group, "a substituted C1 to C6 alkyl group" which R1 represents is an alkyl group having 1 to 6 carbon atoms, which is chained, branched, cyclic or combination thereof, and the upper limit of the number of substituted substituents is the number of hydrogen atoms which the alkyl group has. Examples of the substituent include a halogen atom, a hydroxyl group, a cyano group, a nitro group, a phenyl group (this phenyl group may be substituted with a C1 to C4 alkyl group which may be substituted with a halogen, a C1 to C4 alkyloxy group which may be substituted with a halogen, a hydroxyl group, or a halogen atom), a phenoxy group (this phenoxy group may be substituted with a C1 to C4 alkyl group which may be substituted with a halogen, a C1 to C4 alkyloxy group which may be substituted with a halogen, a hydroxyl group, or a halogen atom), a benzyloxy group (the phenyl group in this benzyloxy group may be substituted with a C1 to C4 alkyl group which may be substituted with a halogen, a C1 to C4 alkyloxy group which may be substituted with a halogen, a hydroxyl group, or a halogen atom), and the like. Specific examples of the substituent include a trifluoromethyl group, a trichloromethyl group, a difluorochloromethyl group, a difluoromethyl group, a dichloromethyl group, a dibromomethyl group, a chloromethyl group, a difluoroethyl group, a dichloroethyl group, a 2,2,2-trifluoroethyl group, a pentafluoroethyl group, a difluorocyclopropyl group, a 2-cyanoethyl group, and a 2-nitroethyl group, and the like. Preferably, the C1-C6 alkyl group is a 2,2,2-trifluoroethyl group, a trifluoromethyl group, a difluorochloromethyl group, a difluoromethyl group, and a pentafluoroethyl group, and more preferably, the substituent is a trifluoromethyl group, a difluorochloromethyl group, a difluoromethyl group, and a pentafluoroethyl group, and particularly, a trifluoromethyl group.

In Formula (I), "a C1 to C6 alkyl group which may be substituted with a halogen atom", which R3 when R represents a Formula (c) group is an alkyl group having 1 to 6 carbon atoms, which is chained, branched, cyclic or combination thereof, and the upper limit of the number of substituted halogen atoms is the number of hydrogen atoms which the alkyl group has. When a branched or cyclic alkyl group is included, it is obvious that the number of carbons is 3 or more. Specific examples of the C1-C6 alkyl group include a methyl group, an ethyl group, an n-propyl group, an

isopropyl group, an n-butyl group, a t-butyl group, a cyclopropyl group, a cyclopentyl group, a cyclohexyl group, a trifluoromethyl group, a trichloromethyl group, a difluorochloromethyl group, a difluoromethyl group, a dichloromethyl group, a dibromomethyl group, a chloromethyl group, a difluoroethyl group, a dichloroethyl group, a 2,2,2-trifluoroethyl group, a pentafluoroethyl group, a difluorocyclopropyl group, a trifluoroisopropyl group, and a hexafluoroisopropyl group, and the like.

R3 is, preferably, an ethyl group, an isopropyl group, a cyclopropyl group, a trifluoromethyl group, a difluorochloromethyl group, a difluoromethyl group, and a pentafluoroethyl group, more preferably, a trifluoromethyl group, a difluorochloromethyl group, a difluoromethyl group, and a pentafluoroethyl group, and particularly preferably, a trifluoromethyl group. R5 is, preferably, a trifluoromethyl group, a trichloromethyl group, a dichloromethyl group, a difluoromethyl group, a difluorochloromethyl group, a chloromethyl group, and a pentafluoroethyl group, more preferably, a trifluoromethyl group, a difluoromethyl group, a difluorochloromethylgroup, apentafluoroethylgroup, and particularly preferably, a trifluoromethyl group. R7 is, preferably, a trifluoromethyl group, a trichloromethyl group, a dichloromethyl group, a difluoromethyl group, a difluorochloromethyl group, a chloromethyl group, and a pentafluoroethyl group, more preferably, a trifluoromethyl group, a difluoromethyl group, a difluorochloromethylgroup, and a pentafluoroethyl group, and particularly preferably, a trifluoromethyl group.

In Formula (I), when R represents a Formula (b) group, "a C1 to C6 alkyl group which is substituted with a halogen atom" which R2 represents is an alkyl group having 1 to 6 carbon atoms, which is chained, branched, cyclic or combination thereof, and the upper limit of the number of substituted halogen atoms is the number of hydrogen atoms which the alkyl group has. When a branched or cyclic alkyl group is included, it is obvious that the number of carbons is 3 or more. Specific examples of the C1-C6 alkyl group includes a trifluoromethyl group, a trichloromethyl group, a difluorochloromethyl group, a difluoromethyl group, a dichloromethyl group, a dibromomethyl group, a chloromethyl group, a difluoroethyl group, a dichloroethyl group, a 2,2,2-trifluoroethyl group, a 1-(trifluoromethyl)ethyl group, a 1-trifluoromethyl-2,2,2-trifluoroethyl group, a pentafluoroethyl group, and a difluorocyclopropyl group, and the like, and preferably, the alkyl group is a 2,2,2-trifluoroethyl group, a 1-(trifluoromethyl)ethyl group, a 1-trifluoromethyl-2,2,2-trifluoroethyl group, a pentafluoroethyl group, and a difluorocyclopropyl group, and the like, and preferably, the alkyl group is a 2,2,2-trifluoroethyl group, a 1-(trifluoromethyl)ethyl group, and a 1-trifluoromethyl-2,2,2-trifluoroethyl group.

"A C2 to C6 alkenyl group which may be substituted with a halogen atom", which R1, R2, R3, represents, is an alkenyl group having 2 to 6 carbon atoms, which is chained, branched, cyclic or combination thereof, and the upper limit of the number of substituted halogen atoms is the number of hydrogen atoms which the alkenyl group has. When a branched or cyclic alkenyl group is included, it is obvious that the number of carbons is 3 or more. Specifically, the C2-C6 alkenyl group is an ethenyl group, a 1-propenyl group, a 2-propenyl group, a 2-fluoro-1-propenyl group, a 2-methyl-1-propenyl group, and the like. R6 is, preferably, a 2-propenyl group, and R6a is, preferably, an ethenyl group.

"A C2 to C6 alkynyl group which may be substituted with a halogen atom", which R1, R2, R3 represents, is an alkynyl group having 2 to 6 carbon atoms, which is chained, branched or combination thereof, and the upper limit of the number of substituted halogen atoms is the number of hydrogen atoms which the alkynyl group has. When a branched or cyclic alkynyl group is included, it is obvious that the number of carbons is 3 or more. Specific examples thereof include a 1-propynyl group, a 2-propynyl group, a 1-butynyl group, a 2-butynyl group, a 1-pentynyl group, a 2-pentynyl group, a 3-pentynyl group and the like, and preferred examples thereof include a 1-propynyl group, a 2-propynyl group and a 2-butynyl group.

The (C6 to C10) aryl of "a substituted or unsubstituted (C6 to C10) aryl group, a substituted or unsubstituted (C6 to C10) aryl (C1 to C6) alkyl group, a substituted or unsubstituted (C6 to C10) aryl (C2 to C6) alkenyl group and a substituted or unsubstituted (C6 to C10) aryl (C2 to C6) alkynyl group", which R2, R3 represent, specifically represents a phenyl group and a naphthyl group, and the (C1 to C6) alkyl group, the (C2 to C6) alkenyl group and the (C2 to C6) alkynyl group may have a straight chain, branch or ring. Examples of the substituent which may be substituted with an aryl group include a halogen atom, a C1 to C4 alkyl group which may be substituted with halogen, a C1 to C4 alkyloxy group which may be substituted with halogen, a C3 to C6 cyclic alkyl group, a methylsulfonyl group, a methoxy group, a nitro group, a cyano group and the like. Specific examples of the (C6-C10) aryl include a phenyl group, a benzyl group, a 2-phenylethyl group, a 2-phenylethenyl group, a 2-phenylethynyl group, a 4-methylphenyl group, a 2-cyanophenyl group, a 3-chlorophenyl group, a 4-methoxyphenyl group, and a 3-cyanophenyl group, a 1,1-diphenylmethyl group, a naphthylethyl group, a naphthylpropyl group, and the like, and preferably, a benzyl group, a 2-phenylethylgroup, a naphthylethyl group, and a naphthylpropynyl group.

The (C1 to C6) alkyl group, (C2 to C6) alkenyl group and (C2 to C6) alkynyl group of "a substituted or unsubstituted phenoxy group (C1 to C6) alkyl group, a substituted or unsubstituted phenoxy group (C2 to C6) alkenyl group and a substituted or unsubstituted phenoxy group (C2 to C6) alkynyl group", which R3 represents, may have a straight chain, branch or ring. Examples of the substituent which may be substituted with a phenoxy group include a halogen atom, a C1 to C4 alkyl group which may be substituted with halogen, a C1 to C4 alkyloxy group which may be substituted with halogen, a C3 to C6 cyclic alkyl group, a methylsulfonyl group, a methoxy group, a nitro group, a cyano group and the like. Specific examples of the (C1 to C6) alkyl group, a (C2 to C6) alkenyl group, and the (C2 to C6) alkynyl group include

a phenoxy group, a phenoxymethyl group, a 2-phenoxyethyl group, a 2-phenoxyethenyl group, a 2-phenoxyethynyl group, a 4-chlorophenoxy group, and a 2-methylphenoxy group, and the like, and preferably, a 2-phenoxyethyl group.

The 5- to 10-membered heterocycle of "a substituted or unsubstituted 5- to 10-membered heterocycle, a substituted or unsubstituted 5- to 10-membered heterocycle (C1 to C6) alkyl group, a substituted or unsubstituted 5- to 10-membered heterocycle (C2 to C6) alkenyl group and a substituted or unsubstituted 5- to 10-membered heterocycle (C2 to C6) alkynyl group", which R2, R3 represent, represents a ring including one to four heteroatoms such as an oxygen atom, a sulfur atom or a nitrogen atom and the like as an atom constituting the ring, and examples thereof include a furanyl group, a thienyl group, a pyridyl group, a pyrrolidinyl group, a piperidinyl group, a piperazinyl group, a pyrimidinyl group, a morpholinyl group, a thiazolyl group, an imidazolyl group, a triazolyl group, a tetrahydrofuranyl group, a quinolinyl group and the like. Examples of the substituent which may be substituted with a heterocycle include a halogen atom, a C1 to C4 alkyl group which may be substituted with halogen, a C1 to C4 alkyloxy group which may be substituted with halogen, a C3 to C6 cyclic alkyl group, a methylsulfonyl group, a methoxy group, a nitro group, a cyano group and the like. The (C1 to C6) alkyl group, (C2 to C6) alkenyl group and (C2 to C6) alkenyl group may have a straight chain, branch or ring. Specifically, the 5- to 10-membered heterocycle is a 2-pyridyl group, a 3-pyridyl group, a 4-pyridyl group, a 2-pyridylmethyl group, a 3-pyridylmethyl group, a 4-pyridylmethyl group, a 2-(4-pyridyl)ethenyl group, a 2-(4-pyridyl)ethynyl group, a 2-furanylmethyl group, a 2-thienylmethyl group, a 2-tetrahydrofuranylmethyl group, and the like, and R4 is, preferably, a 2-pyridylmethyl group, a 3-pyridylmethyl group, a 4-pyridylmethyl group, a 2-furanylmethyl group, a 2-thienylmethyl group, and a 2-tetrahydrofuranylmethyl group.

The (C1 to C4) alkoxy of "a (C1 to C4) alkoxy (C1 to C5) alkyl group, a (C1 to C4) alkoxy (C2 to C5) alkenyl group and a (C1 to C4) alkoxy (C2 to C5) alkynyl group", which R3 represents, represents a (C1 to C4) alkyloxy, alkenyloxy and alkynyloxy having a straight chain, branch or ring. Specific examples of the (C1 to C4) alkoxy includes a methoxymethyl group, a 2-methoxyethyl group, an ethoxymethyl group, a 2-ethoxyethyl group, a 3-methoxy-2-propenyl group, and a 3-methoxy-2-propynyl group, and the like, and R4 is, preferably, a 2-methoxyethyl group.

The (C1 to C4) alkylthio of "a (C1 to C4) alkylthio (C1 to C5) alkyl group, a (C1 to C4) alkylthio (C2 to C5) alkenyl group and a (C1 to C4) alkylthio (C2 to C5) alkynyl group", which R3 represents, represents a (C1 to C4) alkylthio, alkenylthio and alkynylthio having a straight chain, branch or ring. The (C1 to C4) alkylthio includes a methylthiomethyl group, a 2-methylthioethyl group, an ethylthiomethyl group, a 2-ethylthioethyl group, a 3-methylthio-2-propenyl group, and a 3-methylthio-2-propynyl group, and the like, and R4 is, preferably, a 2-methylthioethyl group.

In a compound represented by Formula (I),
R represents the following Formula (c) group

[Chemical Formula 21]

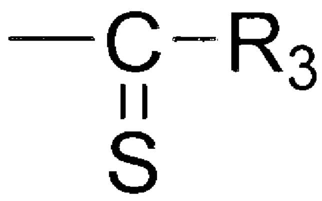

(c)

Ar represents a 6-chloro-3-pyridyl group, a 2-chloro-5-thiazolyl group, a 6-chloro-5-fluoro-3-pyridyl group, a 6-fluoro-3-pyridyl group, a 6-bromo-3-pyridyl group, a 2-chloro-5-pyrimidyl group and a 6-trifluoromethyl-3-pyridyl group,
A represents a ring represented by A-1,
Y represents a hydrogen atom, and
R3 represents a trifluoromethyl group, a difluoromethyl group, a chlorodifluoromethyl group and a pentafluoroethyl group.

Specific examples of the compound represented by Formula (I) include compounds shown in the following Table A (Tables 3 to 21) and Table B (Tables 22 to 37). Compounds of the inventon in Table A are those in which R represents a combination of substituents corresponding to rows no. 20 to 40, 648 and 649 below of Table B.

[Table 3-1]

| Table A | | | | | |
|---|---|---|---|---|---|
| | Compound No. | Ar | A | Y | R |
| Table 1 | 1-5~1-710 | 6-Chloro-3-pyridyl | A-1 | H | represents a combination of substituents corresponding to each row of Nos. (1 and 6) below of Table B |
| Table 2 | 2-1~2-710 | 2-Chloro-5-thiazolyl | A-1 | H | represents a combination of substituents corresponding to each row of Table B |
| Table 3 | 3-2~3-710 | 6-Fluoro-3-pyridyl | A-1 | H | represents a combination of substituents corresponding to each row of Nos. (1 and 3) below of Table B |
| Table 4 | 4-2~4-710 | 6-Bromo-3-pyridyl | A-1 | H | represents a combination of substituents corresponding to each row of Nos. (1 and 3) below of Table B |
| Table 5 | 5-2~5-710 | 6-Chloro-5-fluoro-3-pyridyl | A-1 | H | represents a combination of substituents corresponding to each row of Nos. (1 and 3) below of Table B |
| Table 6 | 6-2~6-710 | 2-Chloro-5-pyrimidinyl | A-1 | H | represents a combination of substituents corresponding to each row of Nos. (1 and 3) below of Table B |

[Table 3-2]

| | | | | | |
|---|---|---|---|---|---|
| Table 7 | 7-1~7-7 10 | 5-Chloropyra in-2-yl | A-1 | H | represents a combination of substituents corresponding to each row of Table B |
| Table 8 | 8-1~8-7 10 | 6-Chloropyridazin-3-yl | A-1 | H | represents a combination of substituents corresponding to each row of Table B |
| Table 9 | 9-1 ~9-710 | 2-Chloro-5-oxazolyl | A-1 | H | represents a combination of substituents corresponding to each row of Table B |
| Table 10 | 10-1~10 -710 | 6-trifluoromethyl-3-pyridyl | A-1 | H | represents a combination of substituents corresponding to each row of Table B |
| Table 11 | 11-1~11 -710 | 3-tetrahydrofuranyl | A-1 | H | represents a combination of substituents corresponding to each row of Table B |
| Table 12 | 12-1~12 -710 | 2-Chloro-4-pyridyl | A-1 | H | represents a combination of substituents corresponding to each row of Table B |
| Table 13 | 13-1~13 -710 | 3-Cyanophenyl | A-1 | H | represents a combination of substituents corresponding to each row of Table B |
| Table 14 | 14-1~14 -710 | 6-Chloro-3-pyridyl | A-1 | 3 F | represents a combination of substituents corresponding to each row of Table B |
| Table 15 | 15-1~15 -710 | 2-Chloro-5-thiazolyl | A-1 | 3 F | represents a combination of substituents corresponding to each row of Table B |

[Table 3-3]

| | | | | | |
|---|---|---|---|---|---|
| Table 16 | 16-1~16 -710 | 6-Fluoro-3-pyridyl | A-1 | 3 F | represents a combination of substituents corresponding to each row of Table B |
| Table 17 | 17-1~17 -710 | 6-Bromo-3-pyridyl | A-1 | 3 F | represents a combination of substituents corresponding to each row of Table B |

(continued)

| | | | | | |
|---|---|---|---|---|---|
| Table 18 | 18-1~18 -710 | 6-Chloro-5-fluoro-3-pyridyl | A-1 | 3 F | represents a combination of substituents corresponding to each row of Table B |
| Table 19 | 19-1~19 -710 | 2-Chloro-5-pyrimidinyl | A-1 | 3 F | represents a combination of substituents corresponding to each row of Table B |
| Table 20 | 20-1~20 -710 | 5-Chloropyrazin-2-yl | A-1 | 3 F | represents a combination of substituents corresponding to each row of Table B |
| Table 21 | 21-1~21 -710 | 6-Chloropyridazin-3-yl | A-1 | 3 F | represents a combination of substituents corresponding to each row of Table B |
| Table 22 | 22-1~22 -710 | 2-Chloro-5-oxazolyl | A-1 | 3 F | represents a combination of substituents corresponding to each row of Table B |
| Table 23 | 23-1~23 -710 | 6-trifluorome thyl-3-pyridyl | A-1 | 3 F | represents a combination of substituents corresponding to each row of Table B |
| Table 24 | 24-1~24 -710 | 3-tetrahydrofuranyl | A-1 | 3 F | represents a combination of substituents corresponding to each row of Table B |

[Table 3-4]

| | | | | | |
|---|---|---|---|---|---|
| Table 25 | 25-1~25 -710 | 6-Chloro-3-pyridyl | A-1 | 4 F | represents a combination of substituents corresponding to each row of Table B |
| Table 26 | 26-1~26 -710 | 2-Chloro-5-thiazolyl | A-1 | 4 F | represents a combination of substituents corresponding to each row of Table B |
| Table 27 | 27-1~27 -710 | 6-Fluoro-3-pyridyl | A-1 | 4 F | represents a combination of substituents corresponding to each row of Table B |
| Table 28 | 28-1~28 -710 | 6-Bromo-3-pyridyl | A-1 | 4 F | represents a combination of substituents corresponding to each row of Table B |
| Table 29 | 29-1~29 -710 | 6-Chloro-5-fluoro-3-pyridyl | A-1 | 4 F | represents a combination of substituents corresponding to each row of Table B |
| Table 30 | 30-1~30 -710 | 2-Chloro-5-pyrimidinyl | A-1 | 4 F | represents a combination of substituents corresponding to each row of Table B |
| Table 31 | 31-1~31 -710 | 5-Chloropyrazin-2-yl | A-1 | 4 F | represents a combination of substituents corresponding to each row of Table B |
| Table 32 | 32-1~32 -710 | 6-Chloropyridazin-3-yl | A-1 | 4 - F | represents a combination of substituents corresponding to each row of Table B |

[Table 4-1]

| Table A | | | | | |
|---|---|---|---|---|---|
| | Compound No. | Ar | A | Y | R |
| Table 33 | 33-1~33-710 | 2-Chloro-5-oxazolyl | A - 1 | 4-F | represents a combination of substituents corresponding to each row of Table B |
| Table 34 | 34-1~34-710 | 6-trifluoromethyl-3-pyridyl | A - 1 | 4-F | represents a combination of substituents corresponding to each row of Table B |

(continued)

| Table A | | | | | |
|---|---|---|---|---|---|
| | Compound No. | Ar | A | Y | R |
| Table 35 | 35-1~35-710 | 3-tetrahydrofuranyl | A - 1 | 4-F | represents a combination of substituents corresponding to each row of Table B |
| Table 36 | 36-1~36-710 | 6-Chloro-3-pyridyl | A - 1 | 5-F | represents a combination of substituents corresponding to each row of Table B |

[Table 4-2]

| | | | | | |
|---|---|---|---|---|---|
| Table 37 | 37-1~37-710 | 2-Chloro-5-thiazolyl | A 1 | 5-F | represents a combination of substituents corresponding to each row of Table B |
| Table 38 | 38-1~38-710 | 6-Fluoro-3-pyridyl | A - 1 | 5-F | represents a combination of substituents corresponding to each row of Table B |
| Table 39 | 39-1~39-710 | 6-Bromo-3-pyridyl | A - 1 | 5-F | represents a combination of substituents corresponding to each row of Table B |
| Table 40 | 40-1~40-710 | 6-Chloro-5-fluoro-3-pyridyl | A - 1 | 5-F | represents a combination of substituents corresponding to each row of Table B |

[Table 4-3]

| | | | | | |
|---|---|---|---|---|---|
| Table 41 | 41-1~41-710 | 2-Chloro-5-pyrimidinyl | A 1 | 5-F | represents a combination of substituents corresponding to each row of Table B |
| Table 42 | 42-1~42-710 | 5-Chloropyrazin-2-yl | A 1 | 5-F | represents a combination of substituents corresponding to each row of Table B |
| Table 43 | 43-1~43-710 | 6-Chloropyridazin-3-yl | A 1 | 5-F | represents a combination of substituents corresponding to each row of Table B |
| Table 44 | 44-1~44-710 | 2-Chloro-5-oxazolyl | A 1 | 5-F | represents a combination of substituents corresponding to each row of Table B |

[Table 4-4]

| | | | | | |
|---|---|---|---|---|---|
| Table 45 | 45-1~45-710 | 6-trifluoromethyl-3-pyridyl | A 1 | 5-F | represents a combination of substituents corresponding to each row of Table B |
| Table 46 | 46-1~46-710 | 3-tetrahydrofuranyl | A - 1 | 5-F | represents a combination of substituents corresponding to each row of Table B |
| Table 47 | 47-1~47-710 | 6-Chloro-3-pyridyl | A - 1 | 6-F | represents a combination of substituents corresponding to each row of Table B |
| Table 48 | 48-1~48-710 | 2-Chloro-5-thiazolyl | A - 1 | 6-F | represents a combination of substituents corresponding to each row of Table B |

[Table 4-5]

| | | | | | |
|---|---|---|---|---|---|
| Table 49 | 49-1~49-710 | 6-Fluoro-3-pyridyl | A 1 | 6-F | represents a combination of substituents corresponding to each row of Table B |
| Table 50 | 50-1~50-710 | 6-Bromo-3-pyridyl | A-1 | 6-F | represents a combination of substituents corresponding to each row of Table B |
| Tabl e 51 | 51-1~51-710 | 6-Chloro-5-fluoro-3-pyridyl | A-1 | 6-F | represents a combination of substituents corresponding to each row of Table B |
| Table 52 | 52-1~52-710 | 2-Chloro-5-pyrimidinyl | A-1 | 6-F | represents a combination of substituents corresponding to each row of Table B |

[Table 4-6]

| | | | | | |
|---|---|---|---|---|---|
| Table 53 | 53-1~53-710 | 5-Chloropyrazin-2-yl | A-1 | 6-F | represents a combination of substituents corresponding to each row of Table B |
| Table 54 | 54-1~54-710 | 6-Chloropyridazin-3-yl | A-1 | 6-F | represents a combination of substituents corresponding to each row of Table B |
| Table 55 | 55-1~55-710 | 2-Chloro-5-oxazolyl | A-1 | 6-F | represents a combination of substituents corresponding to each row of Table B |
| Table 56 | 56-1~56-710 | 6-trifluoromethyl-3-pyridyl | A-1 | 6-F | represents a combination of substituents corresponding to each row of Table B |

[Table 4-7]

| | | | | | |
|---|---|---|---|---|---|
| Table 57 | 57-1~57-710 | 3-tetrahydrofuranyl | A-1 | 6-F | represents a combination of substituents corresponding to each row of Table B |
| Table 58 | 58-1~58-710 | 6-Chloro-3-pyridyl | A-1 | 3-Cl | represents a combination of substituents corresponding to each row of Table B |
| Table 59 | 59-1~59-710 | 2-Chloro-5-thiazolyl | A-1 | 3-Cl | represents a combination of substituents corresponding to each row of Table B |
| Table 60 | 60-1~60-710 | 6-Fluoro-3-pyridyl | A-1 | 3-Cl | represents a combination of substituents corresponding to each row of Table B |

[Table 4-8]

| | | | | | |
|---|---|---|---|---|---|
| Table 61 | 61-1~61-710 | 6-Bromo-3-pyridyl | A 1 | 3-Cl | represents a combination of substituents corresponding to each row of Table B |
| Table 62 | 62-1~62-710 | 6-Chloro-5-fluoro-3-pyridyl | A 1 | 3-Cl | represents a combination of substituents corresponding to each row of Table B |

(continued)

| | | | | | |
|---|---|---|---|---|---|
| Table 63 | 63-1~63-642 | 2-Chloro-5-pyrimidinyl | A-1 | 3-Cl | represents a combination of substituents corresponding to each row of Table B |
| Table 64 | 64-1~64-710 | 5-Chloropyrazin-2-yl | A-1 | 3-Cl | represents a combination of substituents corresponding to each row of Table B |

[Table 5-1]

| Table A | | | | | |
|---|---|---|---|---|---|
| | Compound No. | Ar | A | Y | R |
| Table 65 | 65-1~65 -710 | 6-Chloropyridazin-3-yl | A 1 | 3-Cl | represents a combination of substituents corresponding to each row of Table B |
| Table 66 | 66-1~66 -710 | 2-Chloro-5-oxazolyl | A 1 | 3-Cl | represents a combination of substituents corresponding to each row of Table B |
| Table 67 | 67-1~67 -710 | 6-trifluoromethyl-3-pyridyl | A 1 | 3-Cl | represents a combination of substituents corresponding to each row of Table B |
| Table 68 | 68-1~68 -710 | 3-tetrahydrofuranyl | A 1 | 3-Cl | represents a combination of substituents corresponding to each row of Table B |
| Table 69 | 69-1~69 -710 | 6-Chloro-3-pyridyl | A 1 | 4-Cl | represents a combination of substituents corresponding to each row of Table B |
| Table 70 | 70-1~70 -710 | 2-Chloro-5-thiazolyl | A 1 | 4-Cl | represents a combination of substituents corresponding to each row of Table B |
| Table 71 | 71-1~71 -710 | 6-Fluoro-3-pyridyl | A 1 | 4-Cl | represents a combination of substituents corresponding to each row of Table B |

[Table 5-2]

| | | | | | |
|---|---|---|---|---|---|
| Table 72 | 72-1~72 -710 | 6-Bromo-3-pyridyl | A 1 | 4-Cl | represents a combination of substituents corresponding to each row of Table B |
| Table 73 | 73-1~73 -710 | 6-Chloro-5-fu oro-3-pyridyl | A 1 | 4-Cl | represents a combination of substituents corresponding to each row of Table B |
| Table 74 | 74-1~74 -710 | 2-Chloro-5-pyr imidinyl | A 1 | 4-Cl | represents a combination of substituents corresponding to each row of Table B |
| Table 75 | 75-1~75 -710 | 5-Chloropyrazin-2-yl | A 1 | 4-Cl | represents a combination of substituents corresponding to each row of Table B |
| Table 76 | 76-1~76 -710 | 6-Chloropyridazin-3-yl | A 1 | 4-Cl | represents a combination of substituents corresponding to each row of Table B |
| Table 77 | 77-1~77 -710 | 2-Chloro-5-oxazolyl | A 1 | 4-Cl | represents a combination of substituents corresponding to each row of Table B |
| Table 78 | 78-1~78 -710 | 6-trifluoromethyl-3-pyridyl | A 1 | 4-Cl | represents a combination of substituents corresponding to each row of Table B |
| Table 79 | 79-1~79 -710 | 3-tetrahydrofuranyl | A 1 | 4-Cl | represents a combination of substituents corresponding to each row of Table B |
| Table 80 | 80-1~80 -710 | 6-Chloro-3-pyridyl | A 1 | 5-Cl | represents a combination of substituents corresponding to each row of Table B |

[Table 5-3]

| | | | | | |
|---|---|---|---|---|---|
| Table 81 | 81-1~81 -710 | 2-Chloro-5-thiazolyl | A 1 | 5-Cl | represents a combination of substituents corresponding to each row of Table B |
| Table 82 | 82-1~82 -710 | 6-Fluoro-3-pyridyl | A 1 | 5-Cl | represents a combination of substituents corresponding to each row of Table B |
| Table 83 | 83-1~83 -710 | 6-Bromo-3-pyridyl | A 1 | 5-Cl | represents a combination of substituents corresponding to each row of Table B |
| Table 84 | 84-1~84 -710 | 6-Chloro-5-fluoro-3-pyridyl | A 1 | 5-Cl | represents a combination of substituents corresponding to each row of Table B |
| Table 85 | 85-1~85 -710 | 2-Chloro-5-pyrimidinyl | A 1 | 5-Cl | represents a combination of substituents corresponding to each row of Table B |
| Table 86 | 86-1~86 -710 | 5-Chloropyrazin-2-yl | A 1 | 5-Cl | represents a combination of substituents corresponding to each row of Table B |
| Table 87 | 87-1~87 -710 | 6-Chloropyridazin-3-yl | A 1 | 5-Cl | represents a combination of substituents corresponding to each row of Table B |
| Table 88 | 88-1~88 -710 | 2-Chloro-5-oxazolyl | A 1 | 5-Cl | represents a combination of substituents corresponding to each row of Table B |
| Table 89 | 89-1 ~89-710 | 6-trifluoromethyl-3-pyridyl | A 1 | 5-Cl | represents a combination of substituents corresponding to each row of Table B |

[Table 5-4]

| | | | | | |
|---|---|---|---|---|---|
| Table 90 | 90-1~90 -710 | 3-tetrahydrofuranyl | A 1 | 5-Cl | represents a combination of substituents corresponding to each row of Table B |
| Table 91 | 91-1~91 -710 | 6-Chloro-3-pyridyl | A 1 | 6-Cl | represents a combination of substituents corresponding to each row of Table B |
| Table 92 | 92-1~92 -710 | 2-Chloro-5-thiazolyl | A 1 | 6-Cl | represents a combination of substituents corresponding to each row of Table B |
| Table 93 | 93-1~93 -710 | 6-Fluoro-3-pyridyl | A 1 | 6-Cl | represents a combination of substituents corresponding to each row of Table B |
| Table 94 | 94-1~94 -710 | 6-Bromo-3-pyridyl | A 1 | 6-Cl | represents a combination of substituents corresponding to each row of Table B |
| Table 95 | 95-1~95 -710 | 6-Chloro-5-fluoro-3-pyridyl | A 1 | 6-Cl | represents a combination of substituents corresponding to each row of Table B |
| Table 96 | 96-1~96 -710 | 2-Chloro-5-pyrimidinyl | A 1 | 6-Cl | represents a combination of substituents corresponding to each row of Table B |

[Table 6-1]

| Table A | | | | | |
|---|---|---|---|---|---|
| | Compound No. | Ar | A | Y | R |
| Table 97 | 97-1~97 -710 | 5-Chloropyrazin-2-yl | A-1 | 6-Cl | represents a combination of substituents corresponding to each row of Table B |
| Table 98 | 98-1~98 -710 | 6-Chloropyridazin-3-yl | A-1 | 6-Cl | represents a combination of substituents corresponding to each row of Table B |

(continued)

| Table A | | | | | |
|---|---|---|---|---|---|
| | Compound No. | Ar | A | Y | R |
| Table 99 | 99-1~99 -710 | 2-Chloro-5-oxazolyl | A-1 | 6-Cl | represents a combination of substituents corresponding to each row of Table B |
| Table 100 | 100-1~1 00-710 | 6-trifluoromethyl-3-pyridyl | A-1 | 6-Cl | represents a combination of substituents corresponding to each row of Table B |
| Table 101 | 101-1~1 01-710 | 3-tetrahydrofuranyl | A-1 | 6-Cl | represents a combination of substituents corresponding to each row of Table B |
| Table 102 | 102-1~1 02-710 | 6-Chloro-3-pyridyl | A-1 | 3-CN | represents a combination of substituents corresponding to each row of Table B |
| Table 103 | 103-1~1 03-710 | 2-Chloro-5-thiazolyl | A-1 | 3-CN | represents a combination of substituents corresponding to each row of Table B |
| Table 104 | 104-1~1 04-710 | 6-Fluoro-3-pyridyl | A-1 | 3-CN | represents a combination of substituents corresponding to each row of Table B |

[Table 6-2]

| | | | | | |
|---|---|---|---|---|---|
| Table 105 | 105-1~1 05-710 | 6-Bromo-3-pyridyl | A-1 | 3-CN | represents a combination of substituents corresponding to each row of Table B |
| Table 106 | 106-1~1 06-710 | 6-Chloro-5-fluoro-3-pyridyl | A-1 | 3-CN | represents a combination of substituents corresponding to each row of Table B |
| Table 107 | 107-1~1 07-710 | 2-Chloro-5-pyrimidinyl | A-1 | 3-CN | represents a combination of substituents corresponding to each row of Table B |
| Table 108 | 108-1~1 08-710 | 5-Chloropyrazin-2-yl | A-1 | 3-CN | represents a combination of substituents corresponding to each row of Table B |
| Table 109 | 109-1~1 09-710 | 6-Chloropyridazin-3-yl | A-1 | 3-CN | represents a combination of substituents corresponding to each row of Table B |
| Table 110 | 110-1~1 10-710 | 2-Chloro-5-oxazolyl | A-1 | 3-CN | represents a combination of substituents corresponding to each row of Table B |
| Table 111 | 111-1~1 11-710 | 6-trifluoromethyl-3-pyridyl | A-1 | 3-CN | represents a combination of substituents corresponding to each row of Table B |
| Table 112 | 112-1~1 12-710 | 3-tetrahydrofuranyl | A-1 | 3-CN | represents a combination of substituents corresponding to each row of Table B |
| Table 113 | 113-1~1 13-710 | 6-Chloro-3-pyridyl | A-1 | 4-CN | represents a combination of substituents corresponding to each row of Table B |

[Table 6-3]

| | | | | | |
|---|---|---|---|---|---|
| Table 114 | 114-1~1 14-710 | 2-Chloro-5-thiazolyl | A-1 | 4-CN | represents a combination of substituents corresponding to each row of Table B |

(continued)

| | | | | | |
|---|---|---|---|---|---|
| Table 115 | 115-1~1 15-710 | 6-Fluoro-3-pyridyl | A-1 | 4-CN | represents a combination of substituents corresponding to each row of Table B |
| Table 116 | 116-1~1 16-710 | 6-Bromo-3-pyridyl | A-1 | 4-CN | represents a combination of substituents corresponding to each row of Table B |
| Table 117 | 117-1~1 17-710 | 6-Chloro-5-Fluoro-3-pyridyl | A-1 | 4-CN | represents a combination of substituents corresponding to each row of Table B |
| Table 118 | 118-1~1 18-710 | 2-Chloro-5-pyrimidinyl | A-1 | 4-CN | represents a combination of substituents corresponding to each row of Table B |
| Table 119 | 119-1~1 19-710 | 5-Chloropyrazin-2-yl | A-1 | 4-CN | represents a combination of substituents corresponding to each row of Table B |
| Table 120 | 120-1~1 20-710 | 6-Chloropyridazin-3-yl | A-1 | 4-CN | represents a combination of substituents corresponding to each row of Table B |
| Table 121 | 121-1~1 21-710 | 2-Chloro-5-oxazolyl | A-1 | 4-CN | represents a combination of substituents corresponding to each row of Table B |
| Table 122 | 122-1~1 22-710 | 6-trifluoromethyl-3-pyridyl | A-1 | 4-CN | represents a combination of substituents corresponding to each row of Table B |

[Table 6-4]

| | | | | | |
|---|---|---|---|---|---|
| Table 123 | 123-1~1 23-710 | 3-tetrahydrofuranyl | A-1 | 4-CN | represents a combination of substituents corresponding to each row of Table B |
| Table 124 | 124-1~1 24-710 | 6-Chloro-3-pyridyl | A-1 | 5-CN | represents a combination of substituents corresponding to each row of Table B |
| Table 125 | 125-1~1 55-710 | 2-Chloro-5-thiazolyl | A-1 | 5-CN | represents a combination of substituents corresponding to each row of Table B |
| Table 126 | 126-1~1 26-710 | 6-Fluoro-3-pyridyl | A-1 | 5-CN | represents a combination of substituents corresponding to each row of Table B |
| Table 127 | 127-1~1 27-710 | 6-Bromo-3-pyridyl | A-1 | 5-CN | represents a combination of substituents corresponding to each row of Table B |
| Table 128 | 128-1~1 28-710 | 6-Chloro-5-fluoro-3-pyridyl | A-1 | 5-CN | represents a combination of substituents corresponding to each row of Table B |

[Table 7-1]

| Table A | | | | | |
|---|---|---|---|---|---|
| | Compound No. | Ar | A | Y | R |
| Table 129 | 129-1~1 29-710 | 2-Chloro-5-pyrimidinyl | A-1 | 5-CN | represents a combination of substituents corresponding to each row of Table B |
| Table 130 | 130-1~1 30-710 | 5-Chloropyrazin-2-yl | A-1 | 5-CN | represents a combination of substituents corresponding to each row of Table B |
| Table 131 | 131-1~1 31-710 | 6-Chloropyridazin-3-yl | A-1 | 5-CN | represents a combination of substituents corresponding to each row of Table B |

[Table 7-2]

| | | | | | |
|---|---|---|---|---|---|
| Table 132 | 132-1~132-710 | 2-Chloro-5-oxazolyl | A-1 | 5-CN | represents a combination of substituents corresponding to each row of Table B |
| Table 133 | 133-1~133-710 | 6-trifluoromethyl-3-pyridyl | A-1 | 5-CN | represents a combination of substituents corresponding to each row of Table B |
| Table 134 | 134-1~134-710 | 3-tetrahydrofuranyl | A-1 | 5-CN | represents a combination of substituents corresponding to each row of Table B |
| Table 135 | 135-1~135-710 | 6-Chloro-3-pyridyl | A-1 | 6-CN | represents a combination of substituents corresponding to each row of Table B |

[Table 7-3]

| | | | | | |
|---|---|---|---|---|---|
| Table 136 | 136-1~136-710 | 2-Chloro-5-thiazolyl | A-1 | 6-CN | represents a combination of substituents corresponding to each row of Table B |
| Table 137 | 111-1~137-710 | 6-Fluoro-3-pyridyl | A-1 | 6-CN | represents a combination of substituents corresponding to each row of Table B |
| Table 138 | 138-1~138-710 | 6-Bromo-3-pyridyl | A-1 | 6-CN | represents a combination of substituents corresponding to each row of Table B |
| Table 139 | 139-1~139-710 | 6-Chloro-5-fluoro-3-pyridy l | A-1 | 6-CN | represents a combination of substituents corresponding to each row of Table B |

[Table 7-4]

| | | | | | |
|---|---|---|---|---|---|
| Table 140 | 140-1 ~14071 0 | 2-Chloro-5-pyrimidinyl | A-1 | 6-CN | represents a combination of substituents corresponding to each row of Table B |
| Table 141 | 141-1~141-710 | 5-Chloropyrazin-2-yl | A-1 | 6-CN | represents a combination of substituents corresponding to each row of Table B |
| Table 142 | 142-1~142-710 | 6-Chloropyridazin-3-yl | A-1 | 6-CN | represents a combination of substituents corresponding to each row of Table B |
| Table 143 | 143-1~143-710 | 2-Chloro-5-oxazolyl | A-1 | 6-CN | represents a combination of substituents corresponding to each row of Table B |

[Table 7-5]

| | | | | | |
|---|---|---|---|---|---|
| Table 144 | 144-1~144-710 | 6-trifluoromethyl-3-pyridy l | A-1 | 6-CN | represents a combination of substituents corresponding to each row of Table B |
| Table 145 | 145-1~145-710 | 3-tetrahydrofuranyl | A-1 | 6-CN | represents a combination of substituents corresponding to each row of Table B |
| Table 146 | 146-1~146-710 | 6-Chloro-3-pyridyl | A-1 | 3-OH | represents a combination of substituents corresponding to each row of Table B |
| Table 147 | 147-1~147-710 | 2-Chloro-5-thiazolyl | A-1 | 3-OH | represents a combination of substituents corresponding to each row of Table B |

[Table 7-6]

| | | | | | |
|---|---|---|---|---|---|
| Table 148 | 148-1~148-710 | 6-Fluoro-3-pyridyl | A-1 | 3-OH | represents a combination of substituents corresponding to each row of Table B |

(continued)

| | | | | | |
|---|---|---|---|---|---|
| Table 149 | 149-1~1 49-710 | 6-Bromo-3-pyridyl | A-1 | 3-OH | represents a combination of substituents corresponding to each row of Table B |
| Table 150 | 150-1~1 50-710 | 6-Chloro-5-Fluoro-3-pyridyl | A-1 | 3-OH | represents a combination of substituents corresponding to each row of Table B |
| Table 151 | 151-1~1 51-710 | 2-Chloro-5-pyrimidinyl | A-1 | 3-OH | represents a combination of substituents corresponding to each row of Table B |

[Table 7-7]

| | | | | | |
|---|---|---|---|---|---|
| Table 152 | 152-1~1 52-710 | 5-Chloropyrazin-2-yl | A-1 | 3-OH | represents a combination of substituents corresponding to each row of Table B |
| Table 153 | 153-1~1 53-710 | 6-Chloropyridazin-3-yl | A-1 | 3-OH | represents a combination of substituents corresponding to each row of Table B |
| Table 154 | 154-1~1 54-710 | 2-Chloro-5-oxazolyl | A-1 | 3-OH | represents a combination of substituents corresponding to each row of Table B |
| Table 155 | 155-1~1 55-710 | 6-trifluoromethyl-3-pyridyl | A-1 | 3-OH | represents a combination of substituents corresponding to each row of Table B |

[Table 7-8]

| | | | | | |
|---|---|---|---|---|---|
| Table 156 | 156-1~1 56-710 | 3-tetrahydrofuranyl | A-1 | 3-OH | represents a combination of substituents corresponding to each row of Table B |
| Table 157 | 157-1~1 57-710 | 6-Chloro-3-pyridyl | A-1 | 4-OH | represents a combination of substituents corresponding to each row of Table B |
| Table 158 | 158-1~1 58-710 | 2-Chloro-5-thiazolyl | A-1 | 4-OH | represents a combination of substituents corresponding to each row of Table B |
| Table 159 | 159-1~1 59-710 | 6-Fluoro-3-pyridyl | A-1 | 4-OH | represents a combination of substituents corresponding to each row of Table B |

[Table 7-9]

| | | | | | |
|---|---|---|---|---|---|
| Table 160 | 111-1~1 60-710 | 6-Bromo-3-pyridyl | A-1 | 4-OH | represents a combination of substituents corresponding to each row of Table B |

[Table 8-1]

| Table A | | | | | |
|---|---|---|---|---|---|
| | Compound No. | Ar | A | Y | R |
| Table 161 | 161-1~1 61-710 | 6-Chloro-5-fluoro-3-pyridyl | A-1 | 4-OH | represents a combination of substituents corresponding to each row of Table B |

(continued)

| Table A | | | | | |
|---|---|---|---|---|---|
| | Compound No. | Ar | A | Y | R |
| Table 162 | 162-1~1 62-710 | 2-Chloro-5-pyrimidinyl | A-1 | 4-OH | represents a combination of substituents corresponding to each row of Table B |
| Table 163 | 163-1~1 63-710 | 5-Chloropyrazin-2-yl | A-1 | 4-OH | represents a combination of substituents corresponding to each row of Table B |
| Table 164 | 164-1~1 64-710 | 6-Chloropyridazin-3-yl | A-1 | 4-OH | represents a combination of substituents corresponding to each row of Table B |
| Table 165 | 165-1~1 65-710 | 2-Chloro-5-oxazolyl | A-1 | 4-OH | represents a combination of substituents corresponding to each row of Table B |
| Table 166 | 166-1~1 66-710 | 6-trifluoromethyl-3-pyridyl | A-1 | 4-OH | represents a combination of substituents corresponding to each row of Table B |

[Table 8-2]

| | | | | | |
|---|---|---|---|---|---|
| Table 167 | 167-1~1 67-710 | 3-tetrahydrofuranyl | A-1 | 4-OH | represents a combination of substituents corresponding to each row of Table B |
| Table 168 | 168-1~1 68-710 | 6-Chloro-3-pyridyl | A-1 | 5-OH | represents a combination of substituents corresponding to each row of Table B |
| Table 169 | 169-1~1 69-710 | 2-Chloro-5-thiazolyl | A-1 | 5-OH | represents a combination of substituents corresponding to each row of Table B |
| Table 170 | 170-1~1 70-710 | 6-Fluoro-3-pyridyl | A-1 | 5-OH | represents a combination of substituents corresponding to each row of Table B |
| Table 171 | 171-1~1 71-710 | 6-Bromo-3-pyridyl | A-1 | 5-OH | represents a combination of substituents corresponding to each row of Table B |
| Table 172 | 172-1~1 72-710 | 6-Chloro-5-fluoro-3-pyridyl | A-1 | 5-OH | represents a combination of substituents corresponding to each row of Table B |
| Table 173 | 173-1~1 73-710 | 2-Chloro-5-pyrimidinyl | A-1 | 5-OH | represents a combination of substituents corresponding to each row of Table B |

[Table 8-3]

| | | | | | |
|---|---|---|---|---|---|
| Table 174 | 174-1~1 74-710 | 5-Chloropyrazin-2-yl | A-1 | 5-OH | represents a combination of substituents corresponding to each row of Table B |
| Table 175 | 175-1~1 75-710 | 6-Chloropyridazin-3-yl | A-1 | 5-OH | represents a combination of substituents corresponding to each row of Table B |
| Table 176 | 176-1~1 76-710 | 2-Chloro-5-oxazolyl | A-1 | 5-OH | represents a combination of substituents corresponding to each row of Table B |
| Table 177 | 177-1~7 7-710 | 6-trifluoromethyl-3-pyridyl | A-1 | 5-OH | represents a combination of substituents corresponding to each row of Table B |
| Table 178 | 178-1~1 78-710 | 3-tetrahydrofuranyl | A-1 | 5-OH | represents a combination of substituents corresponding to each row of Table B |

(continued)

| | | | | | |
|---|---|---|---|---|---|
| Table 179 | 179-1~1 79-710 | 6-Chloro-3-pyridyl | A-1 | 6-OH | represents a combination of substituents corresponding to each row of Table B |
| Table 180 | 180-1~1 80-710 | 2-Chloro-5-thiazolyl | A-1 | 6-OH | represents a combination of substituents corresponding to each row of Table B |

[Table 8-4]

| | | | | | |
|---|---|---|---|---|---|
| Table 181 | 181-1~1 81-710 | 6-Fluoro-3-pyridyl | A-1 | 6-OH | represents a combination of substituents corresponding to each row of Table B |
| Table 182 | 182-1~1 82-710 | 6-Bromo-3-pyridyl | A-1 | 6-OH | represents a combination of substituents corresponding to each row of Table B |
| Table 183 | 183-1~1 83-710 | 6-Chloro-5-fluoro-3-pyridyl | A-1 | 6-OH | represents a combination of substituents corresponding to each row of Table B |
| Table 184 | 184-1~1 84-710 | 2-Chloro-5-pyrimidinyl | A-1 | 6-OH | represents a combination of substituents corresponding to each row of Table B |
| Table 185 | 185-1~1 85-710 | 5-Chloropyrazin-2-yl | A-1 | 6-OH | represents a combination of substituents corresponding to each row of Table B |
| Table 186 | 186-1~1 86-710 | 6-Chloropyridazin-3-yl | A-1 | 6-OH | represents a combination of substituents corresponding to each row of Table B |
| Table 187 | 187-1~1 87-710 | 2-Chloro-5-oxazolyl | A-1 | 6-OH | represents a combination of substituents corresponding to each row of Table B |

[Table 8-5]

| | | | | | |
|---|---|---|---|---|---|
| Table 188 | 188-1~1 88-710 | 6-trifluoromethyl-3-pyridyl | A-1 | 6-OH | represents a combination of substituents corresponding to each row of Table B |
| Table 189 | 189-1~1 89-710 | 3-tetrahydrofuranyl | A-1 | 6-OH | represents a combination of substituents corresponding to each row of Table B |
| Table 190 | 190-1~1 90-710 | 6-Chloro-3-pyridyl | A-13 | H | represents a combination of substituents corresponding to each row of Table B |
| Table 191 | 191-1~1 91-710 | 2-Chloro-5-thiazolyl | A-13 | H | represents a combination of substituents corresponding to each row of Table B |
| Table 192 | 192-1~1 92-710 | 6-Fluoro-3-pyridyl | A-13 | H | represents a combination of substituents corresponding to each row of Table B |

[Table 9-1]

| Table A | | | | | |
|---|---|---|---|---|---|
| | Compound No. | Ar | A | Y | R |
| Table 193 | 193-1~1 93-710 | 6-Bromo-3-pyridyl | A-13 | H | represents a combination of substituents corresponding to each row of Table B |
| Table 194 | 194-1~1 94-710 | 6-Chloro-5-fluoro-3-pyridyl | A-13 | H | represents a combination of substituents corresponding to each row of Table B |

(continued)

| Table A | | | | | |
|---|---|---|---|---|---|
| | Compound No. | Ar | A | Y | R |
| Table 195 | 195-1~1 95-710 | 2-Chloro-5-pyrimidinyl | A-13 | H | represents a combination of substituents corresponding to each row of Table B |
| Table 196 | 196-1~1 96-710 | 5-Chloropyrazin-2-yl | A-13 | H | represents a combination of substituents corresponding to each row of Table B |
| Table 197 | 197-1~1 97-710 | 6-Chloropyridazin-3-yl | A-13 | H | represents a combination of substituents corresponding to each row of Table B |
| Table 198 | 198-1~1 98-710 | 2-Chloro-5-oxazolyl | A-13 | H | represents a combination of substituents corresponding to each row of Table B |

[Table 9-2]

| | | | | | |
|---|---|---|---|---|---|
| Table 199 | 199-1~1 99-710 | 6-trifluoromethyl-3-pyridyl | A-13 | H | represents a combination of substituents corresponding to each row of Table B |
| Table 200 | 200-1~2 00-710 | 3-tetrahydrofuranyl | A-13 | H | represents a combination of substituents corresponding to each row of Table B |
| Table 201 | 201-1~2 01-710 | 6-Chloro-3-pyridyl | A-14 | H | represents a combination of substituents corresponding to each row of Table B |
| Table 202 | 202-1~2 02-710 | 2-Chloro-5-thiazolyl | A-14 | H | represents a combination of substituents corresponding to each row of Table B |
| Table 203 | 203-1~2 03-710 | 6-Fluoro-3-pyridyl | A-14 | H | represents a combination of substituents corresponding to each row of Table B |
| Table 204 | 204-1~2 04-710 | 6-Bromo-3-pyridyl | A-14 | H | represents a combination of substituents corresponding to each row of Table B |
| Table 205 | 205-1~2 05-710 | 6-Chloro-5-fluoro-3-pyridyl | A-14 | H | represents a combination of substituents corresponding to each row of Table B |

[Table 9-3]

| | | | | | |
|---|---|---|---|---|---|
| Table 206 | 206-1~2 06-710 | 2-Chloro-5-pyrimidinyl | A-14 | H | represents a combination of substituents corresponding to each row of Table B |
| Table 207 | 207-1~2 07-710 | 5-Chloropyrazin-2-yl | A-14 | H | represents a combination of substituents corresponding to each row of Table B |
| Table 208 | 208-1~2 08-710 | 6-Chloropyridazin-3-yl | A-14 | H | represents a combination of substituents corresponding to each row of Table B |

(continued)

| | | | | | |
|---|---|---|---|---|---|
| Table 209 | 209-1~2 09-710 | 2-Chloro-5-oxazolyl | A-14 | H | represents a combination of substituents corresponding to each row of Table B |
| Table 210 | 210-1~2 10-710 | 6-trifluoromethyl-3-pyridyl | A-14 | H | represents a combination of substituents corresponding to each row of Table B |
| Table 211 | 211-1~2 11-710 | 3-tetrahydrofuranyl | A-14 | H | represents a combination of substituents corresponding to each row of Table B |
| Table 212 | 212-1~2 12-710 | 6-Chloro-3-pyridyl | A-15 | H | represents a combination of substituents corresponding to each row of Table B |

[Table 9-4]

| | | | | | |
|---|---|---|---|---|---|
| Table 213 | 213-1~2 13-710 | 2-Chloro-5-thiazolyl | A-15 | H | represents a combination of substituents corresponding to each row of Table B |
| Table 214 | 214-1~2 14-710 | 6-Fluoro-3-pyridyl | A-15 | H | represents a combination of substituents corresponding to each row of Table B |
| Table 215 | 215-1~2 15-710 | 6-Bromo-3-pyridyl | A-15 | H | represents a combination of substituents corresponding to each row of Table B |
| Table 216 | 216-1~2 16-710 | 6-Chloro-5-fluoro-3-pyridyl | A-15 | H | represents a combination of substituents corresponding to each row of Table B |
| Table 217 | 217-1~2 17-710 | 2-Chloro-5-pyrimidinyl | A-15 | H | represents a combination of substituents corresponding to each row of Table B |
| Table 218 | 218-1~2 18-710 | 5-Chloropyrazin-2-yl | A-15 | H | represents a combination of substituents corresponding to each row of Table B |
| Table 219 | 219-1~2 19-710 | 6-Chloropyridazin-3-yl | A-15 | H | represents a combination of substituents corresponding to each row of Table B |

[Table 9-5]

| | | | | | |
|---|---|---|---|---|---|
| Table 220 | 220-1~2 20-710 | 2-Chloro-5-oxazolyl | A-15 | H | represents a combination of substituents corresponding to each row of Table B |
| Table 221 | 221-1~2 21-710 | 6-trifluoromethyl-3-pyridyl | A-15 | H | represents a combination of substituents corresponding to each row of Table B |
| Table 222 | 222-1~2 22-710 | 3-tetrahydrofuranyl | A-15 | H | represents a combination of substituents corresponding to each row of Table B |
| Table 223 | 223-1~2 23-710 | 6-Chloro-3-pyridyl | A-16 | H | represents a combination of substituents corresponding to each row of Table B |
| Table 224 | 224-1~2 24-710 | 2-Chloro-5-thiazolyl | A-16 | H | represents a combination of substituents corresponding to each row of Table B |

[Table 10-1]

| Table A | | | | | |
|---|---|---|---|---|---|
| | Compound No. | Ar | A | Y | R |
| Table 225 | 225-1~22 5-710 | 6-Fluoro-3-pyridyl | A-16 | H | represents a combination of substituents corresponding to each row of Table B |
| Table 226 | 226-1~22 6-710 | 6-Bromo-3-pyridyl | A-16 | H | represents a combination of substituents corresponding to each row of Table B |
| Table 227 | 227-1~22 7-710 | 6-Chloro-5-fluoro-3-pyridyl | A-16 | H | represents a combination of substituents corresponding to each row of Table B |
| Table 228 | 228-1~22 8-710 | 2-Chloro-5-pyrimidinyl | A-16 | H | represents a combination of substituents corresponding to each row of Table B |
| Table 229 | 229-1~22 9-710 | 5-Chloropyrazin-2-yl | A-16 | H | represents a combination of substituents corresponding to each row of Table B |
| Table 230 | 230-1~23 0-710 | 6-Chloropyridazin-3-yl | A-16 | H | represents a combination of substituents corresponding to each row of Table B |

[Table 10-2]

| | | | | | |
|---|---|---|---|---|---|
| Table 231 | 231-1~ 23 1-710 | 2-Chloro-5-oxazolyl | A-16 | H | represents a combination of substituents corresponding to each row of Table B |
| Table 232 | 232-1~ 23 2-710 | 6-trifluoromethyl-3-pyridyl | A-16 | H | represents a combination of substituents corresponding to each row of Table B |
| Table 233 | 233-1~ 23 3-710 | 3-tetrahydrofuranyl | A-16 | H | represents a combination of substituents corresponding to each row of Table B |
| Table 234 | 234-1~ 23 4-710 | 6-Chloro-3-pyridyl | A-2 | H | represents a combination of substituents corresponding to each row of Table B |
| Table 235 | 235-1~ 23 5-710 | 6-Chloro-3-pyridyl | A-3 | H | represents a combination of substituents corresponding to each row of Table B |
| Table 236 | 236-1~ 23 6-710 | 6-Chloro-3-pyridyl | A-4 | H | represents a combination of substituents corresponding to each row of Table B |
| Table 237 | 237-1~ 23 7-710 | 6-Chloro-3-pyridyl | A-5 | H | represents a combination of substituents corresponding to each row of Table B |

[Table 10-3]

| | | | | | |
|---|---|---|---|---|---|
| Table 238 | 238-1~238-710 | 6-Chloro-3-pyridyl | A-6 | H | represents a combination of substituents corresponding to each row of Table B |
| Table 239 | 239-1~239-710 | 6-Chloro-3-pyridyl | A-7 | H | represents a combination of substituents corresponding to each row of Table B |
| Table 240 | 240-1~240-710 | 6-Chloro-3-pyridyl | A-8 | H | represents a combination of substituents corresponding to each row of Table B |
| Table 241 | 241-1~241-710 | 6-Chloro-3-pyridyl | A-9 | H | represents a combination of substituents corresponding to each row of Table B |
| Table 242 | 242-1~242-710 | 6-Chloro-3-pyridyl | A-10 | H | represents a combination of substituents corresponding to each row of Table B |
| Table 243 | 243-1~243-710 | 6-Chloro-3-pyridyl | A-11 | H | represents a combination of substituents corresponding to each row of Table B |
| Table 244 | 244-1~244-710 | 6-Chloro-3-pyridyl | A-12 | H | represents a combination of substituents corresponding to each row of Table B |

[Table 10-4]

| | | | | | |
|---|---|---|---|---|---|
| Table 245 | 245-1~245-710 | 6-Chloro-3-pyridyl | A-17 | H | represents a combination of substituents corresponding to each row of Table B |
| Table 246 | 246-1~246-710 | 6-Chloro-3-pyridyl | A-18 | H | represents a combination of substituents corresponding to each row of Table B |
| Table 247 | 247-1~247-710 | 6-Chloro-3-pyridyl | A-19 | H | represents a combination of substituents corresponding to each row of Table B |
| Table 248 | 248-1~248-710 | 6-Chloro-3-pyridyl | A-20 | H | represents a combination of substituents corresponding to each row of Table B |
| Table 249 | 249-1~249-710 | 6-Chloro-3-pyridyl | A-21 | H | represents a combination of substituents corresponding to each row of Table B |
| Table 250 | 250-1~250-710 | 6-Chloro-3-pyridyl | A-22 | H | represents a combination of substituents corresponding to each row of Table B |
| Table 251 | 251-1~251-710 | 6-Chloro-3-pyridyl | A-23 | H | represents a combination of substituents corresponding to each row of Table B |

[Table 10-5]

| | | | | | |
|---|---|---|---|---|---|
| Table 252 | 252-1~252-710 | 6-Chloro-3-pyridyl | A-24 | H | represents a combination of substituents corresponding to each row of Table B |
| Table 253 | 253-1~253-710 | 6-Chloro-3-pyridyl | A-25 | H | represents a combination of substituents corresponding to each row of Table B |
| Table 254 | 254-1~254-710 | 6-Chloro-3-pyridyl | A-26 | H | represents a combination of substituents corresponding to each row of Table B |
| Table 255 | 255-1~255-710 | 6-Chloro-3-pyridyl | A-27 | H | represents a combination of substituents corresponding to each row of Table B |
| Table 256 | 256-1~256-710 | 6-Chloro-3-pyridyl | A-28 | H | represents a combination of substituents corresponding to each row of Table B |

[Table 11-1]

| Table A | | | | | |
|---|---|---|---|---|---|
| | Compound No. | Ar | A | Y | R |
| Table 257 | 257-1~25 7-710 | 6-Chloro-3-pyridyl | A-2 9 | H | represents a combination of substituents corresponding to each row of Table B |
| Table 258 | 258-1~25 8-710 | 6-Chloro-3-pyridyl | A-3 0 | H | represents a combination of substituents corresponding to each row of Table B |
| Table 259 | 259-1~25 9-710 | 6-Chloro-3-pyridyl | A-3 1 | H | represents a combination of substituents corresponding to each row of Table B |
| Table 260 | 260-1~26 0-710 | 6-Chloro-3-pyridyl | A-3 2 | H | represents a combination of substituents corresponding to each row of Table B |
| Table 261 | 261-1~26 1-710 | 6-Chloro-3-pyridyl | A-3 3 | H | represents a combination of substituents corresponding to each row of Table B |
| Table 262 | 262-1~26 2-710 | 6-Chloro-3-pyridyl | A-3 4 | H | represents a combination of substituents corresponding to each row of Table B |

[Table 11-2]

| | | | | | |
|---|---|---|---|---|---|
| Table 263 | 263-1~26 3-710 | 6-Chloro-3-pyridyl | A-3 5 | H | represents a combination of substituents corresponding to each row of Table B |
| Table 264 | 264-1~26 4-710 | 6-Chloro-3-pyridyl | A-3 6 | H | represents a combination of substituents corresponding to each row of Table B |
| Table 265 | 265-1~26 5-710 | 6-Chloro-3-pyridyl | A-3 7 | H | represents a combination of substituents corresponding to each row of Table B |
| Table 266 | 266-1~26 6-710 | 6-Chloro-3-pyridyl | A-3 8 | H | represents a combination of substituents corresponding to each row of Table B |
| Table 267 | 267-1~26 7-710 | 6-Chloro-3-pyridyl | A-3 9 | H | represents a combination of substituents corresponding to each row of Table B |
| Table 268 | 268-1~26 8-710 | 6-Chloro-3-pyridyl | A-4 0 | H | represents a combination of substituents corresponding to each row of Table B |
| Table 269 | 269-1~26 9-710 | 6-Chloro-3-pyridyl | A-2 | H | represents a combination of substituents corresponding to each row of Table B |

[Table 11-3]

| | | | | | |
|---|---|---|---|---|---|
| Table 270 | 270-1~27 0-710 | 6-Chloro-3-pyridyl | A-3 | H | represents a combination of substituents corresponding to each row of Table B |
| Table 271 | 271-1~27 1-710 | 6-Chloro-3-pyridyl | A-4 | H | represents a combination of substituents corresponding to each row of Table B |
| Table 272 | 272-1~27 2-710 | 6-Chloro-3-pyridyl | A-5 | H | represents a combination of substituents corresponding to each row of Table B |
| Table 273 | 273-1~27 3-710 | 6-Chloro-3-pyridyl | A-6 | H | represents a combination of substituents corresponding to each row of Table B |
| Table 274 | 274-1~27 4-710 | 6-Chloro-3-pyridyl | A-7 | H | represents a combination of substituents corresponding to each row of Table B |
| Table 275 | 275-1~27 5-710 | 6-Chloro-3-pyridyl | A-8 | H | represents a combination of substituents corresponding to each row of Table B |

(continued)

| | | | | | |
|---|---|---|---|---|---|
| Table 276 | 276-1~27 6-710 | 6-Chloro-3-pyridyl | A-9 | H | represents a combination of substituents corresponding to each row of Table B |

[Table 11-4]

| | | | | | |
|---|---|---|---|---|---|
| Table 277 | 277-1~27 7-710 | 6-Chloro-3-py ridyl | A-1 0 | H | represents a combination of substituents corresponding to each row of Table B |
| Table 278 | 278-1~27 8-710 | 6-Chloro-3-py ridyl | A-1 1 | H | represents a combination of substituents corresponding to each row of Table B |
| Table 279 | 279-1~27 9-710 | 6-Chloro-3-py ridyl | A-1 2 | H | represents a combination of substituents corresponding to each row of Table B |
| Table 280 | 280-1~28 0-710 | 6-Chloro-3-py ridyl | A-1 7 | H | represents a combination of substituents corresponding to each row of Table B |
| Table 281 | 281-1~28 1-710 | 6-Chloro-3-py ridyl | A-1 8 | H | represents a combination of substituents corresponding to each row of Table B |
| Table 282 | 282-1~28 2-710 | 6-Chloro-3-py ridyl | A-1 9 | H | represents a combination of substituents corresponding to each row of Table B |
| Table 283 | 283-1~28 3-710 | 6-Chloro-3-py ridyl | A-2 0 | H | represents a combination of substituents corresponding to each row of Table B |

[Table 11-5]

| | | | | | |
|---|---|---|---|---|---|
| Table 284 | 284-1~28 4-710 | 6-Chloro-3-py ridyl | A-2 1 | H | represents a combination of substituents corresponding to each row of Table B |
| Table 285 | 285-1~28 5-710 | 6-Chloro-3-py ridyl | A-2 2 | H | represents a combination of substituents corresponding to each row of Table B |
| Table 286 | 286-1~28 6-710 | 6-Chloro-3-py ridyl | A-2 3 | H | represents a combination of substituents corresponding to each row of Table B |
| Table 287 | 287-1~28 7-710 | 6-Chloro-3-py ridyl | A-2 4 | H | represents a combination of substituents corresponding to each row of Table B |
| Table 288 | 288-1~28 8-710 | 6-Chloro-3-py ridyl | A-2 5 | H | represents a combination of substituents corresponding to each row of Table B |

[Table 12-1]

| Table A | | | | | |
|---|---|---|---|---|---|
| | Compound No. | Ar | A | Y | R |
| Table 289 | 289-1~28 9-710 | 6-Chloro-3-py ridyl | A-2 6 | H | represents a combination of substituents corresponding to each row of Table B |
| Table 290 | 290-1~29 0-710 | 6-Chloro-3-py ridyl | A-2 7 | H | represents a combination of substituents corresponding to each row of Table B |
| Table 291 | 291-1~29 1-710 | 6-Chloro-3-py ridyl | A-2 8 | H | represents a combination of substituents corresponding to each row of Table B |
| Table 292 | 292-1~29 2-710 | 6-Chloro-3-py ridyl | A-2 9 | H | represents a combination of substituents corresponding to each row of Table B |

(continued)

| Table A | | | | | |
|---|---|---|---|---|---|
| | Compound No. | Ar | A | Y | R |
| Table 293 | 293-1~29 3-710 | 6-Chloro-3-py ridyl | A-3 0 | H | represents a combination of substituents corresponding to each row of Table B |
| Table 294 | 294-1~29 4-710 | 6-Chloro-3-py ridyl | A-3 1 | H | represents a combination of substituents corresponding to each row of Table B |

[Table 12-2]

| | | | | | |
|---|---|---|---|---|---|
| Table 295 | 295-1~29 5-710 | 6-Chloro-3-py ridyl | A-3 2 | H | represents a combination of substituents corresponding to each row of Table B |
| Table 296 | 296-1~29 6-710 | 6-Chloro-3-py ridyl | A-3 3 | H | represents a combination of substituents corresponding to each row of Table B |
| Table 297 | 297-1~29 7-710 | 6-Chloro-3-py ridyl | A-3 4 | H | represents a combination of substituents corresponding to each row of Table B |
| Table 298 | 298-1~29 8-710 | 6-Chloro-3-py ridyl | A-3 5 | H | represents a combination of substituents corresponding to each row of Table B |
| Table 299 | 299-1~29 9-710 | 6-Chloro-3-py ridyl | A-3 6 | H | represents a combination of substituents corresponding to each row of Table B |
| Table 300 | 300-1~30 0-710 | 6-Chloro-3-py ridyl | A-3 7 | H | represents a combination of substituents corresponding to each row of Table B |
| Table 301 | 301-1~30 1-710 | 6-Chloro-3-py ridyl | A-3 8 | H | represents a combination of substituents corresponding to each row of Table B |

[Table 12-3]

| | | | | | |
|---|---|---|---|---|---|
| Table 302 | 302-1~30 2-710 | 6-Chloro-3-py ridyl | A-3 9 | H | represents a combination of substituents corresponding to each row of Table B |
| Table 303 | 303-1~30 3-710 | 6-Chloro-3-py ridyl | A-4 0 | H | represents a combination of substituents corresponding to each row of Table B |
| Table 304 | 304-1~30 4-710 | 6-Chloro-3-py ridyl | A-2 | H | represents a combination of substituents corresponding to each row of Table B |
| Table 305 | 305-1~30 5-710 | 6-Chloro-3-py ridyl | A-3 | H | represents a combination of substituents corresponding to each row of Table B |
| Table 306 | 306-1~30 6-710 | 6-Chloro-3-py ridyl | A-4 | H | represents a combination of substituents corresponding to each row of Table B |
| Table 307 | 307-1~30 7-710 | 6-Chloro-3-py ridyl | A-5 | H | represents a combination of substituents corresponding to each row of Table B |
| Table 308 | 308-1~30 8-710 | 6-Chloro-3-py ridyl | A-6 | H | represents a combination of substituents corresponding to each row of Table B |

[Table 12-4]

| | | | | | |
|---|---|---|---|---|---|
| Table 309 | 309-1~30 9-710 | 6-Chloro-3-py ridyl | A-7 | H | represents a combination of substituents corresponding to each row of Table B |

(continued)

| | | | | | |
|---|---|---|---|---|---|
| Table 310 | 310-1~31 0-710 | 6-Chloro-3-py ridyl | A-8 | H | represents a combination of substituents corresponding to each row of Table B |
| Table 311 | 311-1~31 1-710 | 6-Chloro-3-py ridyl | A-9 | H | represents a combination of substituents corresponding to each row of Table B |
| Table 312 | 312-1~31 2-710 | 6-Chloro-3-py ridyl | A-1 0 | H | represents a combination of substituents corresponding to each row of Table B |
| Table 313 | 313-1~31 3-710 | 6-Chloro-3-py ridyl | A-1 1 | H | represents a combination of substituents corresponding to each row of Table B |
| Table 314 | 314-1~31 4-710 | 6-Chloro-3-py ridyl | A-1 2 | H | represents a combination of substituents corresponding to each row of Table B |
| Table 315 | 315-1~31 5-710 | 6-Chloro-3-py ridyl | A-1 7 | H | represents a combination of substituents corresponding to each row of Table B |

[Table 12-5]

| | | | | | |
|---|---|---|---|---|---|
| Table 316 | 316-1~31 6-710 | 6-Chloro-3-py ridyl | A-1 8 | H | represents a combination of substituents corresponding to each row of Table B |
| Table 317 | 317-1~31 7-710 | 6-Chloro-3-py ridyl | A-1 9 | H | represents a combination of substituents corresponding to each row of Table B |
| Table 318 | 318-1~31 8-710 | 6-Chloro-3-py ridyl | A-2 0 | H | represents a combination of substituents corresponding to each row of Table B |
| Table 319 | 319-1~31 9-710 | 6-Chloro-3-py ridyl | A-2 1 | H | represents a combination of substituents corresponding to each row of Table B |
| Table 320 | 320-1~32 0-710 | 6-Chloro-3-py ridyl | A-2 2 | H | represents a combination of substituents corresponding to each row of Table B |

[Table 13-1]

| Table A | | | | | |
|---|---|---|---|---|---|
| | Compound No | Ar | A | Y | R |
| Table 321 | 321-1~3 21-710 | 6-Chloro-3-py ridyl | A-2 3 | H | represents a combination of substituents corresponding to each row of Table B |
| Table 322 | 322-1~3 22-710 | 6-Chloro-3-py ridyl | A-2 4 | H | represents a combination of substituents corresponding to each row of Table B |
| Table 323 | 323-1~3 23-710 | 6-Chloro-3-py ridyl | A-2 5 | H | represents a combination of substituents corresponding to each row of Table B |
| Table 324 | 324-1~3 24-710 | 6-Chloro-3-py ridyl | A-2 6 | H | represents a combination of substituents corresponding to each row of Table B |

[Table 13-2]

| | | | | | |
|---|---|---|---|---|---|
| Table 325 | 325-1~3 25-710 | 6-Chloro-3-py ridyl | A-2 7 | H | represents a combination of substituents corresponding to each row of Table B |
| Table 326 | 326-1~3 26-710 | 6-Chloro-3-py ridyl | A-2 8 | H | represents a combination of substituents corresponding to each row of Table B |

(continued)

| | | | | | |
|---|---|---|---|---|---|
| Table 327 | 327-1~3 27-710 | 6-Chloro-3-py ridyl | A-2 9 | H | represents a combination of substituents corresponding to each row of Table B |
| Table 328 | 328-1~3 28-710 | 6-Chloro-3-py ridyl | A-3 0 | H | represents a combination of substituents corresponding to each row of Table B |

[Table 13-3]

| | | | | | |
|---|---|---|---|---|---|
| Table 329 | 329-1~3 29-710 | 6-Chloro-3-py ridyl | A-3 1 | H | represents a combination of substituents corresponding to each row of Table B |
| Table 330 | 330-1~3 30-710 | 6-Chloro-3-py ridyl | A-3 2 | H | represents a combination of substituents corresponding to each row of Table B |
| Table 331 | 331-1~3 31-710 | 6-Chloro-3-py ridyl | A-3 3 | H | represents a combination of substituents corresponding to each row of Table B |
| Table 332 | 332-1~3 32-710 | 6-Chloro-3-py ridyl | A-3 4 | H | represents a combination of substituents corresponding to each row of Table B |

[Table 13-4]

| | | | | | |
|---|---|---|---|---|---|
| Table 333 | 333-1~3 33-710 | 6-Chloro-3-py ridyl | A-3 5 | H | represents a combination of substituents corresponding to each row of Table B |
| Table 334 | 334-1~3 34-710 | 6-Chloro-3-py ridyl | A-3 6 | H | represents a combination of substituents corresponding to each row of Table B |
| Table 335 | 335-1~3 35-710 | 6-Chloro-3-py ridyl | A-3 7 | H | represents a combination of substituents corresponding to each row of Table B |
| Table 336 | 336-1~3 36-710 | 6-Chloro-3-py ridyl | A-3 8 | H | represents a combination of substituents corresponding to each row of Table B |

[Table 13-5]

| | | | | | |
|---|---|---|---|---|---|
| Table 337 | 337-1~3 37-710 | 6-Chloro-3-py ridyl | A-3 9 | H | represents a combination of substituents corresponding to each row of Table B |
| Table 338 | 338-1~3 38-710 | 6-Chloro-3-py ridyl | A-4 0 | H | represents a combination of substituents corresponding to each row of Table B |
| Table 339 | 339-1~3 39-710 | 2-Chloro-5-th iazolyl | A-2 | H | represents a combination of substituents corresponding to each row of Table B |
| Table 340 | 340-1~3 40-710 | 3-Trifluorome thylphenyl | A-3 | H | represents a combination of substituents corresponding to each row of Table B |

[Table 13-6]

| | | | | | |
|---|---|---|---|---|---|
| Table 341 | 341-1~ 3 41-710 | 2-Methylpheny l | A-4 | H | represents a combination of substituents corresponding to each row of Table B |
| Table 342 | 342-1~ 3 42-710 | 3-Methylpheny l | A-5 | H | represents a combination of substituents corresponding to each row of Table B |

(continued)

| | | | | | |
|---|---|---|---|---|---|
| Table 343 | 343-1~3 43-710 | 4-Methylpheny l | A-6 | H | represents a combination of substituents corresponding to each row of Table B |
| Table 344 | 344-1~3 44-710 | 4-Trifluorome thylphenyl | A-7 | H | represents a combination of substituents corresponding to each row of Table B |

[Table 13-7]

| | | | | | |
|---|---|---|---|---|---|
| Table 345 | 345-1~3 45-710 | 2-Trifluorome thylphenyl | A-8 | H | represents a combination of substituents corresponding to each row of Table B |
| Table 346 | 346-1~3 46-710 | 2-Methoxyphen yl | A-9 | H | represents a combination of substituents corresponding to each row of Table B |
| Table 347 | 347-1~3 47-710 | 3-Methoxyphen yl | A-1 0 | H | represents a combination of substituents corresponding to each row of Table B |
| Table 348 | 348-1~3 48-710 | 4-Methoxyphen yl | A-1 1 | H | represents a combination of substituents corresponding to each row of Table B |

[Table 13-8]

| | | | | | |
|---|---|---|---|---|---|
| Table 349 | 349-1~3 49-710 | 2-Cyanophenyl | A-1 2 | H | represents a combination of substituents corresponding to each row of Table B |
| Table 350 | 350-1~3 50-710 | 3-Cyanophenyl | A-1 7 | H | represents a combination of substituents corresponding to each row of Table B |
| Table 351 | 351-1~3 51-710 | 4-Cyanophenyl | A-1 8 | H | represents a combination of substituents corresponding to each row of Table B |
| Table 352 | 352-1~3 52-710 | 2-Nitrophenyl | A-1 9 | H | represents a combination of substituents corresponding to each row of Table B |

[Table 14-1]

| Table A | | | | | |
|---|---|---|---|---|---|
| | Compound No | Ar | A | Y | R |
| Table 353 | 353-1~3 53-710 | 3-Nitrophenyl | A-2 0 | H | represents a combination of substituents corresponding to each row of Table B |
| Table 354 | 354-1~3 54-710 | 4-Nitrophenyl | A-2 1 | H | represents a combination of substituents corresponding to each row of Table B |
| Table 355 | 355-1~3 55-710 | 3-Hydroxy-2-p yridyl | A-2 2 | H | represents a combination of substituents corresponding to each row of Table B |
| Table 356 | 356-1~3 56-710 | 4-hydroxy-2-p yridyl | A-2 3 | H | represents a combination of substituents corresponding to each row of Table B |

[Table 14-2]

| | | | | | |
|---|---|---|---|---|---|
| Table 357 | 357-1~3 57-710 | 5-hydroxy-2-p yridyl | A-2 4 | H | represents a combination of substituents corresponding to each row of Table B |
| Table 358 | 358-1~3 58-710 | 6-hydroxy-2-p yridyl | A-2 5 | H | represents a combination of substituents corresponding to each row of Table B |
| Table 359 | 359-1~3 59-710 | 2-Hydroxy-3-p yridyl | A-2 6 | H | represents a combination of substituents corresponding to each row of Table B |
| Table 360 | 360-1~3 60-710 | 5-Hydroxy-3-p yridyl | A-2 7 | H | represents a combination of substituents corresponding to each row of Table B |

[Table 14-3]

| | | | | | |
|---|---|---|---|---|---|
| Table 361 | 361-1~3 61-710 | 6-Hydroxy-3-p yridyl | A-2 8 | H | represents a combination of substituents corresponding to each row of Table B |
| Table 362 | 362-1~3 62-710 | 4-Hydroxy-3-p yridyl | A-2 9 | H | represents a combination of substituents corresponding to each row of Table B |
| Table 363 | 363-1~3 63-710 | 2-Hydroxy-4-p yridyl | A-3 0 | H | represents a combination of substituents corresponding to each row of Table B |
| Table 364 | 364-1~3 64-710 | 3-Hydroxy-4-p yridyl | A-3 1 | H | represents a combination of substituents corresponding to each row of Table B |

[Table 14-4]

| | | | | | |
|---|---|---|---|---|---|
| Table 365 | 365-1~3 65-710 | 3-Chloro-2-py ridyl | A-3 2 | H | represents a combination of substituents corresponding to each row of Table B |
| Table 366 | 366-1~3 66-710 | 4-Chloro-2-py ridyl | A-3 3 | H | represents a combination of substituents corresponding to each row of Table B |
| Table 367 | 367-1~3 67-710 | 5-Chloro-2-py ridyl | A-3 4 | H | represents a combination of substituents corresponding to each row of Table B |
| Table 368 | 368-1~3 68-710 | 6-Chloro-2-py ridyl | A-3 5 | H | represents a combination of substituents corresponding to each row of Table B |

[Table 14-5]

| | | | | | |
|---|---|---|---|---|---|
| Table 369 | 369-1~3 69-710 | 2-Chloro-3-py ridyl | A-3 6 | H | represents a combination of substituents corresponding to each row of Table B |
| Table 370 | 370-1~3 70-710 | 5-Chloro-3-py ridyl | A-3 7 | H | represents a combination of substituents corresponding to each row of Table B |
| Table 371 | 371-1~3 71-710 | 6-Chloro-3-py ridyl | A-3 8 | H | represents a combination of substituents corresponding to each row of Table B |
| Table 372 | 372-1~3 72-710 | 4-Chloro-3-py ridyl | A-3 9 | H | represents a combination of substituents corresponding to each row of Table B |

[Table 14-6]

| | | | | | |
|---|---|---|---|---|---|
| Table 373 | 373-1~3 73-710 | 2-Chloro-4-py ridyl | A-4 0 | H | represents a combination of substituents corresponding to each row of Table B |

(continued)

| | | | | | |
|---|---|---|---|---|---|
| Table 374 | 374-1~3 74-710 | 3-Chloro-4-py ridyl | A-2 | H | represents a combination of substituents corresponding to each row of Table B |
| Table 375 | 375-1~3 75-710 | 3-bromo-2-pyr idyl | A-3 | H | represents a combination of substituents corresponding to each row of Table B |
| Table 376 | 376-1~3 76-710 | 4-bromo-2-pyr idyl | A-4 | H | represents a combination of substituents corresponding to each row of Table B |

[Table 14-7]

| | | | | | |
|---|---|---|---|---|---|
| Table 377 | 377-1~3 77-710 | 5-bromo-2-pyr idyl | A-5 | H | represents a combination of substituents corresponding to each row of Table B |
| Table 378 | 378-1~3 78-710 | 6-bromo-2-pyr idyl | A-6 | H | represents a combination of substituents corresponding to each row of Table B |
| Table 379 | 379-1~3 79-710 | 2-bromo-3-pyr idyl | A-7 | H | represents a combination of substituents corresponding to each row of Table B |
| Table 380 | 380-1~3 80-710 | 5-bromo-3-pyr idyl | A-8 | H | represents a combination of substituents corresponding to each row of Table B |

[Table 14-8]

| | | | | | |
|---|---|---|---|---|---|
| Table 381 | 381-1~ 3 81-710 | 6-bromo-3-pyr idyl | A-9 | H | represents a combination of substituents corresponding to each row of Table B |
| Table 382 | 382-1~ 3 82-710 | 4-bromo-3-pyr idyl | A-1 0 | H | represents a combination of substituents corresponding to each row of Table B |
| Table 383 | 383-1~ 3 83-710 | 2-bromo-4-pyr idyl | A-1 1 | H | represents a combination of substituents corresponding to each row of Table B |
| Table 384 | 384-1~ 3 84-710 | 3-bromo-4-pyr idyl | A-1 2 | H | represents a combination of substituents corresponding to each row of Table B |

[Table 15-1]

| Table A | | | | | |
|---|---|---|---|---|---|
| | Compoun d No | Ar | A | Y | R |
| Table 385 | 385-1~3 85-710 | 3-Fluoro-2-py ridyl | A-1 7 | H | represents a combination of substituents corresponding to each row of Table B |
| Table 386 | 386-1~3 86-710 | 4-Fluoro-2-py ridyl | A-1 8 | H | represents a combination of substituents corresponding to each row of Table B |
| Table 387 | 387-1~3 87-710 | 5-Fluoro-2-py ridyl | A-1 9 | H | represents a combination of substituents corresponding to each row of Table B |

[Table 15-2]

| | | | | | |
|---|---|---|---|---|---|
| Table 388 | 388-1~3 88-710 | 6-Fluoro-2-py ridyl | A-2 0 | H | represents a combination of substituents corresponding to each row of Table B |
| Table 389 | 389-1~3 89-710 | 2-Fluoro-3-py ridyl | A-2 1 | H | represents a combination of substituents corresponding to each row of Table B |
| Table 390 | 390-1~3 90-710 | 5-Fluoro-3-py ridyl | A-2 2 | H | represents a combination of substituents corresponding to each row of Table B |
| Table 391 | 391-1~3 91-710 | 6-Fluoro-3-py ridyl | A-2 3 | H | represents a combination of substituents corresponding to each row of Table B |

[Table 15-3]

| | | | | | |
|---|---|---|---|---|---|
| Table 392 | 392-1~3 92-710 | 4-Fluoro-3-py ridyl | A-2 4 | H | represents a combination of substituents corresponding to each row of Table B |
| Table 393 | 393-1~3 93-710 | 2-Fluoro-4-py ridyl | A-2 5 | H | represents a combination of substituents corresponding to each row of Table B |
| Table 394 | 394-1~3 94-710 | 3-Fluoro-4-py ridyl | A-2 6 | H | represents a combination of substituents corresponding to each row of Table B |
| Table 395 | 395-1~3 95-710 | 6-Fluoro-3-py ridyl | A-2 7 | H | represents a combination of substituents corresponding to each row of Table B |

[Table 15-4]

| | | | | | |
|---|---|---|---|---|---|
| Table 396 | 396-1~3 96-710 | 3-iodo-2-pyri dyl | A-2 8 | H | represents a combination of substituents corresponding to each row of Table B |
| Table 397 | 397-1~3 97-710 | 4-iodo-2-pyri dyl | A-2 9 | H | represents a combination of substituents corresponding to each row of Table B |
| Table 398 | 398-1~3 98-710 | 5-iodo-2-pyri dyl | A-3 0 | H | represents a combination of substituents corresponding to each row of Table B |
| Table 399 | 399-1~3 99-710 | 6-iodo-2-pyri dyl | A-3 1 | H | represents a combination of substituents corresponding to each row of Table B |

[Table 15-5]

| | | | | | |
|---|---|---|---|---|---|
| Table 400 | 400-1~4 00-710 | 2-iodo-3-pyri dyl | A-3 2 | H | represents a combination of substituents corresponding to each row of Table B |
| Table 401 | 401-1~4 01-710 | 5-iodo-3-pyri dyl | A-3 3 | H | represents a combination of substituents corresponding to each row of Table B |
| Table 402 | 402-1~4 02-710 | 6-iodo-3-pyri dyl | A-3 4 | H | represents a combination of substituents corresponding to each row of Table B |
| Table 403 | 403-1~4 03-710 | 4-iodo-3-pyri dyl | A-3 5 | H | represents a combination of substituents corresponding to each row of Table B |

[Table 15-6]

| | | | | | |
|---|---|---|---|---|---|
| Table 404 | 404-1~4 04-710 | 2-iodo-4-pyri dyl | A-3 6 | H | represents a combination of substituents corresponding to each row of Table B |

(continued)

| | | | | | |
|---|---|---|---|---|---|
| Table 405 | 405-1~4 05-710 | 3-iodo-4-pyri dyl | A-3 7 | H | represents a combination of substituents corresponding to each row of Table B |
| Table 406 | 406-1~4 06-710 | 6-iodo-3-pyri dyl | A-3 8 | H | represents a combination of substituents corresponding to each row of Table B |
| Table 407 | 407-1~4 07-710 | 6-iodo-3-pyri dyl | A-3 9 | H | represents a combination of substituents corresponding to each row of Table B |

[Table 15-7]

| | | | | | |
|---|---|---|---|---|---|
| Table 408 | 408-1~4 08-710 | 2-tetrahydrof uranyl | A-4 0 | H | represents a combination of substituents corresponding to each row of Table B |
| Table 409 | 409-1~4 09-710 | 3-tetrahydrof uranyl | A-2 | H | represents a combination of substituents corresponding to each row of Table B |
| Table 410 | 410-1~4 10-710 | 5-Chloro-2-th iazolyl | A-3 | H | represents a combination of substituents corresponding to each row of Table B |
| Table 411 | 411-1~4 11-710 | 6-Fluoro-3-py ridyl | A-4 | H | represents a combination of substituents corresponding to each row of Table B |

[Table 15-8]

| | | | | | |
|---|---|---|---|---|---|
| Table 412 | 412-1~4 12-710 | 6-Bromo-3-pyr idyl | A-5 | H | represents a combination of substituents corresponding to each row of Table B |
| Table 413 | 413-1~4 13-710 | 6-Chloro-5-Fl uoro-3-pyridy l | A-6 | H | represents a combination of substituents corresponding to each row of Table B |
| Table 414 | 414-1~4 14-710 | 3,5-Dimethylp henyl | A-7 | H | represents a combination of substituents corresponding to each row of Table B |
| Table 415 | 415-1~4 15-710 | 2,3-Dimethylp henyl | A-8 | H | represents a combination of substituents corresponding to each row of Table B |

[Table 15-9]

| | | | | | |
|---|---|---|---|---|---|
| Table 416 | 416-1~4 16-710 | 2,4-Dimethyop henyl | A-9 | H | represents a combination of substituents corresponding to each row of Table B |

[Table 16-1]

| Table A | | | | | |
|---|---|---|---|---|---|
| | Compound No | Ar | A | Y | R |
| Table 417 | 417-1~41 7-710 | Phenyl | A-1 0 | H | represents a combination of substituents corresponding to each row of Table B |
| Table 418 | 418-1~41 8-710 | cyclopentyl | A-1 1 | H | represents a combination of substituents corresponding to each row of Table B |
| Table 419 | 419-1~41 9-710 | cyclohexyl | A-1 2 | H | represents a combination of substituents corresponding to each row of Table B |
| Table 420 | 420-1~42 0-710 | 3-methylcycl ohexyl | A-1 7 | H | represents a combination of substituents corresponding to each row of Table B |

[Table 16-2]

| | | | | | |
|---|---|---|---|---|---|
| Table 421 | 421-1~42 1-710 | cyclobutyl | A-1 8 | H | represents a combination of substituents corresponding to each row of Table B |
| Table 422 | 422-1 ~422-710 | 2-oxetanyl | A-1 9 | H | represents a combination of substituents corresponding to each row of Table B |
| Table 423 | 423-1~42 3-710 | 3-oxetanyl | A-2 0 | H | represents a combination of substituents corresponding to each row of Table B |
| Table 424 | 424-1~42 4-710 | 2-thietanyl | A-2 1 | H | represents a combination of substituents corresponding to each row of Table B |

[Table 16-3]

| | | | | | |
|---|---|---|---|---|---|
| Table 425 | 425-1~42 5-710 | 3-thietanyl | A-2 2 | H | represents a combination of substituents corresponding to each row of Table B |
| Table 426 | 426-1~42 6-710 | 2-azetidinyl | A-2 3 | H | represents a combination of substituents corresponding to each row of Table B |
| Table 427 | 427-1~42 7-710 | 3-azetidinyl | A-2 4 | H | represents a combination of substituents corresponding to each row of Table B |
| Table 428 | 428-1~42 8-710 | 6-iodo-3-pyr idyl | A-2 5 | H | represents a combination of substituents corresponding to each row of Table B |

[Table 16-4]

| | | | | | |
|---|---|---|---|---|---|
| Table 429 | 429-1~42 9-710 | 6-iodo-3-pyr idyl | A-2 6 | H | represents a combination of substituents corresponding to each row of Table B |
| Table 430 | 430-1~43 0-710 | 2-tetrahydro furanyl | A-2 7 | H | represents a combination of substituents corresponding to each row of Table B |
| Table 431 | 431-1~43 1-710 | 2-Chloro-3-p yridyl | A-2 8 | H | represents a combination of substituents corresponding to each row of Table B |
| Table 432 | 432-1~43 2-710 | 5-Chloro-3-p yridyl | A-2 9 | H | represents a combination of substituents corresponding to each row of Table B |

[Table 16-5]

| | | | | | |
|---|---|---|---|---|---|
| Table 433 | 433-1~43 3-710 | 6-Chloro-3-p yridyl | A-3 0 | H | represents a combination of substituents corresponding to each row of Table B |
| Table 434 | 434-1~43 4-710 | 4-Chloro-3-p yridyl | A-3 1 | H | represents a combination of substituents corresponding to each row of Table B |
| Table 435 | 435-1~43 5-710 | 2-Chloro-4-p yridyl | A-3 2 | H | represents a combination of substituents corresponding to each row of Table B |
| Table 436 | 436-1~43 6-710 | 3-Chloro-4-p yridyl | A-3 3 | H | represents a combination of substituents corresponding to each row of Table B |

[Table 16-6]

| | | | | | |
|---|---|---|---|---|---|
| Table 437 | 437-1~43 7-710 | 3-bromo-2-py ridyl | A-3 4 | H | represents a combination of substituents corresponding to each row of Table B |

(continued)

| | | | | | |
|---|---|---|---|---|---|
| Table 438 | 438-1~43 8-710 | 4-bromo-2-py ridyl | A-3 5 | H | represents a combination of substituents corresponding to each row of Table B |
| Table 439 | 439-1~43 9-710 | 2-Fluoro-4-p yridyl | A-3 6 | H | represents a combination of substituents corresponding to each row of Table B |
| Table 440 | 440-1~44 0-710 | 3-Fluoro-4-p yridyl | A-3 7 | H | represents a combination of substituents corresponding to each row of Table B |

[Table 16-7]

| | | | | | |
|---|---|---|---|---|---|
| Table 441 | 441-1~44 1-710 | 6-Fluoro-3-p yridyl | A-3 8 | H | represents a combination of substituents corresponding to each row of Table B |
| Table 442 | 442-1~44 2-710 | 3-iodo-2-pyr idyl | A-3 9 | H | represents a combination of substituents corresponding to each row of Table B |
| Table 443 | 443-1~44 3-710 | 6-Fluoro-3-p yridyl | A-4 0 | H | represents a combination of substituents corresponding to each row of Table B |
| Table 444 | 444-1~44 4-710 | 2-Chloro-5-t hiazolyl | A-3 8 | H | represents a combination of substituents corresponding to each row of Table B |

[Table 17-1]

| Table A | | | | | |
|---|---|---|---|---|---|
| | Compound No. | Ar | A | Y | R |
| Table 445 | 445-1~44 5-710 | 6-Chloro-3-p yridyl | A-1 | 3-CH 3 | represents a combination of substituents corresponding to each row of Table B |
| Table 446 | 446-1~44 6-710 | 2-Chloro-5-t hiazolyl | A-1 | 3-CH 3 | represents a combination of substituents corresponding to each row of Table B |
| Table 447 | 447-1~44 7-710 | 6-Fluoro-3-p yridyl | A-1 | 3-CH 3 | represents a combination of substituents corresponding to each row of Table B |

[Table 17-2]

| | | | | | |
|---|---|---|---|---|---|
| Table 448 | 448-1~44 8-710 | 6-Bromo-3-py ridyl | A-1 | 3-CH 3 | represents a combination of substituents corresponding to each row of Table B |
| Table 449 | 449-1~44 9-710 | 6-Chloro-5-f luoro-3-pyri dyl | A-1 | 3-CH 3 | represents a combination of substituents corresponding to each row of Table B |
| Table 450 | 450-1~45 0-710 | 2-Chloro-5-p yrimidinyl | A-1 | 3-CH 3 | represents a combination of substituents corresponding to each row of Table B |
| Table 451 | 451-1~45 1-710 | 5-Chloropyra zin-2-yl | A-1 | 3-CH 3 | represents a combination of substituents corresponding to each row of Table B |

[Table 17-3]

| | | | | | |
|---|---|---|---|---|---|
| Table 452 | 452-1~45 2-710 | 6-Chloropyri dazin-3-yl | A-1 | 3-CH 3 | represents a combination of substituents corresponding to each row of Table B |
| Table 453 | 453-1~45 3-710 | 2-Chloro-5-o xazolyl | A-1 | 3-CH 3 | represents a combination of substituents corresponding to each row of Table B |

(continued)

| | | | | | |
|---|---|---|---|---|---|
| Table 454 | 454-1~45 4-710 | 6-trifluorom ethyl-3-pyri dyl | A-1 | 3-CH 3 | represents a combination of substituents corresponding to each row of Table B |
| Table 455 | 455-1~45 5-710 | 3-tetrahydro furanyl | A-1 | 3-CH 3 | represents a combination of substituents corresponding to each row of Table B |

[Table 17-4]

| | | | | | |
|---|---|---|---|---|---|
| Table 456 | 456-1~ 45 6-710 | 6-Chloro-3-p yridyl | A-1 | 4-CH 3 | represents a combination of substituents corresponding to each row of Table B |
| Table 457 | 457-1~ 45 7-710 | 2-Chloro-5-t hiazolyl | A-1 | 4-CH 3 | represents a combination of substituents corresponding to each row of Table B |
| Table 458 | 458-1~ 45 8-710 | 6-Fluoro-3-p yridyl | A-1 | 4-CH 3 | represents a combination of substituents corresponding to each row of Table B |
| Table 459 | 459-1~ 45 9-710 | 6-Bromo-3-py ridyl | A-1 | 4-CH 3 | represents a combination of substituents corresponding to each row of Table B |

[Table 17-5]

| | | | | | |
|---|---|---|---|---|---|
| Table 460 | 460-1~46 0-710 | 6-Chloro-5-F luoro-3-pyri dyl | A-1 | 4-CH 3 | represents a combination of substituents corresponding to each row of Table B |
| Table 461 | 461-1~46 1-710 | 2-Chloro-5-p yrimidinyl | A-1 | 4-CH 3 | represents a combination of substituents corresponding to each row of Table B |
| Table 462 | 462-1~46 2-710 | 5-Chloropyra zin-2-yl | A-1 | 4-CH 3 | represents a combination of substituents corresponding to each row of Table B |
| Table 463 | 463-1~46 3-710 | 6-Chloropyri dazin-3-yl | A-1 | 4-CH 3 | represents a combination of substituents corresponding to each row of Table B |

[Table 17-6]

| | | | | | |
|---|---|---|---|---|---|
| Table 464 | 464-1~46 4-710 | 2-Chloro-5-o xazolyl | A-1 | 4-CH 3 | represents a combination of substituents corresponding to each row of Table B |
| Table 465 | 465-1~46 5-710 | 6-trifluorom ethyl-3-pyri dyl | A-1 | 4-CH 3 | represents a combination of substituents corresponding to each row of Table B |
| Table 466 | 466-1~46 6-710 | 3-tetrahydro furanyl | A-1 | 4-CH 3 | represents a combination of substituents corresponding to each row of Table B |
| Table 467 | 467-1~46 7-710 | 6-Chloro-3-p yridyl | A-1 | 5-CH 3 | represents a combination of substituents corresponding to each row of Table B |

[Table 17-7]

| | | | | | |
|---|---|---|---|---|---|
| Table 468 | 468-1~46 8-710 | 2-Chloro-5-t hiazolyl | A-1 | 5-CH 3 | represents a combination of substituents corresponding to each row of Table B |

(continued)

| | | | | | |
|---|---|---|---|---|---|
| Table 469 | 469-1~46 9-710 | 6-Fluoro-3-p yridyl | A-1 | 5-CH 3 | represents a combination of substituents corresponding to each row of Table B |
| Table 470 | 470-1~47 0-710 | 6-Bromo-3-py ridyl | A-1 | 5-CH 3 | represents a combination of substituents corresponding to each row of Table B |
| Table 471 | 471-1~47 1-710 | 6-Chloro-5-f luoro-3-pyri dyl | A-1 | 5-CH 3 | represents a combination of substituents corresponding to each row of Table B |

[Table 17-8]

| | | | | | |
|---|---|---|---|---|---|
| Table 472 | 472-1~47 2-710 | 2-Chloro-5-p yrimidinyl | A-1 | 5-CH 3 | represents a combination of substituents corresponding to each row of Table B |
| Table 473 | 473-1~47 3-710 | 5-Chloropyra zin-2-yl | A-1 | 5-CH 3 | represents a combination of substituents corresponding to each row of Table B |
| Table 474 | 474-1~47 4-710 | 6-Chloropyri dazin-3-yl | A-1 | 5-CH 3 | represents a combination of substituents corresponding to each row of Table B |
| Table 475 | 475-1~47 5-710 | 2-Chloro-5-o xazolyl | A-1 | 5-CH 3 | represents a combination of substituents corresponding to each row of Table B |

[Table 17-9]

| | | | | | |
|---|---|---|---|---|---|
| Table 476 | 476-1~ 47 6-710 | 6-trifluoromethyl-3-pyridyl | A-1 | 5-CH 3 | represents a combination of substituents corresponding to each row of Table B |

[Table 18-1]

| Table A | | | | | |
|---|---|---|---|---|---|
| | Compound No. | Ar | A | Y | R |
| Table 477 | 477-1~47 7-710 | 3-tetrahydro furanyl | A-1 | 5-CH 3 | represents a combination of substituents corresponding to each row of Table B |
| Table 478 | 478-1~47 8-710 | 6-Chloro-3-p yridyl | A-1 | 6-CH 3 | represents a combination of substituents corresponding to each row of Table B |
| Table 479 | 479-1~47 9-710 | 2-Chloro-5-t hiazolyl | A-1 | 6-CH 3 | represents a combination of substituents corresponding to each row of Table B |

[Table 18-2]

| | | | | | |
|---|---|---|---|---|---|
| Table 480 | 480-1~48 0-710 | 6-Fluoro-3-p yridyl | A-1 | 6-CH 3 | represents a combination of substituents corresponding to each row of Table B |
| Table 481 | 481-1~48 1-710 | 6-Bromo-3-py ridyl | A-1 | 6-CH 3 | represents a combination of substituents corresponding to each row of Table B |
| Table 482 | 482-1~48 2-710 | 6-Chloro-5-f luoro-3-pyri dyl | A-1 | 6-CH 3 | represents a combination of substituents corresponding to each row of Table B |
| Table 483 | 483-1~48 3-710 | 2-Chloro-5-p yrimidinyl | A-1 | 6-CH 3 | represents a combination of substituents corresponding to each row of Table B |

[Table 18-3]

| | | | | | |
|---|---|---|---|---|---|
| Table 484 | 484-1~48 4-710 | 5-Chloropyra zin-2-yl | A-1 | 6-CH 3 | represents a combination of substituents corresponding to each row of Table B |
| Table 485 | 485-1~48 5-710 | 6-Chloropyri dazin-3-yl | A-1 | 6-CH 3 | represents a combination of substituents corresponding to each row of Table B |
| Table 486 | 486-1~48 6-710 | 2-Chloro-5-o xazolyl | A-1 | 6-CH 3 | represents a combination of substituents corresponding to each row of Table B |
| Table 487 | 487-1~48 7-710 | 6-trifluorom ethyl-3-pyri dyl | A-1 | 6-CH 3 | represents a combination of substituents corresponding to each row of Table B |

[Table 18-4]

| | | | | | |
|---|---|---|---|---|---|
| Table 488 | 488-1~ 48 8-710 | 3-tetrahydro furanyl | A-1 | 6-CH 3 | represents a combination of substituents corresponding to each row of Table B |
| Table 489 | 489-1~ 48 9-710 | 6-Chloro-3-p yridyl | A-1 | 3-NO 2 | represents a combination of substituents corresponding to each row of Table B |
| Table 490 | 490-1~ 49 0-710 | 2-Chloro-5-t hiazolyl | A-1 | 3-NO 2 | represents a combination of substituents corresponding to each row of Table B |
| Table 491 | 491-1~ 49 1-710 | 6-Fluoro-3-p yridyl | A-1 | 3-NO 2 | represents a combination of substituents corresponding to each row of Table B |

[Table 18-5]

| | | | | | |
|---|---|---|---|---|---|
| Table 492 | 492-1~49 2-710 | 6-Bromo-3-py ridyl | A-1 | 3-NO 2 | represents a combination of substituents corresponding to each row of Table B |
| Table 493 | 493-1~49 3-710 | 6-Chloro-5-F luoro-3-pyri dyl | A-1 | 3-NO 2 | represents a combination of substituents corresponding to each row of Table B |
| Table 494 | 494-1~49 4-710 | 2-Chloro-5-p yrimidinyl | A-1 | 3-NO 2 | represents a combination of substituents corresponding to each row of Table B |
| Table 495 | 495-1~49 5-710 | 5-Chloropyra zin-2-yl | A-1 | 3-NO 2 | represents a combination of substituents corresponding to each row of Table B |

[Table 18-6]

| | | | | | |
|---|---|---|---|---|---|
| Table 496 | 496-1~49 6-710 | 6-Chloropyri dazin-3-yl | A-1 | 3-NO 2 | represents a combination of substituents corresponding to each row of Table B |
| Table 497 | 497-1~49 7-710 | 2-Chloro-5-o xazolyl | A-1 | 3-NO 2 | represents a combination of substituents corresponding to each row of Table B |
| Table 498 | 498-1~49 8-710 | 6-trifluorom ethyl-3-pyri dyl | A-1 | 3-NO 2 | represents a combination of substituents corresponding to each row of Table B |
| Table 499 | 499-1~49 9-710 | 3-tetrahydro furanyl | A-1 | 3-NO 2 | represents a combination of substituents corresponding to each row of Table B |

[Table 18-7]

| | | | | | |
|---|---|---|---|---|---|
| Table 500 | 500-1~50 0-710 | 6-Chloro-3-p yridyl | A-1 | 4-NO 2 | represents a combination of substituents corresponding to each row of Table B |
| Table 501 | 501-1~50 1-710 | 2-Chloro-5-t hiazolyl | A-1 | 4-NO 2 | represents a combination of substituents corresponding to each row of Table B |
| Table 502 | 502-1~50 2-710 | 6-Fluoro-3-p yridyl | A-1 | 4-NO 2 | represents a combination of substituents corresponding to each row of Table B |
| Table 503 | 503-1~50 3-710 | 6-Bromo-3-py ridyl | A-1 | 4-NO 2 | represents a combination of substituents corresponding to each row of Table B |

[Table 18-8]

| | | | | | |
|---|---|---|---|---|---|
| Table 504 | 504-1~50 4-710 | 6-Chloro-5-f luoro-3-pyri dyl | A-1 | 4-NO 2 | represents a combination of substituents corresponding to each row of Table B |
| Table 505 | 505-1~50 5-710 | 2-Chloro-5-p yrimidinyl | A-1 | 4-NO 2 | represents a combination of substituents corresponding to each row of Table B |
| Table 506 | 506-1~50 6-710 | 5-Chloropyra zin-2-yl | A-1 | 4-NO 2 | represents a combination of substituents corresponding to each row of Table B |
| Table 507 | 507-1~50 7-710 | 6-Chloropyri dazin-3-yl | A-1 | 4-NO 2 | represents a combination of substituents corresponding to each row of Table B |

[Table 18-9]

| | | | | | |
|---|---|---|---|---|---|
| Table 508 | 508-1~50 8-710 | 2-Chloro-5-o xazolyl | A-1 | 4-NO 2 | represents a combination of substituents corresponding to each row of Table B |

[Table 19-1]

| Table A | | | | | |
|---|---|---|---|---|---|
| | Compound No. | Ar | A | Y | R |
| Table 509 | 509~1~50 9-710 | 6-trifluorom ethyl-3-pyri dyl | A-1 | 4-NO 2 | represents a combination of substituents corresponding to each row of Table B |
| Table 510 | 510-1~51 0-710 | 3-tetrahydro furanyl | A-1 | 4-NO 2 | represents a combination of substituents corresponding to each row of Table B |
| Table 511 | 511-1~51 1-710 | 6-Chloro-3-p yridyl | A-1 | 5-NO 2 | represents a combination of substituents corresponding to each row of Table B |

[Table 19-2]

| | | | | | |
|---|---|---|---|---|---|
| Table 512 | 512-1~51 2-710 | 2-Chloro-5-t hiazolyl | A-1 | 5-NO 2 | represents a combination of substituents corresponding to each row of Table B |

(continued)

| | | | | | |
|---|---|---|---|---|---|
| Table 513 | 513-1~51 3-710 | 6-Fluoro-3-p yridyl | A-1 | 5-NO 2 | represents a combination of substituents corresponding to each row of Table B |
| Table 514 | 514-1~51 4-710 | 6-Bromo-3- py ridyl | A-1 | 5-NO 2 | represents a combination of substituents corresponding to each row of Table B |
| Table 515 | 515-1~51 5-710 | 6-Chloro-5-f luoro-3-pyri dyl | A-1 | 5-NO 2 | represents a combination of substituents corresponding to each row of Table B |

[Table 19-3]

| | | | | | |
|---|---|---|---|---|---|
| Table 516 | 516-1~51 6-710 | 2-Chloro-5-p yrimidinyl | A-1 | 5-NO 2 | represents a combination of substituents corresponding to each row of Table B |
| Table 517 | 517-1~51 7-710 | 5-Chloropyra zin-2-yl | A-1 | 5-NO 2 | represents a combination of substituents corresponding to each row of Table B |
| Table 518 | 518-1~51 8-710 | 6-Chloropyri dazin-3-yl | A-1 | 5-NO 2 | represents a combination of substituents corresponding to each row of Table B |
| Table 519 | 519-1~51 9-710 | 2-Chloro-5-o xazolyl | A-1 | 5-NO 2 | represents a combination of substituents corresponding to each row of Table B |

[Table 19-4]

| | | | | | |
|---|---|---|---|---|---|
| Table 520 | 520-1~52 0-710 | 6-trifluorom ethyl-3- pyri dyl | A-1 | 5-NO 2 | represents a combination of substituents corresponding to each row of Table B |
| Table 521 | 521-1~52 1-710 | 3-tetrahydro furanyl | A-1 | 5-NO 2 | represents a combination of substituents corresponding to each row of Table B |
| Table 522 | 522-1~52 2-710 | 6-Chloro-3-p yridyl | A-1 | 6-NO 2 | represents a combination of substituents corresponding to each row of Table B |
| Table 523 | 523-1~52 3-710 | 2-Chloro-5-t hiazolyl | A-1 | 6-NO 2 | represents a combination of substituents corresponding to each row of Table B |

[Table 19-5]

| | | | | | |
|---|---|---|---|---|---|
| Table 524 | 524-1~52 4-710 | 6-Fluoro-3-p yridyl | A-1 | 6-NO 2 | represents a combination of substituents corresponding to each row of Table B |
| Table 525 | 525-1~52 5-710 | 6-Bromo-3-py ridyl | A-1 | 6-NO 2 | represents a combination of substituents corresponding to each row of Table B |
| Table 526 | 526-1~52 6-710 | 6-Chloro-5-F luoro- 3-pyri dyl | A-1 | 6-NO 2 | represents a combination of substituents corresponding to each row of Table B |
| Table 527 | 527-1~52 7-710 | 2-Chloro-5-p yrimidinyl | A-1 | 6-NO 2 | represents a combination of substituents corresponding to each row of Table B |

[Table 19-6]

| | | | | | |
|---|---|---|---|---|---|
| Table 528 | 528-1~52 8-710 | 5-Chloropyra zin-2-yl | A-1 | 6-NO 2 | represents a combination of substituents corresponding to each row of Table B |
| Table 529 | 529-1~52 9-710 | 6-Chloropyri dazin-3-yl | A-1 | 6-NO 2 | represents a combination of substituents corresponding to each row of Table B |

(continued)

| | | | | | |
|---|---|---|---|---|---|
| Table 530 | 530-1~530-710 | 2-Chloro-5-o xazolyl | A-1 | 6-NO 2 | represents a combination of substituents corresponding to each row of Table B |
| Table 531 | 531-1~531-710 | 6-trifluorom ethyl-3-pyri dyl | A-1 | 6-NO 2 | represents a combination of substituents corresponding to each row of Table B |

[Table 19-7]

| | | | | | |
|---|---|---|---|---|---|
| Table 532 | 532-1~53 2-710 | 3-tetrahydro furanyl | A-1 | 6-NO 2 | represents a combination of substituents corresponding to each row of Table B |
| Table 533 | 533-1~53 3-710 | 6-Chloro-3-p yridyl | A-1 | 3-OC H3 | represents a combination of substituents corresponding to each row of Table B |
| Table 534 | 534-1~53 4-710 | 2-Chloro-5-t hiazolyl | A-1 | 3-OC H3 | represents a combination of substituents corresponding to each row of Table B |
| Table 535 | 535-1~53 5-710 | 6-Fluoro-3-p yridyl | A-1 | 3-OC H3 | represents a combination of substituents corresponding to each row of Table B |

[Table 19-8]

| | | | | | |
|---|---|---|---|---|---|
| Table 536 | 536-1~536-710 | 6-Bromo-3-py ridyl | A-1 | 3-OC H3 | represents a combination of substituents corresponding to each row of Table B |
| Table 537 | 537-1~537-710 | 6-Chloro-5-f luoro-3-pyri dyl | A-1 | 3-OC H3 | represents a combination of substituents corresponding to each row of Table B |
| Table 538 | 538-1~538-710 | 2-Chloro-5-p yrimidinyl | A-1 | 3-OC H3 | represents a combination of substituents corresponding to each row of Table B |
| Table 539 | 539-1~539-710 | 5-Chloropyra zin-2-yl | A-1 | 3-OC H3 | represents a combination of substituents corresponding to each row of Table B |

[Table 19-9]

| | | | | | |
|---|---|---|---|---|---|
| Table 540 | 540-1~540-710 | 6-Chloropyri dazin-3-yl | A-1 | 3-OC H3 | represents a combination of substituents corresponding to each row of Table B |

[Table 20-1]

| Table A | | | | | |
|---|---|---|---|---|---|
| | Compound No. | Ar | A | Y | R |
| Table 541 | 541-1~54 1-710 | 2-Chloro-5-o xazolyl | A-1 | 3-OC H3 | represents a combination of substituents corresponding to each row of Table B |
| Table 542 | 542-1~54 2-710 | 6-trifluorom ethyl-3-pyri dyl | A-1 | 3-OC H3 | represents a combination of substituents corresponding to each row of Table B |

(continued)

| Table A | | | | | |
|---|---|---|---|---|---|
| | Compound No. | Ar | A | Y | R |
| Table 543 | 543-1~54 3-710 | 3-tetrahydro furanyl | A-1 | 3-OC H3 | represents a combination of substituents corresponding to each row of Table B |

[Table 20-2]

| | | | | | |
|---|---|---|---|---|---|
| Table 544 | 544-1~ 54 4-710 | 6-Chloro-3-p yridyl | A-1 | 4-OC H3 | represents a combination of substituents corresponding to each row of Table B |
| Table 545 | 545-1~ 54 5-710 | 2-Chloro-5-t hiazolyl | A-1 | 4-OC H3 | represents a combination of substituents corresponding to each row of Table B |
| Table 546 | 546-1~ 54 6-710 | 6-Fluoro-3-p yridyl | A-1 | 4-OC H3 | represents a combination of substituents corresponding to each row of Table B |
| Table 547 | 547-1~ 54 7-710 | 6-Bromo-3-py ridyl | A-1 | 4-OC H3 | represents a combination of substituents corresponding to each row of Table B |

[Table 20-3]

| | | | | | |
|---|---|---|---|---|---|
| Table 548 | 548-1~54 8-710 | 6-Chloro-5-F luoro-3-pyri dyl | A-1 | 4-OC H3 | represents a combination of substituents corresponding to each row of Table B |
| Table 549 | 549-1~54 9-710 | 2-Chloro-5-p yrimidinyl | A-1 | 4-OC H3 | represents a combination of substituents corresponding to each row of Table B |
| Table 550 | 550-1~55 0-710 | 5-Chloropyra zin-2-yl | A-1 | 4-OC H3 | represents a combination of substituents corresponding to each row of Table B |
| Table 551 | 551-1~55 1-710 | 6-Chloropyri dazin-3-yl | A-1 | 4-OC H3 | represents a combination of substituents corresponding to each row of Table B |

[Table 20-4]

| | | | | | |
|---|---|---|---|---|---|
| Table 552 | 552-1~55 2-710 | 2-Chloro-5-oxazolyl | A-1 | 4-OC H3 | represents a combination of substituents corresponding to each row of Table B |
| Table 553 | 553-1~55 3-710 | 6-trifluoromethyl-3-pyri dyl | A-1 | 4-OC H3 | represents a combination of substituents corresponding to each row of Table B |
| Table 554 | 554-1~55 4-710 | 3-tetrahydrofuranyl | A-1 | 4-OC H3 | represents a combination of substituents corresponding to each row of Table B |
| Table 555 | 555-1~55 5-710 | 6-Chloro-3-pyridyl | A-1 | 5-OC H3 | represents a combination of substituents corresponding to each row of Table B |

[Table 20-5]

| | | | | | |
|---|---|---|---|---|---|
| Table 556 | 556-1~55 6-710 | 2-Chloro-5-thiazolyl | A-1 | 5-OC H3 | represents a combination of substituents corresponding to each row of Table B |

(continued)

| | | | | | |
|---|---|---|---|---|---|
| Table 557 | 557-1~55 7-710 | 6-Fluoro-3-pyridyl | A-1 | 5-OC H3 | represents a combination of substituents corresponding to each row of Table B |
| Table 558 | 558-1~55 8-710 | 6-Bromo-3-pyridyl | A-1 | 5-OC H3 | represents a combination of substituents corresponding to each row of Table B |
| Table 559 | 559-1~55 9-710 | 6-Chloro-5-fluoro-3-pyridyl | A-1 | 5-OC H3 | represents a combination of substituents corresponding to each row of Table B |

[Table 20-6]

| | | | | | |
|---|---|---|---|---|---|
| Table 560 | 560-1~56 0-710 | 2-Chloro-5-pyrimidinyl | A-1 | 5-OC H3 | represents a combination of substituents corresponding to each row of Table B |
| Table 561 | 561-1~56 1-710 | 5-Chloropyrazin-2-yl | A-1 | 5-OC H3 | represents a combination of substituents corresponding to each row of Table B |
| Table 562 | 562-1~56 2-710 | 6-Chloropyridazin-3-yl | A-1 | 5-OC H3 | represents a combination of substituents corresponding to each row of Table B |
| Table 563 | 563-1~56 3-710 | 2-Chloro-5-o xazolyl | A-1 | 5-OC H3 | represents a combination of substituents corresponding to each row of Table B |

[Table 20-7]

| | | | | | |
|---|---|---|---|---|---|
| Table 564 | 564-1~56 4-710 | 6-trifluoromethyl-3-pyri dyl | A-1 | 5-OC H3 | represents a combination of substituents corresponding to each row of Table B |
| Table 565 | 565-1~56 5-710 | 3-tetrahydrofuranyl | A-1 | 5-OC H3 | represents a combination of substituents corresponding to each row of Table B |
| Table 566 | 566-1~56 6-710 | 6-Chloro-3-pyridyl | A-1 | 6-OC H3 | represents a combination of substituents corresponding to each row of Table B |
| Table 567 | 567-1~56 7-710 | 2-Chloro-5-thiazolyl | A-1 | 6-OC H3 | represents a combination of substituents corresponding to each row of Table B |

[Table 20-8]

| | | | | | |
|---|---|---|---|---|---|
| Table 568 | 568-1~56 8-710 | 6-Fluoro-3-pyridyl | A-1 | 6-OC H3 | represents a combination of substituents corresponding to each row of Table B |
| Table 569 | 569-1~56 9-710 | 6-Bromo-3-pyridyl | A-1 | 6-OC H3 | represents a combination of substituents corresponding to each row of Table B |
| Table 570 | 570-1~57 0-710 | 6-Chloro-5-Fluoro-3-pyridyl | A-1 | 6-OC H3 | represents a combination of substituents corresponding to each row of Table B |
| Table 571 | 571-1~57 1-710 | 2-Chloro-5-pyrimidinyl | A-1 | 6-OC H3 | represents a combination of substituents corresponding to each row of Table B |

[Table 20-9]

| | | | | | |
|---|---|---|---|---|---|
| Table 572 | 572-1~ 57 2-710 | 5-Chloropyrazin-2-yl | A-1 | 6-OC H3 | represents a combination of substituents corresponding to each row of Table B |

[Table 21-1]

| Table A | | | | | |
|---|---|---|---|---|---|
| | Compound No. | Ar | A | Y | R |
| Table 573 | 573-1~57 3-710 | 6-Chloropyridazin-3-yl | A-1 | 6-OC H3 | represents a combination of substituents corresponding to each row of Table B |
| Table 574 | 574-1~57 4-710 | 2-Chloro-5-o xazolyl | A-1 | 6-OC H3 | represents a combination of substituents corresponding to each row of Table B |
| Table 575 | 575-1~57 5-710 | 6-trifluorom ethyl-3-pyri dyl | A-1 | 6-OC H3 | represents a combination of substituents corresponding to each row of Table B |

[Table 21-2]

| | | | | | |
|---|---|---|---|---|---|
| Table 576 | 576-1~57 6-710 | 3-tetrahydrofuranyl | A-1 | 6-OC H3 | represents a combination of substituents corresponding to each row of Table B |
| Table 577 | 577-1~57 7-710 | 2,6-dichloro-3-pyridyl | A-1 | H | represents a combination of substituents corresponding to each row of Table B |
| Table 578 | 578-1~57 8-710 | 3-pyridyl | A-1 | H | represents a combination of substituents corresponding to each row of Table B |
| Table 579 | 579-1~57 9-710 | 4-pyridyl | A-1 | H | represents a combination of substituents corresponding to each row of Table B |

[Table 21-3]

| | | | | | |
|---|---|---|---|---|---|
| Table 580 | 580-1~58 0-710 | 6-chloro-3-p yridyl-N-oxide | A-1 | H | represents a combination of substituents corresponding to each row of Table B |

[Table 22-1]

| Table B | | |
|---|---|---|
| | R | |
| | 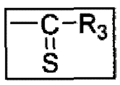 | R3 |
| 20 | | CF3 |
| 21 | | CHF2 |
| 22 | | CF2Cl |
| 23 | | CF2CF3 |
| 24 | | CH2Cl |
| 25 | | CHCl2 |
| 26 | | CCl3 |
| 27 | | CHClBr |
| 28 | | CHBr2 |

(continued)

| Table B | | |
|---|---|---|
| | R | |
| 29 | | 2,3,3-trifluo roacryl |
| 30 | | CH2CHF2 |
| 31 | | CH2CF3 |
| 32 | | CH=CH2 |
| 33 | | CH2C≡CH |

[Table 22-2]

| | | |
|---|---|---|
| 34 | | CH2CF3 |
| 35 | | CH2CH2Ph |
| 36 | | Me |
| 37 | | Et |
| 38 | | n-Pr |
| 39 | | i-Pr |
| 40 | | cyclopropyl |

[Table 35]

| Table B | | |
|---|---|---|
| | R | |
| | 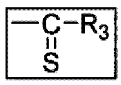 | |
| | | R3 |
| 648 | | C6F5 |
| 649 | | CH2OCH2C6H5 |

Preferred examples of the compound represented by Formula (I) include compounds in the following Table 38.

[Table 38-1]

| Compound No | Ar | A | Y | R |
|---|---|---|---|---|
| 1-20 | 6-Chloro-3-pyridyl | A-1 | H | CSCF3 |

[Table 38-2]

| | | | | |
|---|---|---|---|---|
| 1-21 | 6-Chloro-3-pyridyl | A-1 | H | CSCHF2 |
| 3-20 | 6-Fluoro-3-pyridyl | A-1 | H | CSCF3 |
| 4-20 | 6-Bromo-3-pyridyl | A-1 | H | CSCF3 |

[Table 38-3]

| | | | | |
|---|---|---|---|---|
| 1-22 | 6-Chloro-3-pyridyl | A-1 | H | CSCF2C1 |
| 1-23 | 6-Chloro-3-pyridyl | A-1 | H | CSCF2CF3 |
| 5-20 | 6-Chloro-5-fluoro-3-pyridyl | A-1 | H | CSCF3 |

[Table 38-4]

| | | | | |
|---|---|---|---|---|
| 2-20 | 2-chloro-5-thiazolyl | A-1 | H | CSCF3 |
| 10-20 | 6-trifluorometh yl-3-pyridyl | A-1 | H | CSCF3 |
| 11-20 | 3-THF | A-1 | H | CSCF3 |
| 1-37 | 6-Chloro-3-pyridyl | A-1 | H | CSEt |
| 1-39 | 6-Chloro-3-pyridyl | A-1 | H | CS-i-Pr |

[Table 38-5]

| | | | | |
|---|---|---|---|---|
| 1-40 | 6-Chloro-3-pyridyl | A-1 | H | CS-cyclopropy l |
| 1-35 | 6-Chloro-3-pyridyl | A-1 | H | CSCH2CH2Ph |

Examples of particularly preferable compounds according to the invention include compounds 1-20, 1-21, 3-20, 4-20, 1-22, 1-23, and 5-20.

Examples of insect species against which a pest control agent containing at least one of the compounds of the present invention, which is represented by Formula (I), shows pest control effects are as follows.

Examples of agricultural and horticultural pests include lepidopteran pests (for example, Spodoptera litura, cabbage armyworm, Mythimnaseparata, cabbageworm, cabbage moth, Spodoptera exigua, rice stem borer, grass leaf roller, Naranga aenescens, tortricid, codling moth, leafminer moth, tussock moth, Agrotis spp, Helicoverpa spp, Heliothis spp and the like), hemipteran pests (for example, aphids (Aphididae, Adelgidae, Phylloxeridae) such as Myzus persicae, Aphis gossypii, Aphis fabae, corn leaf aphid, pea aphid, Aulacorthum solani, Aphis craccivora, Macrosiphum euphorbiae, Macrosiphum avenae, Methopolophium dirhodum, Rhopalosiphum padi, greenbug, Brevicorynebrassicae, Lipaphis erysimi, Aphis spiraecola, Rosy apple aphid, apple blight, Toxoptera aurantii, Toxoptera citricidus and the like, leafhoppers such as Nephotettix cincticeps, Empoasca vitis and the like, planthoppers such as Laodelphax striatella, Nilaparvata lugens, Sogatella furcifera and the like, Pentatomorpha such as Eysarcoris ventralis, Nezara viridula, Plautia stali, Trigonotylus caelestialium and the like, whiteflies (Aleyrodidae) such as silverleaf whitefly, Bemisia tabaci, greenhouse whitefly and the like, scale insects (Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, Cerococcidae and the like) such as Pseudococcus comstocki, Planococcus citri, Pseudaulacaspis pentagona, Aonidiella aurantii and the like, coleopteran pests (for example, Lissorhoptrus oryzophilus, Callosobruchus chinensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Bostrichidae, Cerambycidae and the like), Acarina (for example, Tetranychus urticae, Tetranychus kanzawai, Panonychus citri and the like), hymenopteran pests (for example, Tenthredinidae), orthopteran pests (for example, Acridioidea), dipteran pests (for example, housefly and Agromyzidae), thysanopteran pests (for example, Thrips palmi, Frankliniella occidentalis and the like), phytoparasitic nematode (for example, Meloidogyne, Pratylenchus, Aphelenchoides besseyi, Bursaphelenchus xylophilus and the like), and the like.

Examples of animal parasitic pests include Ixodidae (for example, Amblyomma americanum, Amblyomma maculatum, Boophilus microplus, Dermacentor andersoni, Dermacentor occidentalis, Dermacentor variabilis, Haemaphysalis campanulata, Haemaphysalis flava, Haemaphysalis longicornis, Haemaphysalis megaspinosa Saito, Ixodes nipponensis, Ixodes ovatus, Ixodes pacifcus, Ixodes persulcatus, Ixodes ricinus, Ixodes scapularis, Ornithodoros moubata pacifcus and Rhipicephalus sanguineus), Cheyletidae (for example, Cheyletiella blakei and Cheyletiella yasguri), Demodex (for example, Demodex canis and Demodex cati), Psoroptidae (for example, Psoroptescommunis), Sarcoptidae (for example, Chorioptes bovis and Otodectes cynotis), Dermanyssidae (for example, Ornithonyssus sylviarum), Dermanyssus gallinae, Pterolichus (for example, Megninia cubitalis and Pterolichus obtusus), Trombiculidae (for example, Helenicula miyagawai and Leptotrombidium akamushi), Shiphonaptera (for example, Ctenocephalides felis, Pulex irritans, Xenopsylla cheopis and Xenopsylla), Mallophaga (for example, Trichodectes canis and Menopon gallinae),

Anoplura (for example, Haematopinus suis, Linognathus setosus, Pediculus humanus humanus, Pediculus humanus, Pthirus pubis and Cimex lectularius), Musca domestica, Hypoderma bovis, Stomoxys calcitrans, Gasterophilus, Psychodidae (for example, Phlebotomus), Glossina morsitans, Tabanidae, Aedes spp. (for example, Aedes albopictus and Aedes aegypti), Culex spp. (for example, Culex pipiens pallens), Anophelini, Ceratopogonidae, Simuliidae, Reduviidae, Monomorium pharaonis, Nematoda (for example, Strongyloides, Ancylostomatoidea, Strongyloidea (for example, Haemonchus contortus and Nippostrongylus braziliensis), Trichostrongyloidea, Metastrongyloidea (for example, Metastrongylus elongatus, Angiostrongylus cantonensis and Aelurostrongylus abstrutus), Oxyuroidea, Haterakoidea (for example, Ascaridia galli), Ascaridoidea (for example, Anisakis simplex, Ascaris suum, Parascaris equorum, Toxocara canis and Toxocara cati), Spiruroidea (for example, Subuluroidea, Gnathostoma spinigerum, Physaloptea praeputialis, Ascarops strongylina, Draschia megastoma and Ascaria hamulosa, Dracunculus medinensis), Filarioidea (for example, Dirofilaria immitis, lymphatic filarial, Onchocerca volvulus and Loa loa), Dioctophymatoidea, Trichinella (for example, Trichuris vulpis and Trichinella spiralis)), Trematoda (for example, Schistosoma japonicum and Fasciola hepatica), Acanthocephala, Taenia (for example, Pseudophyllidea (for example, Spirometra erinaceieuropaei) and Cyclophyllidea (for example, Dipylidium caninum)), Protozoa, and the like.

Examples of hygiene pests, nuisance pests, stored grain pests, stored product pests and house pests include Culicidae spp. (for example, Aedes albopictus and Culex pipiens pallens), Periplaneta (for example, Periplaneta fuliginosa, Periplaneta japonica and Blattella germanica), Acaridae (for example, Tyrophagusputrescentiae), Diptera (for example, housefly, Sarcophagaperegrina, Psychodidae, Drosophila and Chironomus), Simuliidae, Ceratopogonidae, hymenopteran insects (for example, Formicidae such as Camponotus japonicus, Solenopsis spp. and the like and Hymenoptera such as Vespa mandarinia), Arthropod of Isopoda (for example, Porcellio scaber, Ligia exotica and Armadillidium vulgare), hemipteran insescts (for example, Cimex lectularius), Arthropod of Myriapoda (for example, centipedes, millipedes and Diplopoda), Arthropod of Araneae (for example, Heteropoda venatoria), coleopteran insects (for example, Anisodactylus signatus), Arthropod of Collembola (for example, Onychiurus folsomi), dermapteran insects (for example, Labidura riparia), orthopteran insects (for example, Stenopelmatidae), coleopteran insects (for example, Callosobruchus chinensis, Sitophilus zeamais, Tenebroides mauritanicus, Tribolium castaneum, Anthrenus museorum, Anobiidae, Scolytidae spp., Dermestidae and Chlorophorus diadema inhirsutusMatsushita), lepidopteran insects (for example, Pyralidae and Tineidae), Hemipeplidae, isopteran insects (for example, Coptotermes formosanus, Incisitermes minor (Hagen)and Odontotermes formosanus), Thysanura (for example, Ctenolepisma villosa) and the like.

Among them, preferred examples of insect species to which the pest control agent of the present invention is applied include lepidopteran pests, hemipteran pests, thysanopteran pests, dipteran pests, coleopteran pests, animal parasitic Shiphonaptera or Acari, Dirofilaria immitis, mosquitoes, Periplaneta and isopteran insects (for example, at least one insect species selected from the group consisting of cabbage moth, Spodoptera litura, Aphis gossypii, Myzus persicae, Laodelphax striatella, Nilaparvata lugens, Sogatella furcifera, Nephotettix cincticeps, Trigonotylus caelestialium, Plautia stali, Frankliniella occidentalis, Oulema oryzae, Lissorhoptrus oryzophilus, housefly, Haemaphysalis longicornis, Dirofilaria immitis, Culex pipiens pallens, Blattella germanica and Coptotermes formosanus), more preferred examples thereof include hemipteran pests, coleopteran insects and Ixodidae, and particularly preferred examples thereof include planthoppers, Nephotettix cincticeps and imidacloprid or fipronil-resistant planthoppers.

Accordingly, examples of a pest control agent provided by the present invention include an agricultural and horticultural insecticide, an agent for controlling endoparasites of an animal, an agent for controlling ectoparasites of an animal, an agent for controlling hygiene pests, an agent for controlling nuisance pests, an agent for controlling stored grain and stored product pests, an agent for controlling house pests and the like, but preferred examples thereof include an agricultural and horticultural insecticide, an agent for controlling endoparasites of an animal and an agent for controlling ectoparasites of an animal.

The pest control agent of the present invention may be prepared by using a carrier according to the use thereof in addition to the compound represented by Formula (I).

When the pest control agent of the present invention is an agricultural pest control agent, the agent is usually mixed with an appropriate solid carrier, liquid carrier, gaseous carrier, surfactant, dispersant and other adjuvants for preparation to be provided in any formulation form of emulsifiable concentrates, liquid formulations, suspensions, wettable powders, flowables, dust, granules, tablets, oils, aerosols, fumigants and the like.

Examples of the solid carrier include talc, bentonite, clay, kaolin, diatomaceousearth, vermiculite, white carbon, calcium carbonate and the like.

Examples of the liquid carrier include alcohols such as methanol, n-hexanol, ethylene glycol and the like, ketones such as acetone, methyl ethyl ketone, cyclohexane and the like, aliphatic hydrocarbons such as n-hexane, kerosene, lamp oil and the like, aromatic hydrocarbons such as toluene, xylene, methyl naphthalene and the like, ethers such as diethyl ether, dioxane, tetrahydrofuran and the like, esters such as ethyl acetate and the like, nitriles such as acetonitrile, isobutyl nitrile and the like, acid amides such as dimethylformamide, dimethylacetamide and the like, vegetable oils such as soybean oil, cotton seed oil and the like, dimethyl sulfoxide, water and the like.

Further, examples of the gaseous carrier include LPG, air, nitrogen, carbonic acid gas, dimethyl ether and the

like.

As the surfactant or dispersant for emulsification, dispersion, spreading and the like, it is possible to use, for example, alkylsulfate esters, alkyl (aryl) sulfonates, polyoxyalkylene alkyl (aryl) ethers, polyhydric alcohol esters, lignin sulfonates or the like.

In addition, as the adjuvant for improving the properties of the preparation, it is possible to use, for example, carboxymethylcellulose, gum arabic, polyethylene glycol, calcium stearate or the like.

The aforementioned carriers, surfactants, dispersants and adjuvants may be used either alone or in combination, if necessary.

The content of active ingredients in the preparation is not particularly limited, but is usually in the range from 1 to 75% by weight for the emulsifiable concentrate, from 0.3 to 25% by weight for the dust, from 1 to 90% by weight for the wettable powder, and from 0.5 to 10% by weight for the granule.

The application thereof may be performed before and after the invasion of pest insects.

In particular, it is possible to control pests by applying an effective amount of the compounds represented by Formula (I), a preparation including the same and a mixed formulation of other pest control agents with the same to a subject selected from the group consisting of seeds, roots, tubers, bulbs and rhizomes of plants, germinated plants, seedlings, soil, a nutrient solution in nutrient solution culture and a solid medium in nutrient solution culture and penetrating and migrating the compound, the preparation or the mixed formulation into the plants.

When the subject to be applied is seeds, roots, tubers, bulbs or rhizomes of plants, appropriate examples of the application method are not particularly limited, but include a dipping method, a dust coating method, a smearing method, a spraying method, a pelleting method, a coating method and the like as long as the penetration and migration are not disturbed.

In the case of seeds, examples of the application method include a dipping method, a dust coating method, a smearing method, a spraying method, a pelleting method, a coating method and a fumigating method. The dipping method is a method in which seeds are dipped in a liquid chemical solution, and the dust coating method is classified into a dry dust coating method in which a granular chemical is adhered onto dry seeds, and a wet dust coating method in which a granular chemical is adhered onto seeds which have been slightly soaked in water. Further, the smearing method is a method in which a suspended chemical is applied on the surface of seeds within a mixer and the spraying method is a method in which a suspended chemical is sprayed onto the surface of seeds. In addition, the pelleting method is a method in which a chemical is mixed with a filler and treated when seeds are pelleted together with the filler to form pellets having certain size and shape, the coating method is a method in which a chemical-containing film is coated onto seeds, and the fumigating method is a method in which seeds are sterilized with a chemical which has been gasified within a hermetically sealed container.

When the method is applied to germinated plants and seedlings, these plants may be protected by the treatment of the whole or a part thereof by dipping by applying the method after germination, after budding from soil or before transplantation.

Further, when the method is applied to seeds, roots, tubers, bulbs, rhizomes or the like, examples of the method also include a method in which seeds, roots, tubers, bulbs, rhizomes or the like are planted or dipped in the chemical for a time enough to penetrate and migrate the chemical into the plants. In this case, the time and temperature for dipping is appropriately determined by those skilled in the art depending on the subject to be applied, kind and amount of drug and the like. Moreover, time for penetration and migration is not particularly limited, but is, for example, 1 hour or longer. In addition, the temperature for penetration and migration is, for example, from 5°C to 45°C.

Examples of the method for applying the chemical to soil include a method in which granules of the compounds of the present invention, a preparation including the same and a mixed formulation of other pest control agents with the same are applied into soil or on soil. Preferred soil application methods include spraying, stripe application, groove application, and planting hole application. Here, the spraying treatment includes a surface treatment over the entire area to be treated and a subsequent mechanical introduction into soil.

In addition, application by drenching of soil with a solution prepared by emulsifying or dissolving the nitrogen-containing heterocyclic derivatives having a 2-imino group of the present invention, a preparation including the same and a mixed formulation of other pest control agents with the same in water is also an advantageous soil application method.

When the method is applied to a nutrient solution in nutrient solution culture systems such as solid medium cultivation, such as hydroponicculture, sand culture, NFT (nutrient film technique), rock wool culture and the like for the production of vegetables and flowering plants, it is obvious that the compounds of the present invention, a preparation including the same and a mixed formulation of other pest control agents with the same may be applied directly to artificial culture soil including vermiculite and a solid medium including an artificial mat for growing seedling.

Further, in the application process, an effective amount of the compound of Formula (I) or salts thereof is preferably an amount enough for the compound of Formula (1) to be penetrated and migrated into the plant in the subsequent penetration and migration process.

The effective amount may be appropriately determined by considering the properties of the compound, the kind and amount of subject to be applied, the length of the subsequent penetration and migration process, the temperature and the like, but for example, in the case of a seed, the compound of Formula (I) or salts thereof is applied in an amount of preferably from 1 g to 10 kg and more preferably from 10 g to 1 kg, per 10 kg of the seed. Further, the amount of the compound of Formula (I) or salts thereof applied to soil is preferably from 0.1 g to 10 kg and more preferably from 1 g to 1 kg, per 10 ares of cultivated land. The amount of the compound of Formula (I) or salts thereof treated to leaves and stems of a plant is preferably from 0.1 g to 10 kg and more preferably from 1 g to 1 kg, per 10 ares of cultivated land.

When the pest control agent of the present invention is a control agent for animal parasitic pests, the agent is provided in the form of liquid formulations, emulsifiable concentrates, liquid drops, sprays, foam preparations, tablets, granules, fine subtilaes, dust, capsules, chewable formulations, injections, suppositories, creams, shampoos, rinses, resin agents, fumigants, poison baits and the like, and is particularly preferably provided in the form of liquid formulations and liquid drops.

The liquid formulation may also be blended with a typical adjuvant for preparation, such as an emulsifier, a dispersant, a spreading agent, a wetting agent, a suspending agent, a preservative, a propellant and the like, and may also be blended with a typical film former. As the surfactant for emulsification, dispersion, spreading and the like, it is possible to use, for example, soaps, polyoxyalkylene alkyl (aryl) ethers, polyoxyethylene alkyl aryl ethers, polyoxyethylene fatty acid ester, higher alcohols, alkyl aryl sulfonates and the like. Examples of dispersants include casein, gelatin, polysaccharides, lignin derivatives, saccharides, synthetic water soluble polymers and the like. Examples of spreading and wetting agents include glycerin, polyethylene glycol and the like. Examples of suspending agents include casein, gelatin, hydroxypropylcellulose, gum arabic and the like, and examples of stabilizers include phenolic antioxidants (BHT, BHA and the like), amine antioxidants (diphenylamine and the like), organic sulfur antioxidants and the like. Examples of preservatives include methyl p-oxybenzoate, ethyl p-oxybenzoate, propyl p-oxybenzoate, butyl p-oxybenzoate and the like. The aforementioned carriers, surfactants, dispersants and adjuvants may be used either alone or in combination, if necessary. In addition, perfumes, synergists and the like may also be contained. It is appropriate that the content of the active ingredients in the pest control agent of the present invention is usually from 1 to 75% by weight for the liquid formulation.

Examples of carriers used for the preparation of creams include non-volatile hydrocarbons (liquid paraffin and the like), lanolin hydrogenated fats and oils, higher fatty acids, fatty acid esters, animal and vegetable oils, silicone oils, water and the like. Further, emulsifiers, humectants, antioxidants, perfumes, borax and ultraviolet absorbers may also be used either alone or in combination, if necessary. Examples of emulsifiers include fatty acid sorbitan, polyoxyethylene alkyl ether, and fatty acid polyoxyethylene and the like. It is appropriate that the content of the active ingredients in the pest control agent of the present invention is usually from 0.5 to 70% by weight for the cream.

The capsules, pills or tablets may be used such that the active ingredients in the composition of the present invention are divided into suitable small portions, the small portion is mixed with a diluting solution or a carrier such as starch, lactose, talc, or the like, a disintegrator and/or a binder, such as magnesium stearate is added thereto, and the mixture is tabletted, if necessary.

Injections need to be prepared as an aseptic solution. For injections, the solution may contain, for example, a salt or glucose enough to isotonicate the solution with blood. Examples of available carriers for the preparation of injections include esters such as fatty acid derivatives of glyceride, benzyl benzoate, isopropyl myristate and propylene glycol, and the like, and organic solvents such as N-methylpyrrolidone and glycerol formal. It is appropriate that the content of the active ingredients in the pest control agent of the present invention is usually from 0.01 to 10% by weight for the injection.

Examples of carriers for the preparation of resin agents include vinyl chloride polymers, polyurethane and the like. Plasticizers such as phthalic acid esters, adipic acid esters, stearic acid and the like may be added to these bases, if necessary. After the active ingredients are kneaded into the base, the kneaded product may be molded by injection molding, extrusion molding, press molding and the like. Further, the molded product may also be properly subjected to processes such as molding, cutting or the like to form an ear tag for animals or insecticidal collar for animals.

Examples of carriers for toxic baits include bait substances and attraction substances (farina such as wheat flour, corn flour and the like, starch such as corn starch, potato starch and the like, saccharides such as granulated sugar, malt sugar, honey and the like, food flavors such as glycerin, onion flavor, milk flavor and the like, animal powders such as pupal powder, fish powder and the like, various pheromones and the like). It is appropriate that the content of the active ingredients in the pest control agent of the present invention is usually from 0.0001 to 90% by weight for the toxic bait.

It is possible to control pests by administering the pest control agent of the present invention into an applied animal either orally or by injection, or wholly or partly administering the agent into the body surface of the applied animal. In addition, it is also possible to control pests by covering places, in which the invasion, parasitism and movement of pests are expected, with the pest control agent of the present invention.

The pest control agent of the present invention may be used as it is, but may be diluted with water, liquid

carriers, commercially available shampoos, rinses, baits, breed cage bottoms and the like and applied in some cases.

Further, the pest control agent according to the present invention may be mixed with other insecticides, fungicides, miticides, herbicides, plant growth regulators, fertilizers and the like and the mixture may be used. Examples of a chemical that may be mixed and used include those described in The Pesticide Manual (13th edition and published by the British Crop Protection Council) or the SHIBUYA INDEX (15th edition, 2010 and published by SHIBUYA INDEX RESEARCH GROUP). As insecticides, miticides or nematicides, more specific examples thereof include an organic phosphoric ester compound such as acephate, dichlorvos, EPN, fenitrothion, fenamifos, prothiofos, profenofos, pyraclofos, chlorpyrifos-methyl, diazinon, fosthiazate, imicyafos, trichlorfon, tetrachlorvinphos, bromofenofos and cythioate, a carbamate-based compound such as methomyl, thiodicarb, aldicarb, oxamyl, propoxur, carbaryl, fenobucarb, ethiofencarb, fenothiocarb, pirimicarb, carbofuran and benfuracarb, a nereistoxin derivative such as cartap and thiocyclam, an organochlorine compound such as dicofol and tetradifon, a pyrethroid-based compound such as allethrin, d·d-T allethrin, dl·d-T80 allethrin, pyrethrins, phenothrin, flumethrin, cyfluthrin, d·d-T80 prarethrin, phthalthrin, transfluthrin, resmethrin, cyphenothrin, pyrethrum extract, synepirin222, synepirin 500, permethrin, tefluthrin, cypermethrin, deltamethrin, cyhalothrin, fenvalerate, fluvalinate, ethofenprox and silafluofen, a benzoyl urea-based compound such as diflubenzuron, teflubenzuron, flufenoxuron, chlorfluazuron and lufenuron, a juvenile hormone-like compound such as methoprene and a molting hormone-like compound such as chromafenozide. In addition, examples of other compounds include buprofezin, hexythiazox, amitraz, chlordimeform, pyridaben, fenpyroxymate, pyrimidifen, tebufenpyrad, tolfenpyrad, fluacrypyrim, acequinocyl, cyflumetofen, flubendiamide, ethiprole, fipronil, etoxazole, imidacloprid, clothianidin, thiamethoxam, acetamiprid, nitenpyram, thiacloprid, dinotefuran, pymetrozine, bifenazate, spirodiclofen, spiromesifen, flonicamid, chlorfenapyr, pyriproxyfen, indoxacarb, pyridalyl, spinosad, avermectin, milbemycin, cyenopyrafen, spinetoram, pyrifluquinazon, chlorantraniliprole, cyantraniliprole, spirotetramat, lepimectin, metaflumizone, pyrafluprole, pyriprole, hydramethylnon, triazamate, sulfoxaflor, flupyradifurone, flometoquin, pyflubumide, pyrafluprole, ivermectin, selamectin, moxidectin, doramectin, eprinomectin, milbemycin oxime, deet, metoxadiazone, cyromazine, triflumuron, star anise oil, triclabendazole, flubendazole, fenbendazole, antimony sodium gluconate, levamisole hydrochloride, bithionol, dichlorophen, phenothiazine, piperazine-carbon disulfide, piperazine phosphate, piperazine adipate, piperazine citrate, melarsomine dihydrochloride, metyridine, santonin, pyrantel pamoate, pyrantel, praziquantel, febantel, emodepside, emamectin benzoate, cycloxaprid, 1-((6-chloropyridin-3-yl)methyl)-4-oxo-3-phenyl-4H-pyr ido[1,2-a]pyrimidin-1-ium-2-olate, an organic metal-based compound, a dinitro-based compound, an organic sulfur compound, a urea-based compound, a triazine-based compound, and a hydrazine-based compound.

The pest control agent of the present invention may be used in admixture or in combination with a microbial pesticide such as a BT agent, an entomopathogenic viral agent and the like.

Examples of the fungicide used in admixture or in combination include, for example, a strobilurin-based compound such as azoxystrobin, kresoxym-methyl, trifloxystrobin, metominostrobin, and orysastrobin, an anilinopyrimidine-based compound such as mepanipyrim, pyrimethanil and cyprodinil, an azole-based compound such as triadimefon, bitertanol, triflumizole, etaconazole, metoconazole, propiconazole, penconazole, flusilazole, myclobutanil, cyproconazole, tebuconazole, hexaconazole, prochloraz and simeconazole, a quinoxaline-based compound such as quinomethionate, a dithiocarbamate-based compound suchasmaneb, zineb, mancozeb, polycarbamate and propineb, a phenyl carbamate-based compound such as diethofencarb, an organochlorine compound such as chlorothalonil and quintozene, a benzimidazole-based compound such as benomyl, thiophanate-methyl and carbendazole, a phenyl amide-based compound such as metalaxyl, oxadixyl, ofurase, benalaxyl, furalaxyl and cyprofuram, a sulfenic acid-based compound such as dichlofluanid, a copper-based compound such as copper (II) hydroxide and copper oxyquinoline (oxine-copper), an isoxazole-based compound such as hydroxyisoxazole, an organic phosphorus-based compound such as fosetyl-aluminium and tolclofos-methyl, an N-halogenothioalkyl-based compound such as captan, captafol and folpet, a dicarboximide-based compound such as procymidone, iprodione and vinchlozolin, a carboxanilide-based compound such as flutolanil, mepronil, furamepyr, thifluzamide, boscalid, and penthiopyrad, a morpholine-based compound such as fenpropimorph and dimethomorph, an organic tin-based compound such as fentin hydroxide and fentin acetate, a cyanopyrrole-based compound such as fludioxonil and fenpiclonil, and other examples include tricyclazole, pyroquilon, carpropamid, diclocymet, fenoxanil, fthalide, fluazinam, cymoxanil, triforine, pyrifenox, fenarimol, fenpropidin, pencycuron, ferimzone, cyazofamid, iprovalicarb, benthiavalicarb-isopropyl, iminoctadin-albesilate, cyflufenamid, kasugamycin, validamycin, streptomycin, oxolinic-acid, tebufloquin, probenazole, tiadinil, and isotianil.

Examples of the herbicide used in admixture or in combination include lipid synthesis inhibitors, acetolactic acid synthesis inhibitors, photosynthesis inhibitors, protoporphyrinogen IX oxidation inhibitors, bleaching herbicides, amino acid synthesis inhibitors, dihydropteroate synthase inhibitors, cell division inhibitors, very long chain fatty acid synthesis inhibitors, cellulose biosynthesis inhibitors, uncouplers, auxin-like herbicides, auxin transport inhibitors and the like. Specific examples include alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl ester, metamifop, pinoxaden, profoxydim, propaquizafop, quizalofop, quizalofop-ethyl, quizalofop-tefuryl,

quizalofop-P, quizalofop-P-ethyl, quizalofop-P-tefuryl, sethoxydim, tepraloxydim, tralkoxydim, benfuresate, butylate, cycloate, dalapon, dimepiperate, ethyl dipropylthiocarbamate (EPTC), esprocarb, ethofumesate, flupropanate, molinate, orbencarb, pebulate, prosulfocarb, trichloroacetic acid (TCA), thiobencarb, tiocarbazil, triallate, vernolate, sulfonylureas (amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methylsodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl, tritosulfuron), imazamethabenz, imazamethabenz-methyl, imazamox, imazapic, imazapyr, imazaquin, imazethapyr, triazolopyrimidine herbicides (chloransulam, cloransulam-methyl, diclosulam, flumetsulam, florasulam, metosulam, penoxsulam), pyrimisulfan, pyroxsulam, bispyribac, bispyribac-sodium, pyribenzoxim, pyriftalid, pyriminobac, pyriminobac-methyl, pyrithiobac, pyrithiobac-sodium, flucarbazone, flucarbazone-sodium, propoxycarbazone, propoxycarbazone-sodium, thiencarbazone, thiencarbazone-methyl, triazine herbicides (chlorotriazines, triazinones, triazindiones, methylthiotriazines and pyridazinones, such as ametryn, atrazine, chloridazone, cyanazine, desmetryn, dimethametryn, hexazinone, metribuzin, prometon, prometryn, propazin, simazin, simetryn, terbumeton, terbuthylazin, terbutrynandtrietazin), aryl ureas (for example, chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron), phenyl carbamates (for example, desmedipham, karbutilate, phenmedipham and phenmedipham-ethyl), nitrile herbicides (for example, bromofenoxim, bromoxynil or its salt or ester, and ioxynil or its salt or ester), uracils (for example, bromacil, lenacil and terbacil), bentazon, bentazon-sodium, pyridate, pyridafol, pentanochlor, propanil, photosynthesis inhibitors (for example, diquat, diquat-dibromide, paraquat, paraquat dichloride, paraquat-dimethylsulfate, acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, beflubutamid, diflufenican, fluridone, flurochloridone, flurtamone, norflurazon, picolinafen, aclonifen, amitrole, clomazone, flumeturon, glyphosate and its salt, bialaphos, bilanaphos-sodium, glufosinate, glufosinate-P, glufosinate-ammonium, asulam, dinitroanilines (for example, benfluralin, butralin, dinitramine, ethalfluralin, fluchloralin, oryzalin, pendimethalin, prodiamine and trifluralin), phosphoramidate herbicides (for example, amiprophos, amiprophos-methyl and butamiphos), benzoic acid herbicides (for example, chlorthal and chlorthal-dimethyl), pyridines (for example, dithiopyr and thiazopyr), benzamides (for example, propyzamide and tebutam), chloroacetamides (for example, acetochlor, alachlor, butachlor, dimethachlor, dimethenamid, dimethenamid-P, metazachlor, metolachlor, metolachlor-S, pethoxamid, pretilachlor, propachlor, propisochlor and thenylchlor), oxyacetanilides (for example, flufenacet and mefenacet), acetanilides (for example, diphenamid, naproanilide and napropamide), tetrazolinones (for example, fentrazamide), anilofos, cafenstrole, fenoxasulfone, ipfencarbazone, piperophos, pyroxasulfone, chlorthiamid, dichlobenil, flupoxam, isoxaben, dinoseb, dinoterb, 4,6-dinitro-o-cresol (DNOC) and its salt, 2,4-D and it salt or ester, 2,4-DB and its salt or ester, aminopyralid and its salts (for example aminopyralid-tris(2-hydroxypropyl)ammonium) and esters of these, benazolin, benazolin-ethyl, chloramben and its salt or ester, chlomeprop, clopyralid and its salt or ester, dicamba and its salt or ester, dichlorprop and its salt or ester, dichlorprop-P and its salt or ester, fluroxypyr and its salt or ester, 2-methyl-4-chlorophenoxyacetic acid (MCPA) and its salt or ester, MCPA-thioethyl, 4-(2-methyl-4-chlorophenoxy)butyric acid (MCPB) and its salt or ester, mecoprop and its salt or ester, mecoprop-P and its salt or ester, picloram and its salt or ester, quinclorac, quinmerac, 2,3,6-trichlorobenzoic acid (TBA(2, 3, 6)) and its salt or ester, triclopyr and its salt or ester, aminocyclopyrachlor and its salt or ester, diflufenzopyr and its salt, naptalam and its salt, bromobutide, chlorflurenol, chlorflurenol-methyl, cinmethylin, cumyluron, dalapon, dazomet, difenzoquat, difenzoquat-methyl sulfate, dimethipin, disodium methanearsonate (DSMA), dymron, endothal and its salt, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin, methyl azide, methyl bromide, methyl-dymron, methyl iodide, MSMA, oleic acid, oxaziclomefone, pelargonic acid, pyributicarb, quinoclamine, triaziflam, tridiphane, and 6-chloro-3-(2-cyclopropyl-6-methylphenoxy)-4-pyridazin ol (CAS 499223-49-3) and its salt or ester.

Examples of the agent for controlling insect parasites of animals used in admixture or in combination include organic phosphate ester compounds, carbamate compounds, nereistoxin compounds, organochlorine compounds, pyrethroid compounds, benzoyl urea compounds, juvenile hormone-type compounds, molting hormone-type compounds, neonicotinoid compounds, nerve cell sodium channel blockers, insecticidal macrolactones, gamma-aminobutyric acid (GABA) antagonists, ryanodine receptor agonists, insecticidal ureas and the like. More desirable specific examples are dichlorvos, EPN, fenitrothion, fenamifos, prothiofos, profenofos, pyraclofos, chlorpyrifos-methyl, diazinon, trichlorfon, tetrachlorvinphos, bromofenofos, cythioate, fenthion and other organic phosphate ester compounds; methomyl, thiodicarb, aldicarb, oxamyl, propoxur, carbaryl, fenobucarb, ethiofencarb, fenothiocarb, pirimicarb, carbofuran, benfuracarb and other carbamate compounds; cartap, thiocyclam and other nereistoxin compounds; dicofol, tetradifon and other organochlorine compounds; allethrin, d·d-T allethrin, dl·d-T80 allethrin, pyrethrins, phenothrin, flumethrin, cyfluthrin, d·d-T80 prarethrin, phthalthrin, transfluthrin, resmethrin, cyphenothrin, pyrethrum extract, synepirin 222, synepirin 500, permethrin, tefluthrin, cypermethrin, deltamethrin, cyhalothrin, fenvalerate, fluvalinate, ethofenprox, silafluofen and other pyrethroid compounds; diflubenzuron, teflubenzuron, flufenoxuron, chlorfluazuron, lufenuron and other benzoyl urea compounds; methoprene and other juvenile hormone-type compounds; and chromafenozide and other molting hormone-type compounds. Examples of other compounds include amitraz, chlordimeform, fipronil, etoxazole, imidacloprid, clothianidin, thiamethoxam, acetamiprid, nitenpyram, thiacloprid, dinotefuran, spirodiclofen, pyriproxyfen, indoxacarb, spinosad, spinetoram, avermectin, milbemycin, metaflumizone, pyrafluprole, pyriprole, hydramethylnon, triazamate, sulfoxaflor, flupyradifurone, ivermectin, selamectin, moxidectin, doramectin, eprinomectin, milbemycin oxime, diethylcarbamazine citrate, deet, metoxadiazone, cyromazine, triflumuron, star anise oil, triclabendazole, flubendazole, fenbendazole, antimony sodium gluconate, levamisole hydrochloride, bithionol, dichlorophen, phenothiazine, piperazine carbon bisulfide, piperazine phosphate, piperazine adipate, piperazine citrate, melarsomine dihydrochloride, metyridine, santonin, pyrantel pamoate, pyrantel, praziquantel, febantel, emodepside, derquantel, monepantel, emamectin benzoate, cycloxaprid, and a compound represented by the following Formula (VI), or acid addition salts of these that are allowable as agricultural and veterinary chemicals. Examples of acid addition salts of these include hydrochloride salts, nitrate salts, sulfate salts, phosphate salts or acetate salts or the like.

[Synthesis Methods of Compounds of the Invention, precursors thereof and comparative compounds]

A compound represented by the following Formula (I-1)

[Chemical Formula 24]

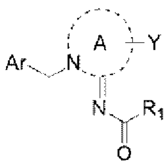

(I−1)

may be obtained by reacting a compound represented by the following Formula (II-1) with a compound represented by ArCH2X [the definition of Ar, A, Y and R1 have the same meaning as described above, and X represents a halogen atom or OTs, OMs and the like] in the presence or absence of a base.

[Chemical Formula 25]

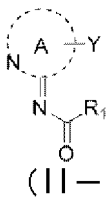

(II−1)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene,

xylene and toluene, alcohols such as methanol, ethanol, propanol and isopropyl alcohol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but N,N-dimethylformamide and the like are preferably used.

The reaction may be performed usually at from 0°C to 200°C, and it is preferred that reagents are added at from 20°C to 40°C and the reaction is performed at from 60°C to 80°C.

The compound represented by Formula (II-1) may be obtained by reacting a compound represented by R1-C (=O)X, R1-C (=O) OC(=O)R1, R1C(=O)OR' [X represents a halogen atom or OTs, OMs and the like, R' represents a C1 to C6 alkyl group, and the definition of R1, A and Y has the same meaning as the definition described above] and the like with a compound represented by in the following Formula (III) in the presence or absence of a base.

[Chemical Formula 26]

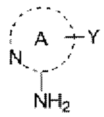

(III)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like as the base.

The reaction may be performed without a solvent or using a solvent which does not affect the reaction. When the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol and propanol, ketones such as acetone and methyl ethyl ketone, aliphatic hydrocarbons such as hexane, heptane and octane, halogen hydrocarbons such as dichloromethane, chloroform, chlorobenzene and dichlorobenzene, and water, either alone or in combination of two or more thereof, but toluene, N,N-dimethylformamide, acetonitrile, ethers, dichloromethane, chloroform and the like are preferably used.

The reaction may be performed usually at from -80°C to 100°C, and is performed preferably in a range from 20°C to 50°C.

The compound represented by Formula (II-1) may be obtained by reacting the compound represented by the Formula (III) with a carboxylic acid represented by R1-COOH [the definition of R1 has the same meaning as the definition described above] using a dehydration condensation agent in the presence or absence of a base, or may be obtained by performing the reaction using phosphorus pentaoxide, sulfuric acid, polyphosphoric acid, thionyl chloride, phosphorus oxychloride and oxalyl dichloride in the absence of a base.

It is possible to use a carbodiimide-based compound such as dicyclohexylcarbodiimide, 1-ethyl-3-(3-dimethylaminopropyl)carbodiimide hydrochloride and the like as the dehydration condensation agent.

When the reaction is performed in the presence of a base, it is possible to use, for example, a carbonate such as potassium carbonate or sodium carbonate and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction is preferably performed by using a solvent, and it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but N,N-dimethylformamide, acetonitrile, ethers, dichloromethane, chloroform and the like are preferably used.

The reaction may be performed usually at from -80°C to 100°C, and is performed preferably in a range from 20°C to 50°C.

The compound represented by Formula (I-1) may be obtained by reacting a compound represented by R1-C(=O)X, R1-C(=O)OC(=O)R1, R1C(=O)OR' [X represents a halogen atom or OTs, OMs and the like, R' represents a C1 to C6 alkyl group, and the definition of Ar, A, Y and R1 has the same meaning as the definition described above]

and the like with a compound represented by the following Formula (IV) in the presence or absence of a base.

[Chemical Formula 27]

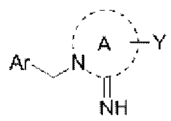

(IV)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction. When a solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether, and tetrahydrofuran, esters such as ethyl acetate and butyl acetate, aromatic hydrocarbons such as benzene, xylene and toluene, alcohols such as methanol, ethanol, and propanol, ketones such as acetone and methyl ethyl ketone, aliphatic hydrocarbons such as hexane, heptane and octane, and halogen hydrocarbons such as dichloromethane, chloroform, chlorobenzene and dichlorobenzene, and water, either alone or in combination of two or more thereof, toluene, N,N-dimethylformamide, acetonitrile, ethers, dichloromethane, chloroform or the like is preferably used.

The reaction may be performed usually at from -80°C to 100°C, and is performed preferably in a range from 20°C to 50°C. The compound represented by Formula (I-1) may be obtained by reacting the above-described compound represented by Formula (IV) with a carboxylic acid represented by R1-COOH [the definition of R1 - has the same meaning as the definition described above] using a dehydration condensation agent in the presence or absence of a base, or may be obtained by performing the reaction using phosphorus pentaoxide, sulfuric acid, polyphosphoric acid, thionyl chloride, phosphorus oxychloride and oxalyl dichloride in the absence of a base.

It is possible to use a carbodiimide-based compound such as dicyclohexylcarbodiimide, 1-ethyl-3-(3-dimethylaminopropyl)carbodiimide hydrochloride and the like as the dehydration condensation agent.

When the reaction is performed in the presence of a base, it is possible to use, for example, a carbonate such as potassium carbonate or sodium carbonate and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction is preferably performed by using a solvent, and it is possible to use, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but dichloromethane, chloroform or the like is preferably used.

The reaction may be performed usually at from -80°C to 100°C, and is performed preferably in a range from 20°C to 50°C. The compound represented by Formula (IV) may be obtained by reacting the above-described compound represented by Formula (III) with a compound represented by ArCH2X [the definition of Ar and X has the same meaning as the definition described above] in the presence or absence of a base.

When the reaction is performed in the presence of a base, it is possible to use, for example, an alkali metal hydride such as sodium hydride, a carbonate such as potassium carbonate or sodium carbonate, an alkali metal hydroxide such as potassium hydroxide and sodium hydroxide, tertiary amines such as triethylamine and 1,8-diazabicyclo[4.3.0]non-5-ene, and unsubstituted or substituent-containing pyridines, such as pyridine and 4-dimethylaminopyridine as the base.

The reaction may be performed without a solvent or using a solvent which does not affect the reaction. When the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol and propanol, ketones such as acetone and methyl ethyl ketone, aliphatic hydrocarbons such as hexane, heptane and octane, and halogen hydrocarbons such as dichloromethane,

chloroform, chlorobenzene and dichlorobenzene, and water, either alone or in combination of two or more thereof, but N,N-dimethylformamide, acetonitrile, ethers, dichloromethane, chloroform and the like is preferably used.

The reaction may be performed usually at from -80°C to 100°C, and is performed preferably in a range from 20°C to 80°C.

When Formula (I-1) is synthesized via Formula (II-1) from the compound represented by Formula (III), or when Formula (I-1) is synthesized via Formula (IV) from the compound represented by Formula (III), the reaction may be continuously performed without taking out Formula (II-1) or Formula (IV), or the reactions from Formula (III) to Formula (I-1) may be simultaneously performed in the same vessel.

[Chemical Formula 28]

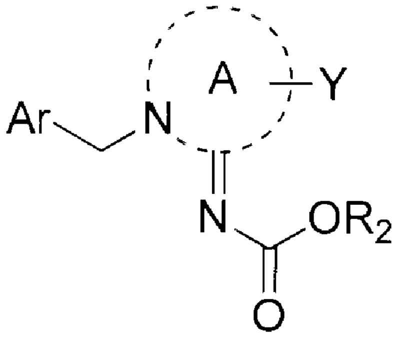

(I-2)

For explanation, the compound represented by Formula (I-2) may be obtained by reacting a compound represented by the following Formula (I-2a) with a compound represented by ArCH2X [the definition of Ar, A, Y and R2 has the same meaning as the definition described above, and X represents a halogen atom or OTs, OMs and the like] in the presence or absence of a base.

[Chemical Formula 29]

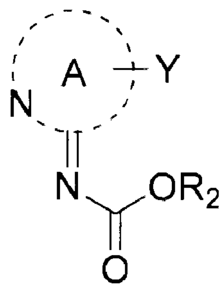

(I-2a)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol, propanol and isopropyl alcohol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but N,N-dimethylformamide and the like are preferably used.

The reaction may be performed usually at from 0°C to 200°C, and it is preferred that reagents are added at from 20°C to 40°C and the reaction is performed at from 60°C to 80°C.

The compound represented by Formula (I-2a) may be obtained by reacting the above-described compound

represented by Formula (III) with a compound represented by R2OC(=O)X (the definition of R2 and X has the same meaning as the definition described above) or represented by the following Formula (I-2b) in the presence or absence of a base.

[Chemical Formula 30]

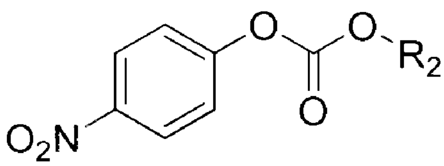

(I－2b)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol, propanol, and isopropyl alcohol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but acetonitrile, dichloromethane, and the like are preferably used.

The reaction may be performed usually at from 0°C to 200°C, and is performed preferably at from 20°C to 80°C.

The compound represented by Formula (I-2) may be obtained by reacting the above-described compound represented by Formula (IV) with a compound represented by R2OC(=O)X (the definition of R2 and X has the same meaning as the definition described above) or represented by the above-described Formula (I-2b) in the presence or absence of a base.

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol, propanol, and isopropyl alcohol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but acetonitrile, dichloromethane, and the like are preferably used.

The reaction may be performed usually at from 0°C to 200°C, and is performed preferably at from 20°C to 80°C.

[Chemical Formula 31]

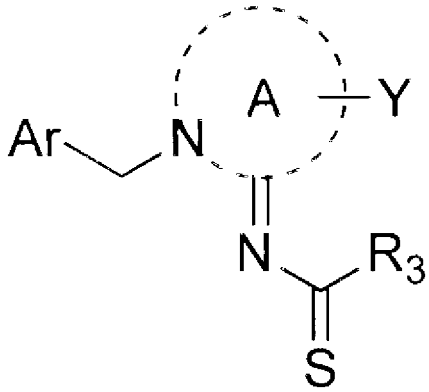

(I-3)

The compound represented by Formula (I-3) may be synthesized by acting a sulfurizing reagent on a compound (the definition of Ar, A, Y and R3 has the same meaning as the definition described above) represented by the following Formula (II-3a), which may be synthesized in the same manner as described in Formula (I-1), in the presence or absence of a base.

[Chemical Formula 32]

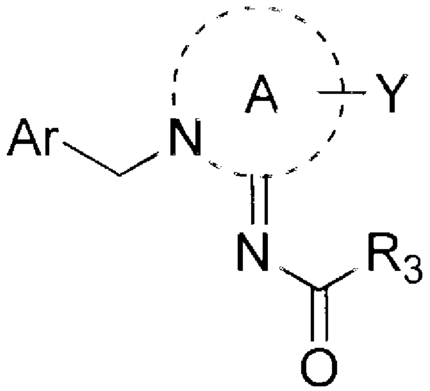

(II-3a)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base, but potassium carbonate, sodium carbonate or the like is preferably used.

As the sulfurizing reagent, phosphorus pentasulfide, Lawesson's reagent, hydrogen sulfide and the like may be used.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol and propanol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but toluene, tetrahydrofuran, and the like are preferably used.

The reaction may be performed usually at from -80°C to 100°C, and is performed preferably in a range from 20°C to 80°C.

The compound represented by Formula (I-3) may be obtained by reacting a compound represented by the following Formula (II-3b) with a compound represented by ArCH2X [the definition of Ar, A, Y and R3 has the same meaning as the definition described above, and X represents a halogen atom or OTs, OMs and the like] in the presence or absence of a base.

[Chemical Formula 33]

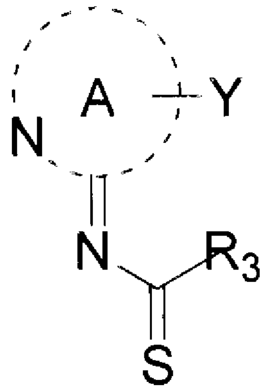

(II-3b)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as triethylamine, 1,8-diazabicyclo[4.3.0]non-5-ene and the like, and unsubstituted or substituent-containing pyridines, such as pyridine, 4-dimethylaminopyridine and the like, as the base.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol propanol, and isopropyl alcohol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but N,N-dimethylformamide is preferably used.

The reaction may be performed usually at from 0°C to 200°C, and it is preferred that reagents are added at from 20°C to 40°C and the reaction is performed at from 60°C to 80°C.

A compound represented by the following Formula (II-3b) may be synthesized by acting a sulfurizing reagent on a compound (the definition of A, Y and R3 has the same meaning as the definition described above) represented by the following Formula (II-3c), which may be synthesized in the same manner as described in Formula (II-1) , in the presence or absence of a base.

[Chemical Formula 34]

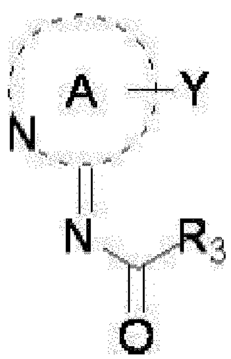

(II-3c)

When the reaction is performed in the presence of a base, it is possible to use, for example, an alkali metal hydride such as sodium hydride and the like, a carbonate such as potassium carbonate or sodium carbonate and the like, an alkali metal hydroxide such as potassium hydroxide, sodium hydroxide and the like, tertiary amines such as

triethylamine, 1,8-diazabicyclo[4.3.0]non-5-ene and the like, and unsubstituted or substituent-containing pyridines, such as pyridine, 4-dimethylaminopyridine and the like, as the base, but potassium carbonate, sodium carbonate or the like is preferably used.

As the sulfurizing reagent, phosphorus pentasulfide, Lawesson's reagent, hydrogen sulfide and the like may be used.

The reaction may be performed without a solvent or using a solvent which does not affect the reaction, and when the solvent is used, it is possible to use solvents such as, for example, amides such as N,N-dimethylformamide and N,N-dimethylacetamide, nitriles such as acetonitrile, sulfoxides such as dimethyl sulfoxide, ethers such as diethyl ether and tetrahydrofuran, esters such as ethyl acetate and butyl acetate, aromatic hydrocarbons such as benzene, xylene and toluene, alcohols such as methanol, ethanol and propanol, ketones such as acetone and methyl ethyl ketone, aliphatic hydrocarbons such as hexane, heptane and octane, and halogen hydrocarbons such as dichloromethane, chloroform, chlorobenzene and dichlorobenzene, either alone or in combination of two or more thereof, but toluene, N,N-dimethylformamide, acetonitrile, ethers, dichloromethane and chloroform are preferably used.

The reaction may be performed usually at from -80°C to 100°C, and is performed preferably in a range from 20°C to 80°C.

Examples

Subsequently, the present invention will be specifically described with reference to Examples, but the present invention is not limited to the Examples. The following refernce examples are not part of the invention, but are descriebed herin for comparative purposes.

**Reference Example 1:**

N-[1-((6-chloropyridine-3-yl)methyl)pyridine-2(1H)-yli dene-2,2,2-trifluoroacetamide (Compound P212)

(1) 25 g (270 mmol) of 2-aminopyridine was dissolved in 200 ml of anhydrous dichloromethane, 41 ml (30 g, 300 mmol) of triethylamine was added thereto, and the mixture was cooled to 0°C. 38 ml (57 g, 270 mmol) of anhydrous trifluoroacetic acid was added dropwise thereto over 15 minutes, and the resulting mixture was stirred at room temperature for 2 hours. After the reaction was completed, the reaction solution was injected into about 100 ml of iced water, and the mixture was stirred for 10 minutes. The mixture was transferred to a separatory funnel to perform liquid separation, and the organic layer was washed twice with 150 ml of water and twice with 150 ml of a 1% HCl aqueous solution, dried over anhydrous magnesium sulfate and concentrated under reduced pressure to obtain 36 g (yield 71%) of 2,2,2-trifluoro-N-(pyridin-2(1H)-ylidene)acetamide.

1H-NMR (CDCl3, δ, ppm) 7.20 (1H, ddd), 7.83 (1H, td), 8.20 (1H, d), 8.35 (1H, d), 10.07 (1H,br)
13C-NMR (CDCl3, δ, ppm) : 115.3, 115.5(q), 121.6, 139.1, 147.9, 149.5, 155.3(q)
MS:m/z=191 (M+H)

(2) 20 g (126 mmol) of 2-chloro 5-chloromethyl pyridine was dissolved in 200 ml of anhydrous acetonitrile, 24 g (126 mmol) of 2,2,2-trifluoro-N-(pyridin-2(1H)-ylidene)acetamide obtained by the above-described method and 21 g (151 mmol) of potassium carbonate were added thereto, and the resulting mixture was heated and refluxed for 6 hours, and then stirred at room temperature for 10 hours. After the reaction was completed, the reaction solution was filtered and the filtrate was concentrated under reduced pressure. Diethyl ether was added thereto for crystallization, and the crystals thus obtained were collected and washed well with diethyl ether and water. The crystals thus obtained were dried under reduced pressure at 60°C for 1 hour to obtain the subject material. Amount obtained 26 g (yield 66%).

1H-NMR (CDCl3, δ, ppm) : 5.57 (2H, s), 6. 92 (1H, td), 7.31 (1H, d), 7.80 (1H, td), 7.87 (1H, dd), 7.99 (1H, dd), 8.48 (2H, m)
13C-NMR (CDCl3, δ, ppm):53.8, 115.5, 117.2(q), 122.1, 124.7,
130.0, 139.2, 140.0, 142.5, 149.7, 151.8, 158.9, 163.5 (q)
MS: m/z=316 (M+H)

(3) Powder X-ray crystal analysis
In thepowderX-ray diffraction, measurement was performed under the following conditions.

Device name: RINT-2200 (Rigaku Corporation)
X-ray: Cu-Ka (40 kV, 20 mA)
Scanning range: 4 to 40°, sampling width: 0.02° and scanning rate: 1°/min
The results are as follows.
Diffraction angle (2θ) 8.7°, 14.2°, 17.5°, 18.3°, 19.8°, 22.4°, 30.9° and 35.3°

(4) Differential Scanning Calorimetry (DSC)
In the differential scanning calorimetry, measurement was performed under the following conditions.

Device name: DSC-60
Sample cell: aluminum
Temperature range: 50°C to 250°C (heating rate: 10°C/min)

As a result, the melting point was observed at 155°C to 158°C.

Another method of Reference Example 1

3.00 g (18.6 mmol) of 2-chloro-5-chloromethyl pyridine was dissolved in 20 ml of anhydrous DMF, 1.75 g (18.6 mmol) of 2-aminopyridine was added thereto, and the resulting mixture was stirred at 80°C for 8 hours and at room temperature for 5 hours. After the reaction was completed, DMF was distilled off under reduced pressure, acetonitrile was added thereto to precipitate a solid, and the solid was collected, washed well with acetonitrile and dried to obtain 2.07 g (yield 44%) of 1-[(6-chloropyridin-3-yl)methyl]pyridin-2(1H)-imine hydrochloride.
1H-NMR (DMSO-d6, δ, ppm) : 5.65(2H, s), 6.96(1H, t), 7.23(1H, m), 7.57(1H, d), 7.80(1H, m), 7.91(1H, m), 8.28(1H, m), 8.49(1H, d), 9.13(2H, brs)
50 mg (0.20 mmol) of the 1-[(6-chloropyridin-3-yl)methyl]pyridin-2(1H)-imine hydrochloride obtained by the above-described method was dissolved in 5 ml of anhydrous dichloromethane, 122 mg (1.00 mmol)of DMAP and 50 mg (0.24 mmol) of anhydrous trifluoroacetic acid were added thereto in sequence under ice cold conditions, and the resulting mixture was stirred at room temperature for 1 hour. After the reaction was completed, the reaction solution was diluted with dichloromethane, washed with 1% hydrochloric acid, and then dried over anhydrous magnesium sulfate. Dichloromethane was distilled off under reduced pressure to obtain the subject material. Amount obtained 42 mg (yield 67%). NMR was the same as that of the above-described method.

**Reference Example 2:**

2,2-dibromo-N-[1-((6-chloropyridin-3-yl)methyl)pyridin -2(1H)-ylidene]-acetamide (Compound P241)

200 mg (0.78 mmol) of the 1-[(6-chloropyridin-3-yl)methyl]pyridin-2(1H)-imine hydrochloride obtained by the method described in another method of Reference Example 1, 238 mg (1.95 mmol) of DMAP and 224 mg (1.17 mmol) of EDC-HCl were dissolved in 10 ml of anhydrous dichloromethane, 101 μl (202 mg, 1.17 mmol) of dibromoacetic acid was added thereto, and the resulting mixture was stirred at room temperature overnight. After the reaction was completed, the reaction solution was diluted with dichloromethane, washed once with water and twice with a 1% HCl aqueous solution, and then dried over anhydrous magnesium sulfate and concentrated under reduced pressure to obtain the subject material. Amount obtained 50 mg (yield 15%)
1H-NMR (CDCl3, δ, ppm) : 5.56(2H, s), 5.99(1H, s), 6.78(1H, td), 7.33(1H, d), 7.69(1H, td), 7.76(1H, dd), 7.93(1H, dd), 8.39(1H, d), 8.50(1H, d)
13C-NMR (CDCl3, δ, ppm) : 44.6, 53.1, 113.7, 121.9, 124.8, 130.1, 138.2, 139.7, 141.2, 149.5, 152.0, 159.4, 172.2

**Reference Example 3:**

N-[1-((6-chloro-5-fluoropyridin-3-yl)methyl)pyridin-2(1H)-y lidene]-2,2,2-trifluoroacetamide (Compound P227)

4.00g (27.6 mmol) of 2-chloro-3-fluoro-5-methyl pyridine was dissolved in 80 ml of carbon tetrachloride, 7.37g (41.4 mmol) of N-bromosuccinimide and 20 mg of benzoyl peroxide were added thereto, and the resulting mixture was heated and refluxed overnight. After the reaction was completed, the reaction solution was returned to room temperature, concentrated under reduced pressure and purified by silica gel column chromatography (hexane: ethyl acetate = 19:1) to obtain 3.06 g (yield 51%) of 5-(bromomethyl)-2-chloro-3-fluoropyridine.
1H-NMR (CDCl3, δ, ppm) : 4.45(2H, s), 7.54(1H, td), 8.23(1H, s)
50 mg (0.22 mmol) of the 5-(bromomethyl)-2-chloro-3-fluoropyridine obtained by the aforementioned method was dissolved in 5 ml of anhydrous acetonitrile, 42 mg (0.22 mmol) of 2,2,2-trifluoro-N-(pyridin-2(1H)-ylidene)acetamide obtained by the method described in (1) of Reference Example 1 and 36 mg (0.26 mmol) of potassium carbonate were added thereto in sequence, and the resulting mixture was heated and refluxed for 7 hours. After the reaction was completed, the reaction solution was returned to room temperature to filter insoluble materials, and the filtrate was concentrated under reduced pressure. Diethyl ether was added thereto to precipitate a solid, and thus the solid was collected, washed with diethyl ether, and then dried under reduced pressure in a desiccator to obtain the subject material. Amount obtained 29 mg (yield 40%).
1H-NMR (CDCl3, $\delta$, ppm) : 5.54 (2H, s), 6.89(1H, td), 7.76(1H, dd), 7.80(1H, td), 7.85(1H, d), 8.29(1H, d), 8.57(1H, d)

**Reference Example 4:**

N-[1-((6-fluoropyridin-3-yl)methyl)pyridin-2(1H)-ylidene]-2 ,2,2-trifluoroacetamide (Compound P229)

500 mg (4.50 mmol) of 2-fluoro-5-methyl pyridine was dissolved in 50 ml of carbon tetrachloride, 1.20 g (6.76 mmol) of N-bromosuccinimide and 20 mg of benzoyl peroxide were added thereto, and the resulting mixture was heated and refluxed for 2.5 hours. After the reaction was completed, the reaction solution was returned to room temperature, and the solvent was distilled off under reduced pressure and purified by silica gel column chromatography (hexane: ethyl acetate = 19:1) to obtain 300 mg (yield 35%) of 5-bromomethyl-2-fluoropyridine.

57 mg (0.30 mmol) of the 5-bromomethyl-2-fluoropyridine obtained by the aforementioned method was dissolved in 10 ml of anhydrous acetonitrile, 57 mg (0.30 mmol) of 2,2,2-trifluoro-N-(pyridin-2(1H)-ylidene)acetamide synthesized by the method described in (1) of Reference Example 1 and 69 mg (0.50 mmol) of potassium carbonate were added thereto in sequence, and the resulting mixture was heated and refluxed for 6 hours. After the reaction was completed, the reaction solution was returned to room temperature to filter insoluble materials, and the filtrate was concentrated under reduced pressure. The filtrate was purified by silica gel column chromatography (hexane: ethyl acetate = 1:1 $\rightarrow$ 3:1) to obtain the subject material. Amount obtained 21 mg (yield 23%).
1H-NMR (CDCl3, $\delta$, ppm) : 5.56 (2H, s), 6.89 (1H, td), 6.94 (1H, d), 7.79 (1H, td), 7.87 (1H, d), 8.03 (1H, m), 8.31 (1H, s), 8.54(1H, d)
MS: m/z = 300(M+H)

**Reference Example 5:**

N-[1-((6-bromopyridin-3-yl)methyl)pyridin-2(1H)-ylidene]-2, 2,2-trifluoroacetamide (Compound P231)

500 mg (2.92 mmol) of 2-bromo-5-methylpyridine was dissolved in 15 ml of carbon tetrachloride, 623 mg (3.50 mmol) of N-bromosuccinimide and 10 mg of benzoyl peroxide were added thereto, and the resulting mixture was heated and refluxed for 19 hours. After the reaction was completed, the reaction solution was returned to room temperature, concentrated under reduced pressure and purified by silica gel column chromatography (hexane: ethyl acetate = 19:1) to obtain 143 mg (yield 20%) of 2-bromo-5-bromomethylpyridine.
1H-NMR (CDCl3, $\delta$, ppm) : 4.42 (2H, s), 7.47(1H, d), 7.59(1H, dd), 8.38(1H, d)

70 mg (0.28 mmol) of the 2-bromo-5-bromomethylpyridine obtained by the aforementioned method was dissolved in 10 ml of anhydrous acetonitrile, 54 mg (0.28 mmol) of 2,2,2-trifluoro-N-(pyridin-2(1H)-ylidene)acetamide synthesized by the method described in (1) of Reference Example 1 and 46 mg (0.34 mmol) of potassium carbonate were added thereto in sequence, and the resulting mixture was heated and refluxed for 6 hours. After the reaction was completed, the reaction solution was returned to room temperature to filter insoluble materials, and the filtrate was concentrated under reduced pressure. Diethyl ether was added thereto to precipitate a solid, and thus the solid was collected, washed with diethyl ether, and then dried under reduced pressure in a desiccator to obtain the subject material. Amount obtained 81 mg (yield 82%).
1H-NMR (CDCl3, $\delta$, ppm) : 5.52 (2H, s), 6.88(1H, t), 7.48 (1H, d), 7.78(2H, m), 7.84(1H, d), 8.44(1H, d), 8.53(1H, d)
MS:m/z = 360(M+H)

**Reference Example 6:**

2-chloro-N-[1-((6-chloropyridin-3-yl)methyl)pyridin-2(1H)-y lidene]-acetamide (Compound P236)

70 mg (0.27 mmol) of the 1-[(6-chloropyridin-3-yl)methyl]pyridin-2(1H)-imine hydrochloride obtained by the method described in another method of Reference Example 1 was dissolved in 4 ml of anhydrous dichloromethane, 82 mg (0.67 mmol) of DMAP, 25mg (0.27 mmol) of chloroacetic acid and 62 mg (0.32 mmol) of EDC-HCl were added thereto in sequence, and the resulting mixture was stirred at room temperature overnight. After the reaction was completed, dichloromethane was added thereto to dilute the mixture, and the mixture was washed with water and a 1% HCl aqueous solution, dried over anhydrous magnesium sulfate and concentrated under reduced pressure to obtain the subject material. Amount obtained 4 mg (yield 5%).
1H-NMR (CDCl3, δ, ppm) : 4. 17 (2H, s), 5.46(2H, s), 6.64(1H, td), 7.31(1H, d), 7.60(1H, td), 7.64(1H, dd), 7.80(1H, dd), 8.32(1H, d), 8.45(1H, d)
MS:m/z = 296(M+H)

**Reference Example 7:**

N-[1-((6-chloropyridin-3-yl)methyl)pyridin-2(1H)-ylidene]-2 ,2-difluoroacetamide (Compound P238)

400 mg (4.26 mmol) of 2-aminopyridine was dissolved in 10 ml of anhydrous dichloromethane, 322 μl (490 mg, 5.11 mmol) of difluoroacetic acid, 982 mg (5.10 mmol) of EDC-HCl and 622 mg (5.11 mmol) of DMAP were added thereto, and the resulting mixture was stirred at room temperature for 61 hours. After the reaction was completed, the reaction solution was diluted with dichloromethane, washed once with water and twice with a 1% HCl aqueous solution, and then dried over anhydrous magnesium sulfate and concentrated under reduced pressure to obtain 102 mg (yield 14%) of 2,2-difluoro-N-(pyridin-2(1H)-ylidene)acetamide.
1H-NMR (CDCl3, δ, ppm) : 6.03(1H, t), 7.15(1H, m), 7.78(1H, td), 8.20(1H, d), 8.34(1H, dd), 8.72(1H, br s)
100 mg (0.58 mmol) of the 2,2-difluoro-N-(pyridin-2(1H)-ylidene)acetamide obtained by the aforementioned method was dissolved in 10 ml of anhydrous acetonitrile, 94 mg (0.58 mmol) of 2-chloro-5-chloromethylpyridine was dissolved in 5 ml of anhydrous acetonitrile and added thereto, and subsequently, 84 mg (0.63 mmol) of potassium carbonate was added thereto and the resulting mixture was heated and refluxed for 140 minutes. After the reaction was completed, the reaction solution was returned to room temperature to filter off insoluble materials, and the filtrate was concentrated under reduced pressure. Ether was added thereto to precipitate a solid, and thus the solid was collected and dried well to obtain the subject material. Amount obtained 63 mg (yield 37%).
1H-NMR (CDCl3, δ, ppm) : 5.52(2H, s), 5.90(1H, t), 6.79(1H, td), 7.33(1H, d), 7.71(1H, m), 7.77(1H, dd), 7.85(1H, dd), 8.45(1H, d), 8.50(1H, d)
13C-NMR (DMSO-d6, δ, ppm): 53.0, 111.0(t), 115.2, 120.7, 124.7, 131.7, 140.6, 141. 6, 143.2, 150.4, 150.9, 158.3, 169.4(t)
MS:m/z = 298 (M+H)

**Reference Example 8:**

2-chloro-N-[1-((6-chloropyridin-3-yl)methyl)pyridin-2(1H)-y lidene]-2,2-difluoroacetamide (Compound P239)

200 mg (2.13 mmol) of 2-aminopyridine was dissolved in 5 ml of dichloromethane, 491 mg (2.55 mol) of EDC-HCl, 311 mg (2.55 mmol) of DMAP and 187 μl (2.23 mmol, 290 mg) of chlorodifluoroacetic acid were added thereto in sequence, and the resulting mixture was stirred overnight. After the reaction was completed, the reaction solution was diluted with dichloromethane, washed with water and 1% hydrochloric acid, and then dried over anhydrous magnesium sulfate to obtain 105 mg (yield 24%) of 2-chloro-2,2-difluoro-N-(pyridin-2(1H)-ylidene)acetami de.
1H-NMR (CDCl3, δ, ppm) : 7.19(1H, dd), 7.82(1H, m), 8.18(1H, d), 8.36(1H, d), 9.35(1H, brs)
53 mg (0.33 mmol) of 2-chloro-5-chloromethyl pyridine dissolved in 6 ml of anhydrous acetonitrile was added to 68 mg (0.33 mmol) of the 2-chloro-2,2-difluoro-N-(pyridin-2(1H)-ylidene)acetami de synthesized by the aforementioned method, and subsequently, 50 mg (0.36 mmol) of potassium carbonate was added thereto and the resulting mixture was heated and refluxed for 1 hour. After the reaction was completed, the reaction solution was returned to room temperature and then concentrated under reduced pressure. Diethyl ether was added thereto to precipitate a solid, and thus the solid was collected and dried to obtain the subject material. Amount obtained 49 mg (yield 45%).
1H-NMR (CDCl3, δ, ppm) : 5.56(2H, s), 6.92(1H, t), 7.33(1H, d), 7.82(1H, m), 7.91(1H, dd), 8.02(1H, d), 8.45(1H, d), 8.48(1H, d)
13C-NMR (CDCl3, δ, ppm): 53.8, 115.2, 120.1 (t), 122.1, 124.8, 139.0, 140.0, 142.3, 150.0, 151.9, 159.1, 159.1, 165.8(t)
MS:m/z = 332(M+H)

**Reference Example 9:**

2,2,2-trichloro-N-[1-((6-chloropyridin-3-yl)methyl)pyridin-2(1H)-ylidene]-acetamide (Compound P235)

70 mg (0.27 mmol) of the 1-[(6-chloropyridin-3-yl)methyl]pyridin-2(1H)-imine hydrochloride obtained by the method described in another method of Reference Example 1 was dissolved in 4 ml of anhydrous dichloromethane, 94

μl (0.68 mmol, 68 mg) of triethylamine and 33 μg (0.27 mmol, 49 mg) of trichloroacetyl chloride were added thereto in sequence, and the resulting mixture was stirred at room temperature for 1 hour. After the reaction was completed, water was added thereto to stop the reaction and liquid separation was performed with dichloromethane and water. The organic layer was washed once with water and twice with 1% hydrochloric acid, dried over anhydrous magnesium sulfate and concentrated under reduced pressure. Diethyl ether was added thereto to precipitate a solid, and thus the solid was collected and dried to obtain the subject material. Amount obtained 61 mg (yield 62%).
1H-NMR (CDCl3, δ, ppm) : 5.59(2H, s), 6.86(1H, t), 7.32(1H, d), 7.78(1H, td), 7.91(2H, m), 8.43(1H, d), 8.50(1H, d)
MS:m/z = 364(M+H)

**Reference Example 10:**

N-[1-((6-chloropyridin-3-yl)methyl)pyridin-2(1H)-ylidene]-2 ,2,3,3,3-pentafluoropropanamide (Compound P242)

300 mg (3.19 mmol) of 2-aminopyridine was dissolved in 15 ml of anhydrous dichloromethane, 919 mg (4.78 mol) of EDC-HCl, 583 mg (4.78 mmol) of DMAP and 397 μl (628 mg, 3.83 mmol) of pentafluoropropionic acid were added thereto in sequence, and the resulting mixture was stirred at room temperature overnight. After the reaction was completed, the reaction solution was diluted with dichloromethane, washed once with water and twice with 1% hydrochloric acid, and then dried over anhydrous magnesium sulfate and concentrated under reduced pressure to obtain 85 mg (yield 11%) of 2,2,3,3,3-pentafluoro-N-(pyridin-2(1H)-ylidene)propana mide.

52 mg (0.32 mmol) of 2-chloro-5-chloromethylpyridine dissolved in 8 ml of anhydrous acetonitrile and 49 mg (0.35 mmol) of potassium carbonate were added to 77 mg (0.32 mmol) of the 2,2,3,3,3-pentafluoro-N-(pyridin-2(1H)-ylidene)propana mide obtained by the aforementioned method, and the resulting mixture was heated and refluxed for 11 hours. After the reaction was completed, the reaction solution was returned to room temperature to filter insoluble materials, and the filtrate was concentrated under reduced pressure. The filtrate was purified by silica gel column chromatography (hexane: ethyl acetate = 1:3) to obtain the subject material. Amount obtained 12 mg (yield 10%).
1H-NMR (CDCl3, δ, ppm) : 5.56(2H, s), 6.90(1H, td), 7.32(1H, d), 7.79(2H, m), 7.84(1H, d), 8.43(1H, d), 8.56(1H, d)
MS:m/z = 366(M+H)

**Reference Example 11:**

N-[1-((2-chloropyrimidin-5-yl)methyl)pyridin-2(1H)-ylidene] -2,2,2-trifluoroacetamide (Compound P243)

1.04 g (8.13 mmol) of 2-chloro-5-methyl pyrimidine was dissolved in 30 ml of carbon tetrachloride, 1.73 g (9.75 mmol) of N-bromosuccinimide and 20 mg of benzoyl peroxide were added thereto, and the resulting mixture was heated and refluxed for 6 hours. After the reaction was completed, the reaction solution was returned to room temperature, concentrated under reduced pressure and purified by silica gel column chromatography (hexane: ethyl acetate = 3:1) to obtain 641 mg (yield 38%) of 5-bromomethyl-2-chloropyridine.
1H-NMR (CDCl3, δ, ppm): 4.42(2H, s), 8.66(2H, s)

104 mg (0.50 mmol) of the 5-bromomethyl-2-chloropyridine obtained by the aforementioned method was dissolved in 6 ml of anhydrous acetonitrile, 96 mg (0.50 mmol) of 2,2,2-trifluoro-N-(pyridin-2(1H)-ylidene)acetamide obtained by the method described in (1) of Reference Example 1 and 76 mg (0.55 mmol) of potassium carbonate were added thereto, and the resulting mixture was heated and refluxed for 1 hour. After the reaction was completed, the reaction solution was returned to room temperature to filter off insoluble materials, and the filtrate was concentrated under reduced pressure. Diethyl ether was added thereto to precipitate a solid, and thus the solid was collected, washed with diethyl ether, and then dried under reduced pressure in a desiccator to obtain the subject material. Amount obtained 92 mg (yield 58%).
1H-NMR (CDCl3, δ, ppm) : 5.54 (2H, s), 6.98(1H, m), 7.87(1H, m), 8.18(1H, m), 8. 48(1H, m), 8.83(2H, m)
13C-NMR (CDCl3, δ, ppm): 60.0, 115.6, 117.1(q), 122.1, 127.5, 139.2, 142.9, 158.8, 160.3(2C), 161.4, 163.8(q)
MS:m/z = 317(M+H)

Compounds P213 to P226, P228, P230, P232 to P234, P240 and P244 described in the Tables 1 and 2 were obtained in the same manner as in Reference Examples 1 to 11.

**Synthetic Example 1:** (Reference Example - for explanation)

2,2-difluoro-N-[1-((6-fluoropyridin-3-yl)methyl)pyridin-2(1 H)-ylidene]acetamide (Compound 3-3)

[Chemical Formula 46]

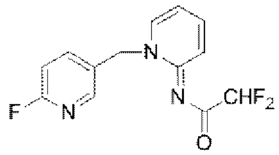

(1) 400 mg (4.26 mmol) of 2-aminopyridine was dissolved in 10 ml of anhydrous dichloromethane, 322 μl (490 mg, 5.11 mmol) of difluoroacetic acid, 982 mg (5.10 mmol) of EDC-HCl and 622 mg (5.11 mmol) of DMAP were added thereto, and the resulting mixture was stirred at room temperature for 61 hours. After the reaction was completed, the reaction solution was diluted with dichloromethane, washed once with water and twice with a 1% HCl aqueous solution, and then dried over anhydrous magnesium sulfate and concentrated under reduced pressure to obtain 102 mg (yield 14%) of 2,2-difluoro-N-(pyridin-2(1H)-ylidene)acetamide.
1H-NMR (CDCl3, δ, ppm) : 6.03(1H, t), 7.15(1H, m), 7.78(1H, td), 8.20(1H, d), 8.34(1H, dd), 8.72(1H, brs)
(2) 128 mg (0.75 mmol) of 5-bromomethyl-2-fluoropyridine was dissolved in 3 ml of anhydrous DMF, 116 mg (0.68 mmol) of 2,2-difluoro-N-[pyridin-2(1H)-ylidene]acetamide was dissolved in 3 ml of anhydrous DMF and added thereto, and subsequently, 103 mg (0.75 mmol) of potassium carbonate was added thereto and the resulting mixture was stirred at 65°C for 2 hours. After the reaction was completed, the reaction solution was returned to room temperature, and ethyl acetate and water were added thereto to perform liquid separation. The organic layer was washed with 1% hydrochloric acid, then dried over anhydrous magnesium sulfate and concentrated under reduced pressure. A small amount of hexane and diethyl ether were added thereto to precipitate crystals, and thus the crystals were collected and dried to obtain the subject material. Amount obtained 50 mg (yield 26%).

**Synthetic Example 2:** (Reference Example - for explanation)

N-[1-((6-chloropyridin-3-yl)methyl)pyrimidin-2(1H)-ylidene] -2,2,2-trifluoroacetamide (Compound 190-2)

[Chemical Formula 47]

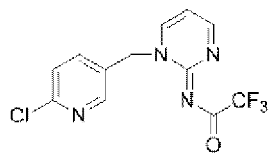

(1) 300 mg (1.86 mmol) of 2-chloro-5-chloromethylpyridine was dissolved in 6 ml of anhydrous DMF, 118 mg (1.24 mmol) of 2-aminopyrimidine was added thereto, and the resulting mixture was stirred at 80°C for 8 hours. After the reaction was completed, the reaction solution was returned to room temperature to distill off DMF under reduced pressure. Diethyl ether was added thereto, and thus crystallization occurred on the wall surface of an eggplant flask. Diethyl ether was removed by decantation and dried well to obtain 1-((6-chloropyridin-3yl)methyl)pyrimidin-2(1H)-imine hydrochloride. Amount obtained 107 mg (yield 34%)
(2) 71 mg (0.27 mmol) of the 1-((6-chloropyridin-3-yl)methyl)pyrimidin-2(1H)-imine hydrochloride obtained by the aforementioned method was suspended in 5 ml of anhydrous dichloromethane, 114 μl (0.83 mmol, 83 mg) of triethylamine and 53 μl (0.38 mmol) of trifluoroacetic anhydride were added thereto in sequence, and the resulting mixture was stirred at room temperature for 2 hours. After the reaction was completed, dichloromethane and water were added to the reaction solution to perform liquid separation, and the organic layer was dried over anhydrous magnesium sulfate and then concentrated under reduced pressure. A small amount of diethyl ether was added thereto to precipitate crystals, and thus the crystals were collected, washed with a small amount of diethyl ether, and then dried to obtain the subject material. Amount obtained 24 mg (yield 28%).

**Synthetic Example 3:** (Reference Example - for explanation)

2,2,2-trifluoroethyl-[1-((6-chloropyridin-3-yl)methyl)pyrid in-(2H)-ylidene]carbamate (Compound 1-17)

[Chemical Formula 48]

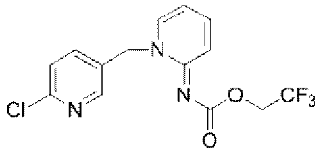

(1) 3.00 g (18.6 mmol) of 2-chloro-5-chloromethylpyridine was dissolved in 20 ml of anhydrous DMF, 1.75 g (18.6 mmol) of 2-aminopyridine was added thereto, and the resulting mixture was stirred at 80°C for 8 hours and at room temperature for 5 hours. After the reaction was completed, DMF was distilled off under reduced pressure, acetonitrile was added thereto to precipitate a solid, and the solid was collected, washed well with acetonitrile and then dried to obtain 2.07 g (yield 44%) of 1-[(6-chloropyridin-3-yl)methyl]pyridin-2(1H)-imine hydrochloride.

1H-NMR (DMSO-d6, $\delta$, ppm): 5.65(2H, s), 6.96(1H, t), 7.23(1H, m), 7.57(1H, d), 7.80(1H, m), 7.91(1H, m), 8.28(1H, m), 8.49(1H, d)

(2) 10 ml of anhydrous acetonitrile was added to 150 mg (0.66 mmol) of the 1-[(6-chloropyridin-3-yl)methyl]pyridin-2(1H)-imine hydrochloride obtained by the aforementioned method, 177 mg (0.66 mmol) of 4-nitrophenyl(2,2,2-trifluoroethyl)carbamate and 200 mg (1.46 mmol) of potassium carbonate were added, and the resulting mixture was stirred at 50°C for 2 hours. After the reaction was completed, the reaction solution was returned to room temperature to filter off insoluble materials, and the filtrate was concentrated under reduced pressure. Dichloromethane and water were added thereto to perform liquid separation, and the organic layer was washed with 1% hydrochloric acid, then dried over anhydrous magnesium sulfate and concentrated under reduced pressure. A small amount of diethyl ether was added thereto to precipitate crystals, and thus the crystals were collected and dried well to obtain the subject material. Amount obtained 48 mg (yield 21%).

**Synthetic Example 4:**

N-[1-((6-chloropyridin-3-yl)methyl)pyridin-2(1H)-ylidene]-2 ,2,2-trifluoroethanethioamide (Compound 1-20)

[Chemical Formula 49]

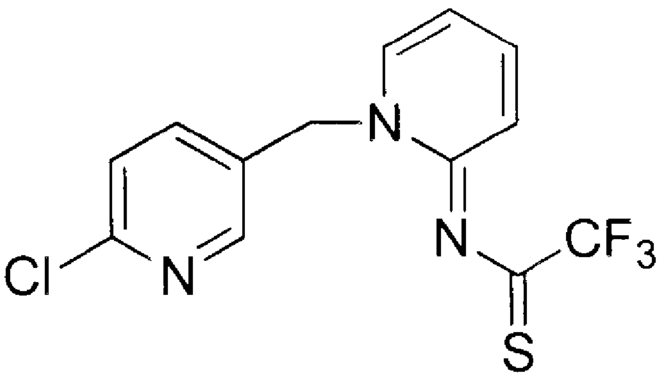

(1) 25 g (270 mmol) of 2-aminopyridine was dissolved in 200 ml of anhydrous dichloromethane, 41 ml (30 g, 300 mmol) of triethylamine was added thereto, and the mixture was cooled to 0°C. 38 ml (57 g, 270 mmol) of anhydrous trifluoroacetic acid was added dropwise thereto over 15 minutes, and the resulting mixture was stirred at room temperature for 2 hours. After the reaction was completed, the reaction solution was injected into about 100 ml of

iced water, and the mixture was stirred for 10 minutes. The mixture was transferred to a separatory funnel to perform liquid separation, and the organic layer was washed twice with 150 ml of water and twice with 150 ml of a 1% HCl aqueous solution, dried over anhydrous magnesium sulfate and concentrated under reduced pressure to obtain 36 g (yield 71%) of 2,2,2-trifluoro-N-(pyridin-2(1H)-ylidene)acetamide.
1H-NMR (CDCl3, $\delta$, ppm) : 7.20(1H, m), 7.83(1H, m), 8.20(1H, d), 8.35(1H, d), 10.07(1H, brs)
13C-NMR (CDCl3, $\delta$, ppm): 115.3, 115.5(q), 121.6, 139.1, 147.9, 149.5, 155.3(q)
(2) 20 g (126 mmol) of 2-chloro 5-chloromethylpyridine was dissolved in 200 ml of anhydrous acetonitrile, 24 g (126 mmol) of 2,2,2-trifluoro-N-(pyridin-2(1H)-ylidene)acetamide obtained by the above-described method and 21 g (151 mmol) of potassium carbonate were added thereto, and the resulting mixture was heated and refluxed for 6 hours, and then stirred at room temperature for 10 hours. After the reaction was completed, the reaction solution was filtered and the filtrate was concentrated under reduced pressure. Diethyl ether was added thereto for crystallization, and the crystals thus obtained were collected and washed well with diethyl ether and water. The crystals thus obtained were dried under reduced pressure at 60°C for 1 hour to obtain N-[1-((6-chloropyridin-3-yl)methyl)pyridin-2(1H)-ylide ne]-2,2,2-trifluoroacetamide. Amount obtained 26 g (yield 66%).
1H-NMR (CDCl3, $\delta$, ppm) : 5.57(2H, s), 6.92(1H, td), 7.31(1H, d), 7.80(1H, td), 7.87(1H, dd), 7.99(1H, dd), 8.48(2H, m)
13C-NMR (CDCl3, $\delta$, ppm): 53.8, 115.5, 117.2(q), 122.1, 124.7, 130.0, 139.2, 140.0, 142.5, 149.7, 151.8, 158.9, 163.5(q)
MS:m/z = 316(M+H)
(3) 180 ml of toluene was added to 16.3 g (36.7 mmol) of phosphorus pentasulfide, 6.72 g (63.4 mmol) of sodium carbonate was added thereto and the resulting mixture was stirred at room temperature for 5 minutes. 20.0 g (63.4 mmol) of the N-[1-((6-chloropyridin-3-yl)methyl)pyridin-2(1H)-ylide ne]-2,2,2-trifluoroacetamide obtained by the above-described method was added thereto, and the resulting mixture was stirred at 50°C for 19 hours. 150 ml of ethyl acetate was added to the reaction solution, the resulting mixture was stirred at 50°C for 10 minutes, then insoluble materials were filtered off, and 250 ml of ethyl acetate was used to wash the mixture. The mixture was transferred to a separatory funnel, washed therein with 300 ml of a saturated sodium bicarbonate water and 200 ml of a saturated saline solution, and then concentrated under reduced pressure. 200 ml of water added thereto to precipitate crystals.

The mixture was stirred at room temperature for 1 hour, and then the crystals were collected, subjected to slurry washing twice with 150 ml of water and twice with 150 ml of hexane, and dried at 60°C under reduced pressure for 2 hours to obtain the subject material. Amount obtained 19.5 g (yield 94%).
1H-NMR (CDCl3, $\delta$, ppm) : 5.48 (2H, s), 7.12(1H, td), 7.34(1H, d), 7.77(1H, dd), 7.96(1H, m), 8.05(1H, dd), 8.45(1H, d), 8.56 (1H, d)
MS:m/z = 332(M+H)

The preparation conditions of the compounds obtained in Synthetic Examples 1 to 4 are shown in Tables 41 to 43 and the spectrum data are shown in the following Tables 48 and 49.

Further, the synthetic methods in the Table are described as follows.

A: the same method as in Synthetic Example 1
B: the same method as in Synthetic Example 2
C: the same method as in Synthetic Example 3
D: the same method as in Synthetic Example 4

[Table 41]

| Compound No. | Raw material 1 | Rawmaterial 2 | Base and the like | Solvent | Reaction temperature, Time | Synthetic method | Yield (%) |
|---|---|---|---|---|---|---|---|
| 1-20 | 20.0 g (63.4 mmol) of N-[1-((6-chloropyr idin-3-yl)methyl)p yridin-2(1H)-ylide ne]-2,2,2-trifluor oacetamide | 16.3 g (36.7 mmol) of phosphorus pentasulfide | 6.72 mg (63.4 mmol) of sodium carbonate | Toluene | 50°C, 19 h | D | 94 |

[Table 42-1]

| Compound No. | Raw material 1 | Raw material 2 | Base and the like | Solvent | Reaction temperature, Time | Synthetic method | Yield (%) |
|---|---|---|---|---|---|---|---|
| 1-21 | 23 mg (0.077 mmol) of N-[1-((6-chloropyri din-3-yl)methyl)pyr idin-2(1H)-ylidene] -2,2-difluoroacetam ide | 41 mg (0.092 mmol) of phosphorus pentasulfide | 10 mg (0.092 mmol) of sodium carbonate | THF | Room tempera ture,2 h | D | 49 |
| 3-20 | 30 mg (0.10 mmol) of N-[1-((6-fluoropyri din-3-yl)methyl)pyr idin-2(1H)-ylidene] -2,2,2-trifluoroace tamide | 49 mg (0.11 mmol) of phosphorus pentasulfide | 12 mg (0.11 mmol) of sodium carbonate | THF | Room tempera ture,3h | D | 49 |
| 4-20 | 30 mg (0.083 mmol) of N-[1-((6-bromopyrid in-3-yl)methyl)pyri din-2(1H)-ylidene]-2,2,2-trifluoroacet amide | 41 mg (0.09 mmol) of phosphorus pentasulfide | 10 mg (0.09 mmol) of sodium carbonate | THF | Room tempera ture,3h | D | 61 |

[Table 42-2]

| | | | | | | | |
|---|---|---|---|---|---|---|---|
| 1-22 | 37 mg (0.11 mmol) of 2-chloro-N-[1-((6-c hloropyridin-3-yl)m ethyl)pyridin-2(1H) -ylidene]-2,2-diflu oroacetamide | 49 mg (0.11 mmol) of phosphorus pentasulfide | 12 mg (0.11 mmol) of sodium carbonate | THF | Room tempera ture,4h | D | 31 |
| 1-23 | 31 mg (0.085 mmol) of N-[1-((6-chloropyri din-3-yl)methyl)pyr idin-2(1H)-ylidene] -2,2,3,3,3-pentafluoropropanamide | 38 mg (0.085 mmol) of phosphorus pentasulfide | 9 mg (0.0854 mmol) of sodium carbonate | THF | Room tempera ture,4h | D | 59 |

[Table 42-3]

| | | | | | | | |
|---|---|---|---|---|---|---|---|
| 5-20 | 36 mg (0.11 mmol) of N-[1-((6-chloro-5-f luoropyridin-3-yl)m ethyl)pyridin-2(1H) -ylidene]-2,2,2-tri fluoroacetamide | 49 mg (0.11 mmol) of phosphorus pentasulfide | 12 mg (0.11 mmol) of sodium carbonate | THF | Room tempera ture,4h | D | 10 0 |

[Table 43-1]

| Compound No. | Raw material 1 | Raw material 2 | Base and the like | Solvent | Reaction temperature, Time | Synthetic method | Yield (%) |
|---|---|---|---|---|---|---|---|
| 2-20 | 70 mg (0.22 mmol) of N-[1-((2-chlorothiazol-5-yl)methyl)pyrid in-2(1H)-ylidene]-2, 2,2-trifluoroacetamide | 107 mg (0.24 mmol) of phosphorus pentasulfide | 25 mg (0.24 mmol) of sodium carbonate | THF | Room temperature, 4h | D | 11 |
| 10-20 | 130 mg (0.37 mmol) of 2,2,2-trifluoro-N-[1 -((6-trifluoromethyl )pyridin-3-yl)methyl ]pyridin-2(1H)-ylidene]-acetamide | 181 mg (0.41 mmol) of phosphorus pentasulfide | 43 mg (0.41 mmol) of sodium carbonate | THF | Room temperature,4h | D | 93 |

[Table 43-2]

| | | | | | | | |
|---|---|---|---|---|---|---|---|
| 11-20 | 40 mg (0.15 mmol) of 2,2,2-trifluoro-N-[1 -((tetrahydrofuran-3 -yl)methyl]pyridin-2(1H)-ylidene))acetamide | 65 mg (0.11 mmol) of phosphorus pentasulfide | 16 mg (0.15 mmol) of sodium carbonate | THF | Room tempera ture, 4h | D | 53 |
| 1-37 | 78 mg (0.28 mmol) of N-[1-((6-chloropyrid in-3-yl)methyl)pyrid in-2(1H)-ylidene]-pr opionamide | 125 mg (0.28 mmol) of phosphorus pentasulfide | 30 mg (0.28 mmol) of sodium carbonate | THF | Room tempera ture, 2h | D | 21 |
| 1-39 | 180 mg (0.96 mmol) of N-[1-((6-chloropyrid in-3-yl)methyl)pyrid in-2(1H)-ylidene]-is obutylamide | 341 mg (0.75 mmol) of phosphorus pentasulfide | 102 mg (0.96 mmol) of sodium carbonate | THF | Room tempera ture, 2h | D | 29 |
| 1-40 | 54 mg (0.19 mmol) of N-[1-((6-chloropyrid in-3-yl)methyl)pyrid in-2(1H)-ylidene]-cy clopropane carboxyamide | 54 mg (0.19 mmol) of phosphorus pentasulfide | 20 mg (0.19 mmol) of sodium carbonate | THF | Room tempera ture, 2h | D | 12 |

[Table 43-3]

| | | | | | | | |
|---|---|---|---|---|---|---|---|
| 1-35 | 26 mg (0.074 mmol) of N-[1-((6-chloropyridin-3-yl)methyl)pyrid in-2(1H)-ylidene]-3-phyenylpropanamide | 26 mg (0.06 mmol) of phosphorus pentasulfide | 8 mg (0.074 mmol) of sodium carbonate | THF | Room tempera ture, 1. 5h | D | 23 |

[Table 48-1]

| Compound No. | 1H-NMR (CDCl3, δ, ppm) | MS or IR (KBr, v, $cm^{-1}$) |
|---|---|---|
| 1-20 | 5.48 (2H, s), 7.12 (1H, td), 7.34 (1H, d), 7.77 (1H, dd), 7.96 (1H, m), 8.05 (1H, dd), 8.45 (1H, d), 8.56 (1H, d) | m/z = 332 (M+H) |

[Table 48-2]

| | | |
|---|---|---|
| 1-21 | 5.49 (2H, s), 6.21 (1H, t), 7.05 (1H, td), 7.34 (1H, d), 7.82 (1H, dd), 7.90 (1H, m), 7.94 (1H, dd), 8.45 (1H, d), 8.49 (1H, d) | m/z = 314.0346 (M+H) |
| 3-20 | 5.51 (2H, s), 6.95 (1H, d), 7.15 (1H, td), 7.96 (2H, m), 8.09 (1H, d), 8.29 (1H, d), 8.52 (1H, d) | m/z = 316.0559 (M+H) |
| 4-20 | 5.47 (2H, s), 7.13 (1H, m), 7.50 (1H, m), 7.66 (1H, m), 7.97 (1H, m), 8.07 (1H, m), 8.43(1 H,s), 8.54 (1H, m) | m/z = 375.9 (M+H) |
| 1-22 | 5.49 (2H, s), 7.09 (1H, td), 7.35 (1H, d), 7.78 (1H, dd), 7.95 (2H, m), 8.46 (1H, d), 8.55 (1 H, d) | m/z = 347.9972 (M+H) |
| 1-23 | 5.47 (2H, s), 7.10 (1H, td), 7.34 (1H, d), 7.68 (1H, dd), 7.95 (2H, m), 8.41 (1H.d), 8.55 (1 H,dd) | m/z = 382.0246 (M+H) |
| 5-20 | 5.49 (2H, s), 7.10 (1H, m), 7.65 (1H, dd), 7.96 (1H, m), 8.00 (1H, m), 8.27 (1H, d), 8.63 (1H, d) | m/z = 350.0188 (M+H) |

[Table 49-1]

| Compound No. | 1H-NMR (CDCl3, δ, ppm) | MS or IR (KBr, v, $cm^{-1}$) |
|---|---|---|
| 2-20 | 5.57 (2H, s), 7.12 (1H, m), 7.68 (1H, s), 7.97 (1H, m), 8.12 (1H, d), 8.67 (1H, d) | m/z = 338 (M+H) |
| 10-20 | 5.58 (2H, s), 7.12 (1H, m), 7.70 (1H, d), 7.97 (2H, m), 8.02 (1H, 8. 62 (1H, d), 8.77 (1H, s) | m/z = 366 (M+H) |
| 11-20 | 1.69 (1H, m), 2.07 (1H, m), 2.84 (1H, m), 3.59 (1H, dd), 3.71 (1H, dd), 3.77 (1H, m), 3.96 (1H, m), 4.13 (1H, dd), 4.42 (1H, dd), 7.11 (1H, m), 7.92 (1H, dd), 7.98 (1H, m), 8.40 (1H, d) | m/z = 291 (M+H) |

[Table 49-2]

| | | |
|---|---|---|
| 1-37 | 1.28 (3H, t), 2.88 (2H, q), 5.41 (2H, s), 6.86 (1H, t), 7.35 (1H, d), 7.75 (3H, m), 8.10 (1H, d), 8.44 (1H, d) | m/z = 292 (M+H) |
| 1-39 | 1.26 (6H, d), 2.55 (1H, m), 5.51 (2H, s), 6.98 (1H, m), 7.36 (1H, d), 7.76 (1H, dd), 7.77 (2H, m), 8.08 (1H, d), 8.44 (1H, d) | m/z = 306 (M+H) |
| 1-40 | 0.92 (2H, m), 1.22 (2H, m), 2.40 (1H, m), 5.36 (2H, s), 6.77 (1H, td), 7.34 (1H, d), 7.66 (2H, m), 7.71 (1H, dd), 8.14 (1H, d), 8.41 (1H, d) | m/z = 304 (M+H) |
| 1-35 | 3.18 (4H, m), 5.05 (2H, s), 6.83 (1H, td), 7.05 (1H, t), 7.25 (2H, m), 7.38 (3H, m), 7.59 (1H, dd), 7.67 (1H, d), 7.72 (1H, td), 7.99 (1H, d), 8.30 (1 H,d) | m/z = 368 (M+H) |

(Synthetic Example of Comparative Compound)

Comparative Example 1: N-[1-chloropyridine-3-yl]methyl)pyridine-2(1H)-ylidene ]cyanamide (Patent Document 5, Compound 20)

[Chemical Formula 58]

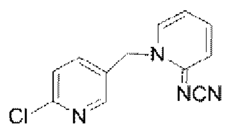

128 mg (0.58 mmol) of the 1-[(6-chloropyridin-3-yl)methyl]pyridine-2(1H)-imine obtained by the above-described method was dissolved in 5 ml of anhydrous DMF, 40 mg (net 24 mg, 1.04 mmol) of NaH (oil phase, purity 60%) was added thereto, and the resulting mixture was stirred at room temperature for 30 minutes. 60 mg (0.57 mmol) of cyanogen bromide was added thereto and the resulting mixture was stirred overnight. After the reaction was completed, water and ethyl acetate were added to the reaction solution to perform liquid separation. The organic layer was dried over anhydrous magnesium sulfate, then concentrated under reduced pressure and purified by a TLC plate (one sheet of 0.5 mm plate, evolved with 100% ethyl acetate) to obtain the subject material. Amount obtained 14 mg (yield 10%).
$^{1}$N-NMR (CDCl3, δ, ppm) : 5.28 (2H, s), 6.55 (1H, m), 7.33 (2H, m), 7.56 (2H, m), 7.75 (1H, dd), 8.40 (1H, d)

Comparative Example 2: N-[1-((6-chloropyridine-3-yl)methyl)pyridine-2(2H)-yli dene]acetamide (Patent Document 3, Compound 2)

[Chemical Formula 59]

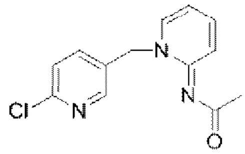

20 ml of anhydrous dichloromethane was added to 118 mg (0.46 mmol) the 1-[(6-[chloropyridin-3-yl)methyl]pyridin-2(1H)-imine hydrochloride obtained by the above-described method, 159 μl (1.16 mmol, 116 mg) of triethylamine and 33 μl of acetyl chloride were added thereto, and the resulting mixture was stirred at room temperature for 15 minutes. Water was added to the reaction solution to stop the reaction and liquid separation was performed with chloroform and water. The organic layer was washed with a saturated ammonium chloride aqueous solution and then concentrated, hexane was added thereto to precipitate a solid, and thus the solid was collected, washed and subjected to bath drying to obtain the subject material. Amount obtained 21 mg (yield 17%).
$^1$N-NMR ($CDCl_3$, δ, ppm) : 2.21 (3H, s), 5.35 (2H, s), 6.46 (1H, m), 7.32 (1H, d), 7.48 (2H, m), 7.75 (1H, d), 8.10 (1H, dd), 8.45 (1H, dd)
MS:m/z = 322 (M+H)

Comparative Example 3:

3-[1-((6-chloropyridine-3-yl)methyl)imidazolidine-2-yl idene]-1,1,1-trifluoropropane-2-on (Patent Document 2, Example 4)

[Chemical Formula 60]

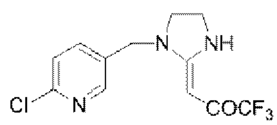

20 ml of ethylenediamine was added to 2.0 g (12.4 mmol) of 2-chloro-5-chloromethylpyridine, and the resulting mixture was stirred overnight. After the reaction was completed, the mixture was concentrated under reduced pressure and acetonitrile was added thereto to filter off insoluble materials. The mixture was concentrated under reduced pressure to obtain 2.45 g (yield: 100%) of N-((6-chloropyridin-3-yl)methyl)ethane-1,2-diamine.

77 mg (0.42 mmol) of the N-((6-chloropyridin-3-yl)methyl)ethan-1,2-diamine obtained by the aforementioned method was dissolved in 8 ml of anhydrous acetonitrile, the resulting solution was added to 60 mg (0.28 mmol) of the 1,1,1-trifluoro-4,4-bis(methylthio)-3-butylen-2-one obtained by the above-described method, and the resulting mixture was heated and refluxed for 40 minutes. After the reaction was completed, the reaction solution was returned to room temperature and concentrated under reduced pressure, and ethyl acetate and water were added thereto to perform liquid separation. The organic layer was washed with anhydrous magnesium sulfate, concentrated under reduced pressure, and purified by silica gel column chromatography (hexane: ethyl acetate = 3:1) to obtain the subject material. Amount obtained 59 mg (yield 69%).
$^1$N-NMR ($CDCl_3$, δ, ppm) : 3.49 (2H, t), 3.78 (2H, t), 4.40 (2H, s), 5.13 (1H, s), 7.37 (1H, d), 7.56 (1H, dd), 8.31 (1H, d), 9.34 (1H, br s)
m/z = 306 (M+H)

Comparative Example 4:

3-[3-((6-chloropyridine-3-yl)methyl)thiazoline-2-ylide ne]-1,1,1-trofluoropropane-2-on (Patent Document 2, Example 3)

[Chemical Formula 61]

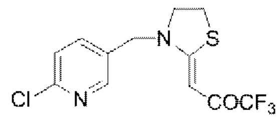

15 ml of anhydrous DMF was added to 1.30 g (33.9 mmol, 780 mg) of NaH (oil phase, purity 60%) and the resulting mixture was cooled to 0°C. 1.52 ml (1.90 g, 17.0 mmol) of 1,1,1-trifluoroacetone was added dropwise thereto, and the resulting mixture was stirred at 0°C for 10 minutes. 7.0 ml (110 mmol, 8.35 g) of carbon disulfide was added thereto and the resulting mixture was stirred at 5°C for 1 hour. Subsequently, the reaction solution was cooled to 0°C, 2.1 ml (34.0 mmol, 4.81 g) of methyl iodide was added thereto, and the resulting mixture was stirred at room temperature overnight. After the reaction was completed, the reaction solution was injected into iced water, and the mixture was stirred until the ice was completely melted. The mixture was transferred to a separatory funnel and extracted with ethyl acetate, and the organic layer was washed with a saturated saline solution, then dried with anhydrous magnesium sulfate and concentrated under reduced pressure. The organic layer was purified by silica gel column chromatography (hexane: ethyl acetate = 95:5) and a fraction including the subject material was collected and concentrated under reduced pressure. Hexane was added thereto to precipitate a solid, and thus the solid was collected, washed with hexane and then dried well to obtain 460 mg of 1,1,1-trifluoro-4,4-bis(methylthio)-3-butene-2-one (yield 13%).
$^1$N-NMR (CDCl3, δ, ppm) : 2.56 (3H, s), 2.58 (2H, s), 6.25 (1H, s)
36 mg (0.46 mmol) of 2-aminoethanethiol dissolved in 10 ml of ethanol was added to 100 mg (0.46 mmol) of the 1,1,1-trifuloro-4,4-bis(methylthio)-3-butene-2-one obtained by the aforementioned method, and the resulting mixture was heated and refluxed for 6 hours, and stirred at room temperature for 13 hours. After the reaction was completed, ethanol was distilled off under reduced pressure, and the mixture was dissolved in ethyl acetate and washed once with water. The mixture was dried over anhydrous magnesium sulfate and then concentrated under reduced pressure to obtain 73 mg (yield 81%) of 1,1,1-trifluoro-3-(thiazolidin-2-ylidene)propan-2-one.
$^1$N-NMR ($CDCl_3$, δ, ppm) : 3.35 (2H, m), 4.02 (2H, m), 5.61 (1H, s), 10.40 (1H, br s)
80 mg (0.50 mmol) of 2-chloro-5-chloromethylpyridine dissolved in 8 ml of anhydrous acetonitrile and 69 mg (0.50 mmol) of potassium carbonate were added to 65 mg (0.33 mmol) of the 1,1,1-trifluoro-3-(thiazolidin-2-ylidene)propan-2-one obtained by the aforementioned method, and the resulting mixture was heated and refluxed for 2 hours. After the reaction was completed, the reaction solution was returned to room temperature to filter off insoluble materials, and the filtrate was concentrated under reduced pressure. The filtrate was purified by silica gel column chromatography (hexane: ethyl acetate = 1:1 → 1:3) to obtain the subject material. Amount obtained 53 mg (yield 50%)
$^1$N-NMR (CDCl3, δ, ppm) : 3.20 (2H, t), 3.73 (2H, t), 4.61 (2H, s), 5.80 (1H, s), 7.36 (1H, d), 7.53 (1H, dd), 8.31 (1H, d)
MS: m/z = 323 (M+H)

Comparative Example 5:

3-[1-((6-chloropyridine-3-yl)methyl)imidazolidine-2-yl idene]-1,1,1,5,5,5-hexafluoropentane-2,4-dione (Patent Document 2, Example 5)

[Chemical Formula 62]

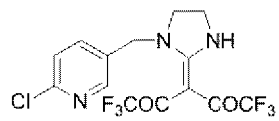

31 mg (0.10 mmol) of the 3-[1-((6-[chloropyridin-3-yl)methyl)imidazolidin-2-yli dene]-1,1,1-trifluoropropan-2-one obtained by the above-described method was dissolved in 2 ml of anhydrous dichloromethane, 20 μl (0.25 mmol, 20 mg) of pyridine and 28 μl (0.20 mmol, 42 mg) of trifluoroacetic anhydride were added in sequence, and the resulting mixture was stirred at room temperature for 30 minutes. The progress of the reaction was confirmed by TLC and the raw material was remaining, and thus 84 μl (0.60 mmol, 62 mg) of trifluoroacetic anhydride was added thereto and the resulting mixture was stirred at room temperature for 1 hour. After the reaction was completed, the reaction solution was concentrated under reduced pressure and purified by a TLC plate (one sheet of 0.5 mm plate, evolved with hexane: ethyl acetate = 2:8) to obtain the subject material. Amount obtained 30 mg (yield 75%).

$^1$N-NMR ($CD_3OD$, δ, ppm): 3.87 (4H, m), 4.51 (2H, s), 7.50 (1H, d), 7.82 (1H, dd), 8.35 (1H, d)
MS: m/z = 402 (M+H)

Comparative Example 6:

N-[1-((6-chloropyridine-3-yl)methyl)imidazolidine-2-yl idene]-2,2,2-trifluoroacetamide (Patent Document 2, Example 7)

[Chemical Formula 63]

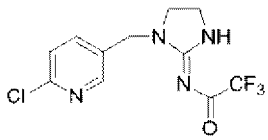

4.61 g (2.49 mmol) of N-((6-chloropyridin-3-yl)methyl)ethane-1,2-diamine was synthesized by the above-described method. The compound was dissolved in 40 ml of anhydrous acetonitrile, 4.60 g (21.3 mmol) of the dimethyl(2,2,2-trifluoroacetyl)carbonimidedithioate obtained by the above-described method was added thereto, and the resulting mixture was heated and refluxed for 90 minutes. After the reaction was completed, the reaction solution was returned to room temperature, the solvent was distilled off under reduced pressure, and the precipitated solid was collected and washed with a small amount of acetonitrile to obtain the subject material. Amount obtained 2.17 g (yield 33%).
$^1$N-NMR (CDCl3, δ, ppm) : 3.50 (2H, m), 3.76 (2H, m), 4.60 (2H, s), 7.34 (1H, d) 7.70 (1H, dd) 8.33 (1H, d)
Melting Point: 168-170°C

Comparative Example 7:

N-[3-((6-chloropyridine-3-yl)methyl)thiazoline-2-ylide ne]-2,2,2-trifluoroacetamide (Patent Document 2, Example 6)

[Chemical Formula 64]

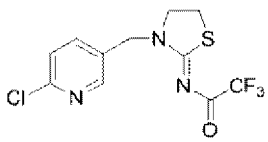

20 ml of ethanol was added to 77 mg (1.0 mmol) of 2-aminoethanethiol, 216 mmol (1.0 mmol) of the dimethyl(2,2,2-trifluoroacetyl)carbonimidedithioate synthesized by the above-described method was added thereto, and the resulting mixture was stirred at room temperature overnight. After the reaction was completed, the solvent was distilled off under reduced pressure and purified by silica gel column chromatography (hexane: ethyl acetate = 1:1) to obtain 100 mg (yield 51%) of 2,2,2-trifluoro-N-(thiazolidin-2-ylidene)acetamide. The reaction was perfomed again by the same synthetic method, and 2,2,2-trifluoro-N-(thiazolidin-2-ylidene)acetamide was put together to obtain 350 mg of the compound.

2 ml of DMF and 18 ml of THF were added to 162 mg (0.82 mmol) of the 2,2,2-trifluoro-N-(thiazolidin-2-ylidene)acetamide obtained by the above-described method, 150 mg (1.09 mmol) of potassium carbonate was added thereto, and the resulting mixture was heated and refluxed for 20 hours. After the reaction was completed, the reaction solution was returned to room temperature to filter insoluble materials, and the filtrate was concentrated under reduced pressure. The filtrate was purified by TLC plates (two sheets of 0.5 mm plates, evolved with 100% ethyl acetate) to obtain the subject material. Amount obtained 230 mg (yield 87%).
$^1$N-NMR (CDCl3, δ, ppm) : 3.27 (2H, m), 3.73 (2H, m), 4.86 (2H, s), 7.36 (1H, d) 7.72 (1H, dd) 8.36 (1H, d)
Melting Point: 96°C

Comparative Example 8:

1-[1-((6-chloropyridine-3-yl)methyl)pyridine-2(1H)-yli dene]-3-ethylthiourea (Patent Document 3, Japanese Patent Application Laid-Open No. 5-78323, Table 1, Compound No. 51)

[Chemical Formula 65]

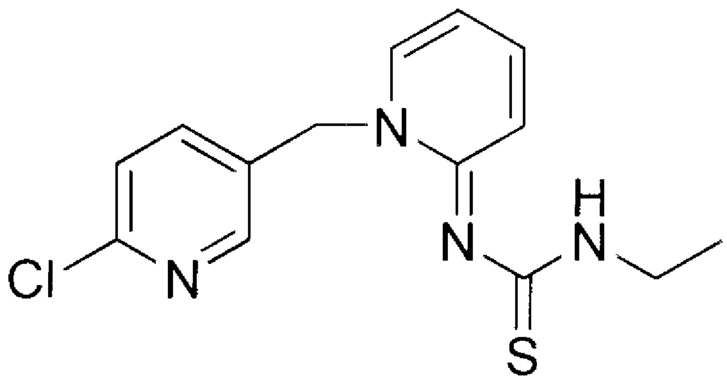

10 ml of acetonitrile was added to 200 mg (0.78 mmol) of 1-[(6-chloropyridin-3-yl)methyl]pyridin-2(1H)-imine hydrochloride synthesized by the method described in Synthetic Example 3, 118 μl (0.86 mmol) of triethylamine and 68 μl (0.78 mmol) of ethyl isothiocyanate were added thereto in sequence, and the resulting mixture was heated and refluxed for 11 hours. After the reaction was completed, the reaction solution was returned to room temperature and concentrated under reduced pressure, and liquid separation was performed with ethyl acetate and a 1% hydrochloric acid aqueous solution. A saturated sodium bicarbonate water was added to the water layer to make the layer basic, and the layer was extracted once with ethyl acetate. The layer was dried over anhydrous magnesium sulfate and then concentrated under reduced pressure to obtain a subject compound. Amount obtained 120 mg (yield 56%).
1H-NMR (CDCl3, δ, ppm) : 1.06, 1.23 (3H, tx2), 3.21, 3.71 (2H, mx2), 5.23, 5.32 (2H, sx2), 6.25, 6.42 (1H, br sx2), 6.37, 6.51 (1H, mx2), 7.31-7.37 (2H, m), 7.47 (1H, m), 7.62 (1H, m), 8.14-8.22 (1H, m), 8.35 (1H, m)
MS: m/z= 307 (M+H)
Melting point: 162-164°C

Comparative Example 9:

1-[1-((6-chloropyridine-3-yl)methyl)pyridine-2(1H)-yli dene]-3-ethoxycarbonylthiourea (Patent Document 3, Japanese Patent Application Laid-Open No. 5-78323, Table 1, Compound No. 56)

[Chemical Formula 66]

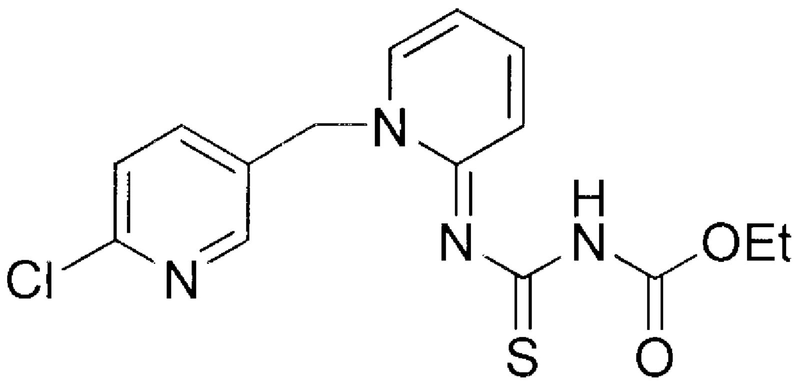

10 ml of acetonitrile was added to 200 mg (0.78 mmol) of 1-[(6-chloropyridin-3-yl)methyl]pyridin-2(1H)-imine hydrochloride synthesized by the method described in Synthetic Example 3, 118 μl (0.86 mmol) of triethylamine and 96 μl (0.82 mmol) of ethoxycarbonyl isothiocyanate were added thereto, and the resulting mixture was heated and refluxed for 1 hour. After the reaction was completed, the reaction solution was returned to room temperature and concentrated under reduced pressure, and liquid separation was performed with ethyl acetate and a saturated sodium bicarbonate water. The organic layer was washed once with water, then dried over anhydrous magnesium sulfate and concentrated under reduced pressure to obtain the subject material. Amount obtained 156 mg (yield 57%). 17 mg of the subject material obtained by purifying 30 mg of the subject material with a TLC plate (one sheet of 0.5 mm plate, evolved twice with hexane: ethyl acetate = 1:3) was provided for the measurement of spectrum data and the biological test.
1H-NMR (CDCl3, δ, ppm) : 1.27 (3H, t), 4.16 (2H, q), 5.52 (2H, s), 6.82 (1H, td), 8.34 (1H, d), 7.72 (2H, m), 7.94 (2H, m), 8.34 (1H, d), 8.46 (1H, d)
MS:m/z= 351 (M+H)
Melting point: 141-143°C

[Preparation Example]

Preparation Example 1 [Granules]

| | |
|---|---|
| Compound | 1-20 5% by weight |
| Bentonite | 40% by weight |
| Talc | 10% by weight |
| Clay | 43% by weight |
| Calcium ligninsulfonate | 2% by weight |

The ingredients were homogeneously ground and mixed, water was added thereto to knead the ingredients thoroughly, and then the mixture was granulated and dried to obtain granules.

Preparation Example 2 [Flowables]

| | |
|---|---|
| Compound 1-21 | 25% by weight |
| POE polystyrylphenyl ether sulfate | 5% by weight |
| Propylene glycol | 6% by weight |
| Bentonite | 1% by weight |
| 1% xanthan-gum aqueous solution | 3% by weight |
| PRONALEX-300 (TOHO Chemical Industry Co., Ltd.) | 0.05% by weight |
| ADDAC827 (KI Chemical Industry Co., Ltd.) | 0.02% by weight |
| Water | added to 100% by weight |

All the ingredients except for the 1% xanthan gum aqueous solution and a suitable amount of water were premixed together from the blending, and the mixture was then ground by a wet grinder. Thereafter, the 1% xanthan gum aqueous solution and the remaining water were added thereto to obtain 100% by weight of flowables.

Preparation Example 3 [Dust]

| | |
|---|---|
| Compound 1-20 | 2% by weight |
| Clay | 60% by weight |
| Talc | 37% by weight |
| Calcium stearate | 1% by weight |

The ingredients were homogeneously mixed to obtain dust.

Preparation Example 4 [Liquid drops]

| | |
|---|---|
| Compound 1-20 | 10% by weight |
| Benzyl alcohol | 74.9% by weight |
| Propylene carbonate | 15% by weight |
| BHT | 0.1% by weight |

The ingredients were homogeneously stirred and dissolved to obtain liquid drops.

Preparation Example 5 [Granules]

| | |
|---|---|
| Compound 1-20 | 2% by weight |
| Probenazole | 24% by weight |
| Binder | 3.0% by weight |
| Guanular improving agent | 0.5% by weight |
| Clay | 70.5% by weight |

The ingredients were homogeneously ground and mixed, water was added thereto to knead the ingredients thoroughly, and then the mixture was granulated and dried to obtain granules.

[Test Example]

Test Example 1 Plutella xylostella control test

A leaf disk having a diameter of 5.0 cm was cut out from a cabbage in pot culture, and a drug solution of the compound of the present invention at a predetermined concentration, which had been prepared so as to be a 50% acetone water (0.05% Tween20 available), was sprayed to the leaf disk. After an air drying process, second instar larvae were released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. Test in duplicate.

$$\text{mortality larvae (\%)} = \{\text{number of dead larvae} / (\text{number of survived larvae} + \text{number of dead larvae})\} \times 100$$

As a result, compounds 1-21, 3-20 and 4-20, exhibited insecticidal activity having a mortality of 80% or higher by a foliar treatment at 500 ppm.

Further, compounds 1-20, 1-21, 4-20,1-22, 1-23, 5-20 and 2-20, exhibited insecticidal activity having a mortality of 80% or higher by a foliar treatment at 100 ppm.

Meanwhile, Comparative Example 8 (Japanese Patent Application Laid-Open No. 5-78323, Compound No. 51 in Table 1) exhibited a mortality of 20% in the treatment at 500 ppm.

Test Example 2 Pest control test against Spodoptera litura

A leaf disk having a diameter of 5.0 cm was cut out from a cabbage in pot culture, and a drug solution of the compound of the present invention at a predetermined concentration, which had been prepared so as to be a 50% acetone water (0.05% Tween20 available), was sprayed to the leaf disk. After an air drying process, third instar larvae were released thereto. Thereafter, the larvae were left to stand in a thermostatic chamber (16 hours of light period-8 hours of dark period) at 25°C. Three days after the release, the larvae were observed for survival or death, and the

mortality of larvae was calculated by the following equation. Test in duplicate.

$$\text{Mortality of larvae (\%)} = \{\text{number of dead larvae} / (\text{number of survived larvae} + \text{number of dead larvae})\} \times 100$$

As a result, compounds 1-21, 3-20 and 4-20 exhibited insecticidal activity having a mortality of 80% or higher by a foliar treatment at 500 ppm. Further, compound 1-20 exhibited insecticidal activity having a mortality of 80% or higher by a foliar treatment at 100 ppm.

Meanwhile, Comparative Example 8 (Japanese Patent Application Laid-Open No. 5-78323, Compound No. 51 in Table 1) and Comparative Example 9 (the same Compound 56) exhibited a mortality of 10% and 11% in the treatment at 500 ppm, respectively.

Test Example 3 Pest control test of Aphis gossypii

A leaf disk having a diameter of 2.0 cm was cut out from a cucumber in pot culture, and a drug solution of the compound of the present invention at a predetermined concentration, which had been prepared so as to be a 50% acetone water (0.05% Tween20 available), was sprayed to the leaf disk. After an air drying process, first instar larvae were released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. Test in duplicate.

$$\text{Mortality of larvae (\%)} = \{\text{number of dead larvae} / (\text{number of survived larvae} + \text{number of dead larvae})\} \times 100$$

As a result, compound 11-20 exhibited insecticidal activity having a mortality of 80% or higher by a foliar treatment at 500 ppm.

Further, compounds 1-20, 1-21, 3-20, 4-20, 2-20, 10-20, 11-20, 1-37, 1-40, and 1-35, exhibited insecticidal activity having a mortality of 80% or higher by a foliar treatment at 100 ppm.

Further, compounds 1-20, 1-21, 3-20, and 1-37, exhibited insecticidal activity having a mortality of 80% or higher by a foliar treatment at 20 ppm.

Test Example 4 Pest control test of Aulacophora femoralis

A leaf disk having a diameter of 2.8cm was cut out from a cucumber in pot culture, and a drug solution of the compound of the present invention at a predetermined concentration, which had been prepared so as to be a 50% acetone water (0.05% Tween20 available), was sprayed to the leaf disk. After an air drying process, adults were released thereto. Thereafter, the adults were left to stand in a thermostatic chamber (16 hours of light period-8 hours of dark period) at 25°C. Three days after the release, the adults were observed for survival or death, and the mortality of adults was calculated by the following equation. Test in duplicate.

$$\text{Mortality of adults (\%)} = \{\text{number of dead adults} / (\text{number of survived adults} + \text{number of dead adults})\} \times 100$$

As a result, compounds 1-20 and 4-20 exhibited insecticidal activity having a mortality of 80% or higher by a foliar treatment at 10 ppm.

Test Example 5 Pest control test against Callosobruchus chinensis

1 μL (/head) of a drug solution of the compound of the present invention prepared at a predetermined concentration with acetone was treated to the back of Callosobruchus chinensis adults. After the drug treatment, the adults were transferred to a plastic cup and left to stand in a thermostatic chamber at 25°C. Twenty four hours after the treatment, the adults were observed for survival or death, and the mortality of adults was calculated by the following equation.

$$\text{Rate of agonized adults (\%)} = \{\text{number of dead adults} / (\text{number of survived adults} + \text{number of dead adults})\} \times 100$$

As a result, compound 1-20 exhibited insecticidal activity having a mortality of 80% or higher in the rate at 0.1 μg/head.

Test Example 6 Pest control test of Laodelphax striatella

A drug solution of the compound of the present invention at a predetermined concentration, which had been prepared so as to be a 50% acetone water (0.05% Tween20 available), was foliar sprayed to a rice seedling in pot culture. After an air drying process, second instar larvae were released thereto. Thereafter, the larvae were left to stand in a thermostatic chamber (16 hours of light period-8 hours of dark period) at 25°C. Six days after the release, the larvae were observed for survival or death, and the mortality of larvae was calculated by the following equation. Test in duplicate.

$$\text{Mortality of larvae (\%)} = \{\text{number of dead larvae} / (\text{number of survived larvae} + \text{number of dead larvae})\} \times 100$$

As a result, compounds 1-20 and 4-20 exhibited insecticidal activity having a mortality of 80% or higher by a foliar treatment at 1.25 ppm.

Test Example 7 Pest control test of Frankliniella occidentalis

A leaf disk having a diameter of 2.8 cm was cut out from a kidney bean in pot culture, and a drug solution of the compound of the present invention at a predetermined concentration, which had been prepared so as to be a 50% acetone water (0.05% Tween20 available), was sprayed to the leaf disk. After an air drying process, first instar larvae were released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. Test in duplicate.

$$\text{Mortality of larvae (\%)} = \{\text{number of dead larvae} / (\text{number of survived larvae} + \text{number of dead larvae})\} \times 100$$

As a result, compounds 1-20, 1-21, 3-20 and 4-20 exhibited insecticidal activity having a mortality of 80% or higher by a foliar treatment at 500 ppm.

Further, compounds 1-20 and 1-21 exhibited insecticidal activity having a mortality of 80% or higher by a foliar treatment at 100 ppm.

Meanwhile, Comparative Example 8 (Japanese Patent Application Laid-Open No. 5-78323, Compound No. 51 in Table 1) exhibited a mortality of 15% in the treatment at 500 ppm.

Test Example 8 Pest control test of Trigonotylus caelestialium

Wheat seedling leaves and stems four days after the dissemination of seedlings were dipped for 30 seconds in a drug solution of the compound of the present invention at a predetermined concentration, which had been prepared so as to be a 50% acetone water (0.05% Tween20 available). After an air drying process, the wheat seedling leaves and stems were placed into a glass tube, and two second instar larvae of Trigonotylus caelestialium were released to the same glass tube. After the larvae were released, the tube was lidded to leave the larvae to stand in a thermostatic chamber at 25°C. In order to supply water to the wheat during the test, water was given to the wheat from the bottom of the glass tube. Three days after the treatment, the larvae were observed for survival or death, and the mortality of larvae was calculated by the following equation.

$$\text{Mortality of larvae (\%)} = \{\text{number of dead larvae} / (\text{number of survived larvae} + \text{number of dead larvae})\} \times 100$$

As a result, compounds 1-20, 1-21, 3-20 and 4-20 exhibited insecticidal activity having a mortality of 80% or higher by a dipping treatment of the drug solution at 50 ppm.

Further, compounds 1-20, 1-21, 3-20, 4-20, 1-22 and 1-23 exhibited insecticidal activity having a mortality of 80% or higher by a dipping treatment of the drug solution at 10 ppm.

Test Example 9 Pest control test of Laodelphax striatella

Wheat seedling roots forty eight hours after the dissemination of seeds were treated with a drug solution of the compound of the present invention at a predetermined concentration, which had been prepared so as to be a 10% acetone water. The drug was absorbed from the roots for 72 hours, and then ten second instar larvae of Laodelphax striatella were each released thereto. Thereafter, the larvae were left to stand in a thermostatic chamber (16 hours of light period-8 hours of dark period) at 25°C. Seven days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

As a result, compounds 1-20, 1-21, 3-20, 4-20 and 2-20 exhibited high insecticidal activity having a mortality of 80% or higher in the rate of 20 μg/seedling.

Further, compounds 1-20, 1-21, 3-20, 4-20, 1-22, 1-23 and 5-20 exhibited insecticidal activity having a mortality of 80% or higher in the rate of 2 μg/seedling.

Meanwhile, Comparative Example 8 (Japanese Patent Application Laid-Open No. 5-78323, Compound No. 51 in Table 1) exhibited a mortality of 50% in the rate of 20 μg/seedling.

<Soil Irrigation Treatment Test>

Test Example 10 Pest control test of Laodelphax striatellua

A rice seedling in pot culture was subjected to soil irrigation treatment with a drug solution of the compound of the present invention at a predetermined concentration, which had been prepared so as to be a 10% acetone water. Three days after the treatment, ten second instar larvae of Laodelphax striatella were each released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

As a result, compounds 1-20, 1-21, 3-20, 4-20, 1-22, 1-23 and 5-20 exhibited high insecticidal activity having a mortality of 80% or higher in the rate of 0.05 mg/seedling.

Meanwhile, Comparative Example 8 (Patent Document 3 Japanese Patent Application Laid-Open No. 5-78323, Compound No. 51 in Table 1) and Comparative Example 9 (the same Compound No. 56) all exhibited a mortality of 0% in the treatment of 0.05 mg/seedling.

Test Example 11 Pest control test of Sogatella furcifera

A rice seedling in pot culture was subjected to soil irrigation treatment with a drug solution of the compound of the present invention at a predetermined concentration, which had been prepared so as to be a 10% acetone water. Three days after the treatment, ten second instar larvae of Sogatella furcifera were each released thereto. Thereafter,

the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

As a result, compounds 1-20 and 4-20 exhibited insecticidal activity having a mortality of 80% or higher in the rate of 0.01 mg/seedling.

Test Example 12 Pest control test of Nilaparvata lugens

A rice seedling in pot culture was subjected to soil irrigation treatment with a drug solution of the compound of the present invention at a predetermined concentration, which had been prepared so as to be a 10% acetone water. Three days after the treatment, ten second instar larvae of Nilaparvata lugens were each released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

As a result, compounds 1-20, 1-21, 3-20, 4-20, 1-23 and 5-20 exhibited insecticidal activity having a mortality of 80% or higher in the rate of 0.05 mg/seedling.
Meanwhile, Comparative Example 8 (Patent Document 3 Japanese Patent Application Laid-Open No. 5-78323, Compound No. 51 in Table 1) and Comparative Example 9 (the same Compound 56) exhibited a mortality of 15% and 0 in the treatment of 0.05 mg/seedling, respectively.

Test Example 13 Pest control test of Oulema oryzae

A rice seedling in pot culture was subjected to soil irrigation treatment with a drug solution of the compound of the present invention at a predetermined concentration, which had been prepared so as to be a 10% acetone water. Three days after the treatment, two second instar larvae of Oulema oryzae were each released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

As a result, compound 1-20 exhibited insecticidal activity having a mortality of 80% or higher in the rate of 0.05mg/seedling.

Test Example 14 Pest control test of Nephotettix cincticeps

A rice seedling in pot culture was subjected to soil irrigation treatment with a drug solution of the compound of the present invention at a predetermined concentration, which had been prepared so as to be a 10% acetone water. Three days after the treatment, five second instar larvae of Nephotettix cincticeps were each released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

Mortality of larvae (%) = {number of dead larvae / (number of survived larvae + number of dead larvae)} × 100

As a result, compound 1-20 exhibited insecticidal activity having a mortality of 80% or higher in the rate of 0.05 mg/seedling.

Effects against drug resistant pests

<Foliar Spray Test>

Test Example 15 Pest control test of Laodelphax striatella

A drug solution of the compound of the present invention at a predetermined concentration, which had been prepared so as to be a 50% acetone water (0.05% Tween20 available), was foliar sprayed to a rice seedling in pot culture. After an air drying process, ten second instar larvae of Laodelphax striatella exhibiting drug resistance to fipronil were released thereto. Thereafter, the larvae were left to stand in a thermostatic chamber (16 hours of light period-8 hours of dark period) at 25°C. Six days after the release, the larvae were observed for survival or death, and the mortality of larvae was calculated by the following equation. Test in duplicate.

Mortality of larvae (%) = {number of dead larvae / (number of survived larvae + number of dead larvae)} × 100

Further, for the origin of test pests, insects of Laodelphax striatella collected outdoors within the Kumamoto prefecture in 2006 were used.
As a result, compound 1-20 exhibited insecticidal activity having a mortality of 80% or higher by a foliar treatment at 1.25 ppm.

Test Example 16 Pest control test of Nilaparvata lugens

A drug solution of the compound of the present invention at a predetermined concentration, which had been prepared so as to be a 50% acetone water (0.05% Tween20 available), was foliar sprayed to a rice seedling in pot culture. After an air drying process, second instar larvae of Nilaparvata lugens exhibiting drug resistance to imidacloprid were released thereto. Thereafter, the larvae were left to stand in a thermostatic chamber (16 hours of light period-8 hours of dark period) at 25°C. Six days after the release, the larvae were observed for survival or death, and the mortality of larvae was calculated by the following equation. Test in duplicate.

Mortality of larvae (%) = {number of dead larvae / (number of survived larvae + number of dead larvae)} × 100

Further, for the origin of test pests, insects of Nilaparvata lugens collected outdoors within the Fukuoka prefecture in 2005 were used.
As a result, compound 1-20 exhibited insecticidal activity having a mortality of 80% or higher by a foliar treatment at 20 ppm.

Test Example 17 Pest control test of Sogatella furcifera

A drug solution of the compound of the present invention at a predetermined concentration, which had been prepared so as to be a 10% acetone water (0.05% Tween20 available), was foliar sprayed to a rice seedling in pot culture. After an air drying process, second instar larvae of Sogatella furcifera exhibiting drug resistance to fipronil were released thereto. Thereafter, the larvae were left to stand in a thermostatic chamber (16 hours of light period-8 hours of dark period) at 25°C. Four days after the release, the larvae were observed for survival or death, the mortality of larvae was calculated by the following equation. Test in duplicate.

$$\text{Mortality of larvae (\%)} = \{\text{number of dead larvae} / (\text{number of survived larvae} + \text{number of dead larvae})\} \times 100$$

Further, for the origin of test pests, insects of Sogatella furcifera collected outdoors within the city of Odawara in 2010 were used.

As a result, compound 1-20 exhibited insecticidal activity having a mortality of 80% or higher by a foliar treatment at 20 ppm.

<Soil Irrigation Test>

Test Example 18 Pest control test of drug-resistant Nilaparvata lugens

A rice seedling in pot culture was subjected to soil irrigation treatment with a drug solution of the compound of the present invention at a predetermined concentration, which had been prepared so as to be a 10% acetone water. Three days after the treatment, ten second instar larvae of Nilaparvata lugens exhibiting drug resistance to imidacloprid were each released thereto. Thereafter, the larvae were left to stand in a thermostatic chamber (16 hours of light period-8 hours of dark period) at 25°C. Three days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

Further, for comparison, the test against some population of Nilaparvata lugens which is highly susceptible to imidacloprid was performed by the same method as described above, and the results thereof are shown in Table 43. As described in Table 43, compounds 1-20, 1-21, 1-22, 1-23, 3-20, 4-20 and 5-20 exhibited equivalent mortality of larvae against drug resistant populations and susceptible populations of Nilaparvata lugens. From the test, it became obvious that 1-20, 1-21, 1-22, 1-23, 3-20, 4-20 and 5-20 exhibited high insecticidal effects even against drug resistant Nilaparvata lugens.

Further, for the origin of test pests, bugs collected outdoors within the Kumamoto prefecture in 2007 as the drug resistant Nilaparvata lugens, and bugs collected within the Kagoshima prefecture and then successively reared indoors for a long time as the imidacloprid susceptible populations of Nilaparvata lugens were used.

[Table 54-1]

| Compounds | Rate (mg/pot) | Insecticidal effects against Nilaparvata lugens (mortality of larvae %) | |
|---|---|---|---|
| | | Susceptible populations | Drug resistant populations |
| | | three days after the treatment | three days after the treatment |
| 1-20 | 0.05 | 96 | 100 |
| | 0.01 | 95 | 81 |
| 1-21 | 0.05 | 67 | 50 |
| 1-22 | 0.05 | 75 | 60 |
| 1-23 | 0.05 | 85 | 70 |
| 3-20 | 0.05 | 100 | 100 |
| 4-20 | 0.05 | 95 | 100 |
| 5-20 | 0.05 | 91 | 100 |
| Comparative Example 3 (Patent Document 2 Example 4) | 0.05 | | 45 |
| Comparative Example 4 (Patent Document 2 Example 3) | 0.05 | | 25 |

(continued)

| Compounds | Rate (mg/pot) | Insecticidal effects against Nilaparvata lugens (mortality of larvae %) | |
|---|---|---|---|
| | | Susceptible populations | Drug resistant populations |
| | | three days after the treatment | three days after the treatment |
| Comparative Example 5 (Patent Document 2 Example 5) | 0.05 | | 25 |
| Comparative Example 6 (Patent Document 2 Example 7) | 0.05 | | 20 |
| Comparative Example 8 (Patent Document 3 Compound 51) | 0.05 | 15 | 13 |

[Table 54-2]

| | | | |
|---|---|---|---|
| Comparative Example 9 (Patent Document 3 Compound 56) | 0.05 | 0 | 31 |
| Imidacloprid | 0.05<br>0.01 | 90<br>73 | 6<br>0 |

Test Example 19 Pest control test of Sogatella furcifera

A rice seedling in pot culture was subjected to soil irrigation treatment with a drug solution of the compound of the present invention at a predetermined concentration, which had been prepared so as to be a 10% acetone water. Three days after the treatment, ten second instar larvae of Sogatella furcifera exhibiting drug resistance to fipronil were each released thereto. Thereafter, the larvae were left to stand in a thermostatic chamber (16 hours of light period-8 hours of dark period) at 25°C. Six days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

Further, for comparison, the test against a populations of Sogatella furcifera which is highly susceptible to fipronil was performed by the same method as described above, and the results thereof are shown in Table 44. As shown in Table 44, compound 1-20 exhibited equivalent mortality of larvae against drug resistant populations and susceptible populations of Sogatella furcifera. From the test, it became obvious that compound 1-20 exhibited high insecticidal effects even against drug resistant Sogatella furcifera.

In addition, for the origin of test pests, bugs collected within the city of Odawara in 2010 as the drug resistant Sogatella furcifera, and bugs collected within the city of Chigasaki in 1970 and then successively reared indoors for a long time as the susceptible populations of Sogatella furcifera were used.

[Table 55]

| Compounds | Rate (mg/po t) | Insecticidal effects against Sogatella furcifera (mortality of larvae %) | |
|---|---|---|---|
| | | Susceptible populations | Drug resistant populations |
| | | six days after the treatment | six days after the treatment |
| 1-20 | 0.01<br>0.005 | 88<br>75 | 88<br>53 |

(continued)

| Compounds | Rate (mg/po t) | Insecticidal effects against Sogatella furcifera (mortality of larvae %) | |
|---|---|---|---|
| | | Susceptible populations | Drug resistant populations |
| | | six days after the treatment | six days after the treatment |
| Fipronil | 0.05 | | 90 |
| | 0.01 | 100 | 40 |
| | 0.005 | 100 | |
| | 0.01 | 78 | |

Test Example 20 Pest control test of Laodelphax striatella

A rice seedling in pot culture was subjected to soil irrigation treatment with a drug solution of the compound of the present invention at a predetermined concentration, which had been prepared so as to be a 10% acetone water. Three days after the treatment, ten second instar larvae of Laodelphax striatella exhibiting drug resistance to fipronil were each released thereto. Thereafter, the larvae were left to stand in a thermostatic chamber (16 hours of light period-8 hours of dark period) at 25°C. Six days after the release, the larvae were observed for survival or death, and the mortality of larvae was calculated by the following equation. The test was performed in duplicate.

$$\text{Mortality of larvae (\%)} = \{\text{number of dead larvae} / (\text{number of survived larvae} + \text{number of dead larvae})\} \times 100$$

Further, for comparison, the test against a populations of Laodelphax striatella which is highly susceptible to fipronil was performed by the same method as described above, and the results thereof are shown in Table 45. As shown in Table 45, compound 1-20 exhibited equivalent mortality of larvae against drug resistant poulations and susceptible poulations of Laodelphax striatella. From the test, it became obvious that compound 1-20 exhibited high insecticidal effects even against drug resistant Laodelphax striatella.

In addition, for the origin of test pests, bugs collected within the Kumamoto prefecture in 2006 as the drug resistant Laodelphax striatella, and bugs successively reared indoors for a long time as the susceptible poulations of Laodelphax striatella were used.

[Table 56]

| Compounds | Rate (mg/po t) | Insecticidal effects against Laodelphax striatella (mortality of larvae %) | |
|---|---|---|---|
| | | Susceptible populations | Drug resistant populations |
| | | six days after the treatment | six days after the treatment |
| 1-20 | 0.005 | 85 | 79 |
| Fipronil | 0.05 | | 90 |
| | 0.02 | | 76 |
| | 0.01 | 100 | |
| | 0.005 | 89 | |

Test Example 21 Pest control test of Musca domestica

A drug solution of the compound of the present invention, which had been adjusted to be 50 ppm with a 40% sucrose liquid, was adsorbed on a pledget, and the pledget was put into a vial. Two adults reared indoors were released thereto. Thereafter, the adults were left to stand indoors at room temperature of 25°C. Four days after the release, the adults were observed for survival or death. When two adults were all agonized in death, it was judged to be effective.

As a result, compounds 1-20, 1-21, 3-20, 4-20, 1-22, 1-23, 5-20 and 2-20 exhibited high activity that all the adults are agonized in death, in the rate at 50 ppm.

Test Example 22 Pest control test of housefly instar larvae

Compound 1-20 was blended with an extremely small amount of DMSO, and then the resulting mixture was dissolved in deionized water to adjust the drug solution. 10 ml of the drug solution adjusted to be 30 ppm was added to 10 g of powder in which wheat bran, MF feed (Oriental Yeast Co., Ltd.) and dry yeast had been mixed in a ratio of 25:5:1, and the mixture was mixed well to prepare a bait for housefly instar larvae. A 50 ml Falcon tube was slightly filled with the bait including the compound, and 20 eggs were released thereto. The mouth of the Falcon tube was covered with a mesh-topped lid, and the tube was allowed to stand at 25°C. Twenty days after the drug treatment, the numbers of instar larvae, chrysalises and adults were measured, and the mortality of larvae, chrysalises and adults was calculated by the following equation. The test was performed by repeating each treatment twice.

Mortality of larvae, chrysalises and adults (%) = {number of dead larvae, chrysalises and adults / (number of survived larvae, chrysalises and adults + number of dead larvae, chrysalises and adults)} × 100

As a result, compound 1-20 exhibited a mortality of 100% in the rate at 30 ppm.

Test Example 23 Pest control test of Haemaphysalis longicornis

30 μL of a acetone solution of the compound of the present invention at 200 ppm and acetone at 10 ppm was put into a 4 mL glass vial. The glass vial was loaded into a shaker and blow-dried while being rotated to form a dry film of the compound on the internal wall of the vial. After the vial was dried for 24 hours or more, ten young mites of Haemaphysalis longicornis were released thereto and the lid of the vial was covered. The vial was allowed to stand in a thermostatic chamber under total dark conditions at 25°C and the humidity of 85%. One day after the release, the insects were observed for survival or death, the mortality of insects was calculated by the following equation. The test was performed in duplicate.

Mortality of insects (%) = {number of dead insects / (number of survived insects + number of dead insects)} × 100

As a result, compounds 1-21, 1-22, 1-23, 5-20 and 2-20 exhibited insecticidal activity having a mortality of 80% or higher in the rate at 200 ppm.

Test Example 24 Pest control test of Haemaphysalis longicornis

A capsule with a diameter of 2 cm and a height of 2 cm was adhered to the back of a mouse. 9.5 μg of the compound of the present invention was dissolved in ethanol, and the resulting mixture was added dropwise to the body surface of the mouse in the capsule. The capsule was sufficiently dried, then ten young mites of Haemaphysalis longicornis were released thereto, and the top of the capsule was hermetically sealed with a lid. The mouse was reared under conditions of 12 hours of light period and 12 hours of dark period at 25°C in a cage. Five days after the release, the capsule was detached therefrom to measure the numbers of living and dead mites and blood-sucking individuals of young mites, and the rate of insects agonized in death was calculated according to the following equation.

Rate of insects agonized in death (%) = {number of insects agonized in death / (number of survived insects + number of insects agonized in death)} × 100

As a result, compounds 1-20, and 4-20, exhibited insecticidal activity having a rate of insects agonized in death of 80% or higher in the 9.5 μg of rate.

Test Example 25 Pest control test of Haemaphysalis longicornis

Three petri dishes with a diameter of 9 cm and a height of 1 cm were adhered to the back of a dog. Compound 1-20 of the present invention was dissolved in ethanol so as to be 5.35 mg/mL, and the mixture was added dropwise to the body surface of the dog in the petri dish. The petri dish was sufficiently dried, and then thirty young mites of Haemaphysalis longicornis were released thereto. Each dog was housed in a cage and reared under conditions of 10 hours of light period and 14 hours of dark period at 23°C. Three days after the release, the petri dishes were detached therefrom to measure the numbers of living and dead mites and blood-sucking individuals of young mites, and the mortality of mites was calculated according to the following equation.

$$\text{Mortality of mites (\%)} = \{\text{number of dead mites} / (\text{number of survived mites} + \text{number of dead mites})\} \times 100$$

As a result, compound 1-20 exhibited a mortality of 100%.

Test Example 26 Pest control test of Ctenocephalides felis

Three petri dishes with a diameter of 9 cm and a height of 1 cm were adhered to the back of a dog. Compound 1-20 of the present invention was dissolved in ethanol so as to be 5.35 mg/mL, and the mixture was added dropwise to the body surface of the dog in the petri dish. The petri dish was sufficiently dried, and then twenty insects of Ctenocephalides felis were released thereto. Each dog was housed in a cage and reared under conditions of 10 hours of light period and 14 hours of dark period at 23°C. Three days after the release, the petri dishes were detached therefrom to measure the numbers of living and dead insects of Ctenocephalides felis and blood-sucking individuals, and the mortality of insects was calculated according to the following equation.

$$\text{Mortality of insects (\%)} = \{\text{number of dead insects} / (\text{number of survived insects} + \text{number of dead insects})\} \times 100$$

As a result, compound 1-20 exhibited a mortality of 100%.

Test Example 27 Pest control test of Coptotermes formosanus

A filter paper was allowed to be soaked in a drug solution of the compound of the present invention at a predetermined concentration, which had been prepared with acetone. The filter paper was sufficiently blow-dried and then put into a plastic petri dish, and 5 insects of Coptotermes formosanus (worker ant) were each released thereto. Thereafter, the insects were left to stand in a thermostatic chamber (16 hours of light period-8 hours of dark period) at 25°C. Seven days after the release, the insects were observed for survival or death, and the mortality of insects was calculated by the following equation. The test was performed in duplicate.

$$\text{Mortality of insects (\%)} = \{\text{number of dead insects} / (\text{number of survived insects} + \text{number of dead insects})\} \times 100$$

As a result, compound 1-20 exhibited insecticidal activity having a mortality of 100% in the rate at 0.5 $\mu g/cm^2$.

The biological activities of the preferred compounds of this invention were dicribed in Tables 57 and 58.

[Table 57-1]

| (foliar treatment) | | | | | | |
|---|---|---|---|---|---|---|
| | | Compound 1-20 | Compound 1-21 | Compound 3-20 | Compound 4-20 | Compound 5-20 |
| Test Example | Concentration (ppm) | % Mortality | % Mortality | % Mortality | % Mortality | % Mortality |
| Test Example1 Plutella xylostella | 100 | 100 | 80 | 55 | 100 | 100 |
| | 20 | 100 | 10 | 0 | 70 | 100 |
| | 5 | 50 | | | | 20 |
| Test Example2 Spodoptera litura | 100 | 90 | 55 | 30 | 20 | 60 |
| Test Example3 Aphis gossypii | 100 | 100 | 100 | 100 | 100 | |
| | 20 | 100 | 100 | 100 | | |
| | 5 | 100 | 100 | 100 | 100 | 100 |
| | 1.25 | 75 | 100 | 100 | 100 | 55 |
| | 0.313 | | 55 | 15 | | |
| Test Example4 Aulacophora femoralis | 5 | 100 | | | | |
| | 1.25 | 100 | | | | |
| Test Example5 Callosobruchus chinensis | 100 | 100 | | | | |
| | 10 | 100 | | | | |

[Table 57-2]

| | | | | | | |
|---|---|---|---|---|---|---|
| Test Example6 Laodelphax striatella | 1.25 | 95 | | | | |
| Test Example7 Frankliniella occidentalis | 100 | 80 | 90 | 45 | 60 | 50 |
| | 20 | 50 | 80 | | | |
| Test Example8 Trigonotylus caelestialium | 50 | 100 | 100 | 100 | 100 | |
| | 10 | 100 | 100 | 100 | 100 | 17 |
| | 2 | 50 | 67 | 50 | 33 | |

[Table 58]

| soil irrigation treatment | | | | | | |
|---|---|---|---|---|---|---|
| | | Compound 1-20 | Compound 1-21 | Compound 3-20 | Compound 4-20 | Compound 5-20 |
| Test Example | Concentration (mg/pot) | % Mortality | % Mortality | % Mortality | % Mortality | % Mortality |
| Test Example10 Laodelphax striatella | 0.05 | 100 | 100 | 90 | 100 | 95 |
| | 0.01 | 91 | 20 | | 100 | 52 |
| | 0.005 | 75 | | | 35 | 26 |

(continued)

| soil irrigation treatment | | | | | | |
|---|---|---|---|---|---|---|
| | | Compound 1-20 | Compound 1-21 | Compound 3-20 | Compound 4-20 | Compound 5-20 |
| Test Example | Concentration (mg/pot) | % Mortality | % Mortality | % Mortality | % Mortality | % Mortality |
| Test Example11 Sogatella furcifera | 0.01 | 88 | | | | |
| | 0.005 | 53 | | | | |
| Test Example12 Nilaparvata lugens | 0.05 | 100 | 85 | 100 | 100 | 100 |
| | 0.01 | 85 | 10 | 100 | 96 | 90 |
| | 0.005 | 80 | | | | |
| Test Example14 Nephotettix cincticeps | 0.01 | 100 | | | | |
| | 0.001 | 67 | | | | |
| | 0.0001 | 67 | | | | |

## Claims

1. A pest-control agent containing a nitrogen-containing heterocyclic derivative having a 2-imino group, which is represented by the following Formula (I), or salts thereof

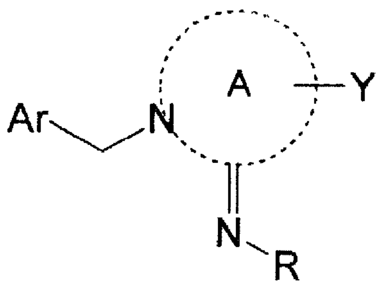

Formula (I),

in the formula Ar represents a phenyl group which may be substituted with any of a halogen atom, a C1 to C4 alkyl group which may be substituted with a halogen atom, a C1 to C4 alkyloxy group which may be substituted with a halogen atom, a hydroxyl group, a cyano group, a nitro group; a 5- to 6-membered heterocycle which may be substituted with any of a halogen atom, a C1 to C4 alkyl group which may be substituted with a halogen atom, a C1 to C4 alkyloxy group which may be substituted with a halogen atom, a hydroxyl group, a cyano group, and a nitro group; or a 4- to 10-membered heterocycloalkyl group,
the ring represented by the following Formula:

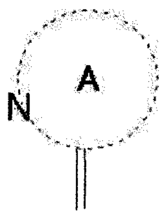

represents any one of rings represented by the following Formulae (A-1) to (A-40) :

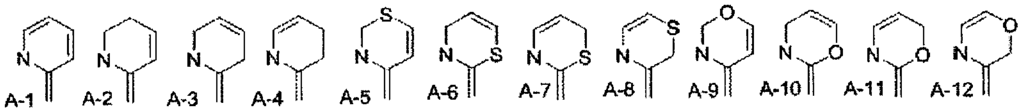

A-1 A-2 A-3 A-4 A-5 A-6 A-7 A-8 A-9 A-10 A-11 A-12

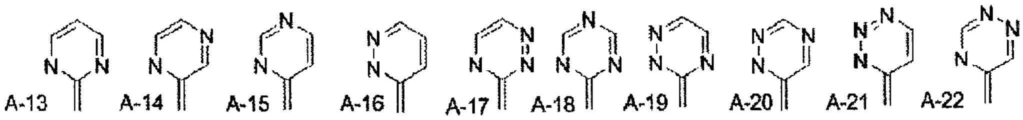

A-13 A-14 A-15 A-16 A-17 A-18 A-19 A-20 A-21 A-22

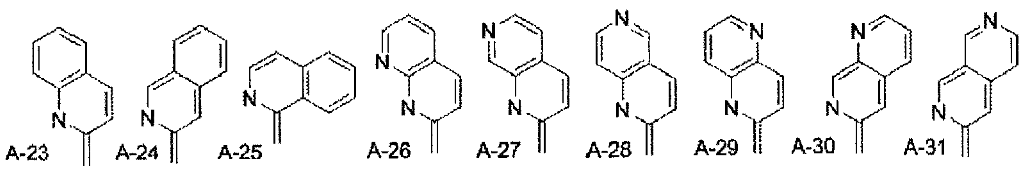

A-23 A-24 A-25 A-26 A-27 A-28 A-29 A-30 A-31

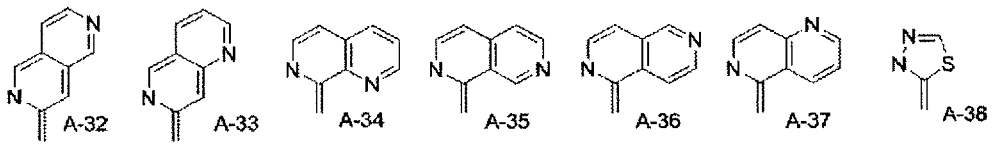

A-32 A-33 A-34 A-35 A-36 A-37 A-38

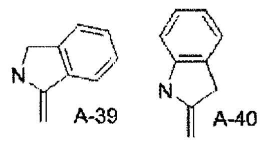

A-39 A-40 ,

Y represents a hydrogen atom, a halogen atom, a hydroxyl group, a C1 to C6 alkyl group which may be substituted with a halogen atom, a C1 to C6 alkyloxy group which may be substituted with a halogen atom, a cyano group, or a nitro group, and
R represents a group represented by the following Formula (c)

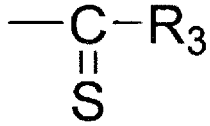

(c)

where R3 represents a C1 to C6 alkyl group which may be substituted with a halogen atom.

**2.** The pest-control agent according to claim 1, wherein

the ring represented by the following Formula:

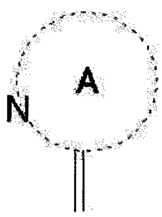

represents any one of rings represented by Formulae (A-1), (A-13) to (A-16), (A-23), (A-25), (A-38), and (A-39).

**3.** The pest-control agent according to claim 1 or 2, wherein Ar in Formula (I) is a 6-chloro-3-pyridyl group, a 6-chloro-5-fluoro-3-pyridyl group, a 6-fluoro-3-pyridyl group, a 6-bromo-3-pyridyl group, a 2-chloro-5-pyrimidyl group, a 2-chloro-5-thiazolyl group, or a 5-chloro-2-pyrazinyl group.

4. The pest-control agent according to any one of claims 1 to 3, wherein

the ring represented by the following Formula:

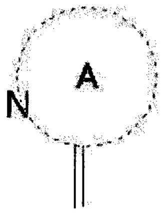

represents a ring represented by Formula (A-1), and Y represents a hydrogen atom, a halogen atom or a cyano group.

5. The pest-control agent according to claim 1, wherein

Ar represents a 6-chloro-3-pyridyl group, a 2-chloro-5-thiazolyl group, a 6-chloro-5-fluoro-3-pyridyl group, a 6-fluoro-3-pyridyl group, a 6-bromo-3-pyridyl group, a 2-chloro-5-pyrimidyl group or a 6-trifluoromethyl-3-pyridyl group,
the ring represented by the following Formula:

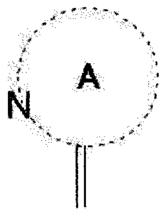

represents a ring represented by Formula (A-1),
Y represents a hydrogen atom, and
R3 represents a trifluoromethyl group, a difluoromethyl group, a chlorodifluoromethyl group or a pentafluoroethyl group.

6. The pest-control agent according to claim 1, wherein
the nitrogen-containing heterocyclic derivative having the 2-imino-group, is N-[1-((6-chloropyridin-3-yl)methyl)pyridin-2(1H)-ylidene]-2,2,2-trifluoroetanethioamide.

7. A method of controlling pest except for the therapeutic treatment of animals by use of the pest control agents according to any one of claims 1 to 6.

8. The method for controlling pests according to claim 7, comprising treating seeds, roots, tubers, bulbs and rhizomes of plants, germinated plants, seedlings, soil, a nutrient solution in nutrient solution culture, or a solid medium in nutrient solution culture with the pest control agent to penetrate and migrate the compound represented by Formula (I) into the plants.

9. The method according to claim 7 or 8, wherein the pest is an agricultural and horticultural pest.

10. The method according to claim 7, wherein the pest is an animal parasitic pest.

11. The method according to any one of claims 7 to 10, wherein the pest is a pest which has drug resistance to imidacloprid or fipronil.

12. A method for preparing a compound represented by Formula (I-3)

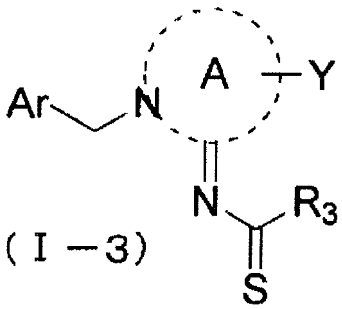

(I-3)

wherein Ar, the ring represented by the following Formula:

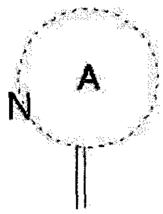

,

Y and R3 have the same meaning as defined for Formula (I) in claim 1,
the method comprises a reaction of converting an oxygen atom in the compound represented by Formula (II-3a)

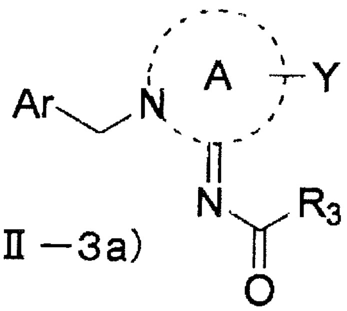

(II-3a)

,

or in the compound represented by Formula (II-3c)

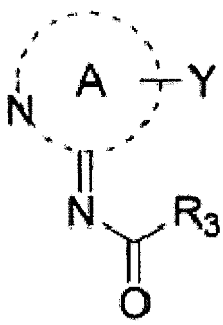

(II-3c)

,

into a sulfur atom,
in said Formulae II-3a and II-3c the ring represented by the following Formula:

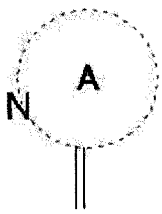
,

Y and R3 have the same meaning as those defined as Formula (I) in claim 1.

**Patentansprüche**

1. Ein Schädlingsbekämpfungsmittel, welches ein Stickstoff enthaltendes heterocyclisches Derivat mit einer 2-Iminogruppe, das von der Formel (I):

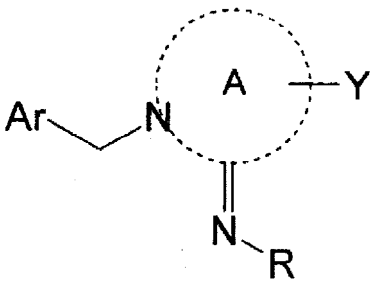

Formel (I)

repräsentiert wird, wobei:

- in der Formel Ar eine Phenylgruppe, die mit einem/ einer von Halogenatom, C1- bis $C_4$-Alkylgruppe, die mit einem Halogenatom substituiert sein kann, oder $C_1$- bis $C_4$-Alkyloxygruppe, die mit einem Halogenatom, einer Hydroxylgruppe, einer Cyanogruppe oder einer Nitrogruppe substituiert sein kann, substituiert sein kann, einen 5- bis 6-gliedrigen Heterocyclus, der mit einem/einer von Halogenatom, $C_1$- bis $C_4$-Alkylgruppe, die mit einem Halogenatom substituiert sein kann, oder $C_1$- bis $C_4$-Alkyloxygruppe, die mit einem Halogenatom, einer Hydroxylgruppe, einer Cyanogruppe und einer Nitrogruppe substituiert sein kann, substituiert sein kann, oder eine 4- bis 10-gliedrige Heterocycloalkylgruppe bedeutet,
- der Ring mit der Formel:

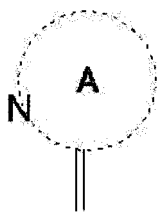

einen der Ringe mit den Formeln (A-1) bis (A-40):

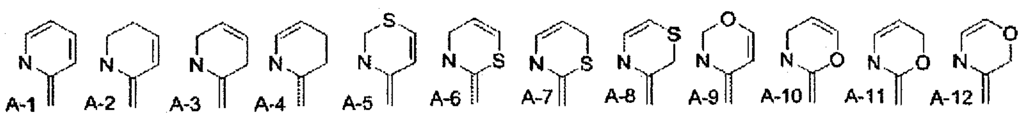
A-1 A-2 A-3 A-4 A-5 A-6 A-7 A-8 A-9 A-10 A-11 A-12

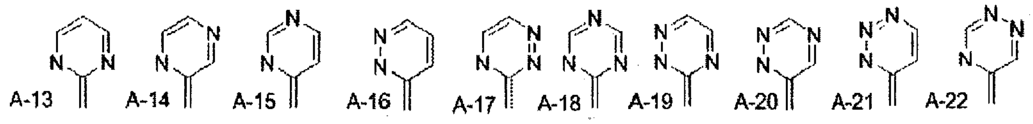
A-13 A-14 A-15 A-16 A-17 A-18 A-19 A-20 A-21 A-22

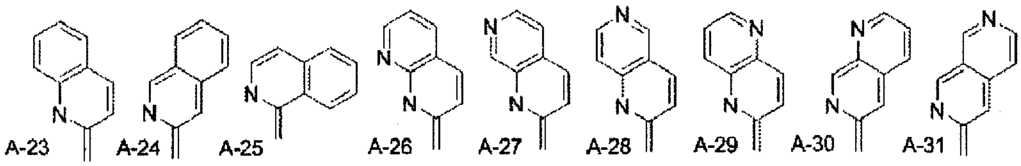
A-23 A-24 A-25 A-26 A-27 A-28 A-29 A-30 A-31

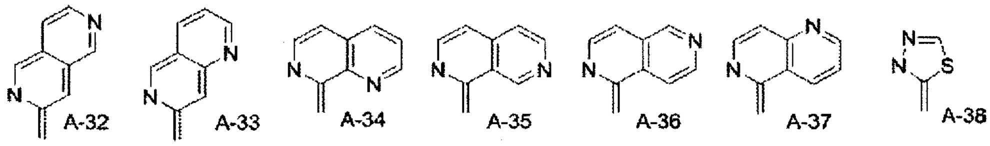
A-32 A-33 A-34 A-35 A-36 A-37 A-38

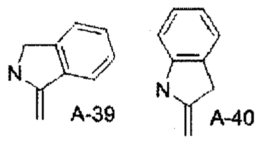
A-39 A-40

bedeutet,

- Y ein Wasserstoffatom, ein Halogenatom, eine Hydroxylgruppe, eine $C_1$- bis $C_6$-Alkylgruppe, die mit einem Halogenatom substituiert sein kann, eine oder $C_1$- bis $C_6$-Alkyloxygruppe, die mit einem Halogenatom, einer Cyanogruppe oder einer Nitrogruppe substituiert sein kann, und
- R eine Gruppe mit der Formel (c):

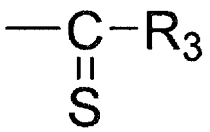
(c),

worin $R_3$ eine $C_1$- bis $C_6$-Alkylgruppe, die mit einem Halogenatom substituiert sein kann, bedeutet, bedeutet,

oder Salze davon enthält.

**2.** Das Schädlingsbekämpfungsmittel nach Anspruch 1, wobei:

- der Ring mit der Formel:

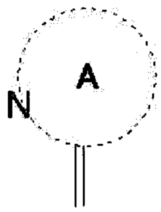

einen der Ringe mit den Formeln (A-1), (A-13) bis (A-16), (A-23), (A-25), (A-38) und (A-39) bedeutet.

**3.** Das Schädlingsbekämpfungsmittel nach Anspruch 1 oder 2, wobei Ar in der Formel (I) eine 6-Chlor-3-pyridylgruppe, eine 6-Chlor-5-fluor-3-pyridylgruppe, eine 6-Fluor-3-pyridylgruppe, eine 6-Brom-3-pyridylgruppe, eine 2-Chlor-5-pyrimidylgruppe, eine 2-Chlor-5-thiazolylgruppe oder eine 5-Chlor-2-pyrazinylgruppe bedeutet.

**4.** Das Schädlingsbekämpfungsmittel nach einem der Ansprüche 1 bis 3, wobei:

- der Ring mit der Formel:

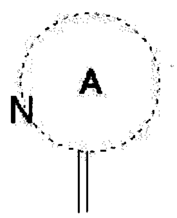

einen Ring mit der Formel (A-1) und Y ein Wasserstoffatom, ein Halogenatom oder eine Cyanogruppe bedeutet.

**5.** Das Schädlingsbekämpfungsmittel nach Anspruch 1, wobei:

- Ar eine 6-Chlor-3-pyridylgruppe, 2-Chlor-5-thiazolylgruppe, 6-Chlor-5-fluor-3-pyridylgruppe, 6-Fluor-3-pyridylgruppe, 6-Brom-3-pyridylgruppe, 2-Chlor-5-pyrimidylgruppe oder 6-Trifluormethyl-3-pyridylgruppe,
- der Ring mit der Formel:

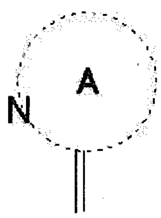

einen Ring mit der Formel (A-1),
- Y ein Wasserstoffatom und
- $R_3$ eine Trifluormethylgruppe, Difluormethylgruppe, Chlordifluormethylgruppe oder Pentafluorethylgruppe

bedeutet.

**6.** Das Schädlingsbekämpfungsmittel nach Anspruch 1, wobei das Stickstoff enthaltende heterocyclische Derivat mit der 2-Iminogruppe N-[1-((6-Chlorpyridin-3-yl)methyl)pyridin-2(1H)-yliden]-2,2,2-trifluorethanthioamid ist.

**7.** Ein Verfahren zur Schädlingsbekämpfung, ausgenommen die therapeutische Behandlung von Tieren, durch die Verwendung der Schädlingsbekämpfungsmittel nach einem der Ansprüche 1 bis 6.

**8.** Das Verfahren zur Schädlingsbekämpfung nach Anspruch 7, welches die Behandlung von Samen, Wurzeln, Knollen, Zwiebeln und Rhizomen von Pflanzen, von gekeimten Pflanzen, Sämlingen, Erde, einer Nährlösung einer Nährlösungskultur oder einem festen Medium in einer Nährlösungskultur mit dem Schädlingsbekämpfungsmittel, um die Verbindung mit der Formel (I) in die Pflanzen eindringen und darin migrieren zu lassen, umfasst.

**9.** Das Verfahren nach Anspruch 7 oder 8, wobei der Schädling ein solcher in der Landwirtschaft und dem Gartenbau ist.

**10.** Das Verfahren nach Anspruch 7, wobei der Schädling ein bei Tieren parasitärer Schädling ist.

**11.** Das Verfahren nach einem der Ansprüche 7 bis 10, wobei der Schädling ein solcher ist, der gegen Imidacloprid oder Fipronil eine Wirkstoffresistenz entwickelt hat.

**12.** Ein Verfahren zur Herstellung einer Verbindung mit der Formel (I-3):

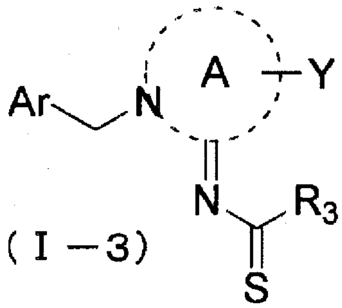

(I-3) ,

worin:

- Ar, der Ring mit der Formel:

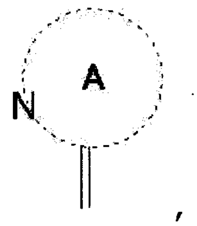

,

- Y und $R_3$ dieselbe Bedeutung wie in Anspruch 1 für Formel (I) definiert haben und
- das Verfahren die Umwandlung eines Sauerstoffatoms der Verbindung mit der Formel (II-3a):

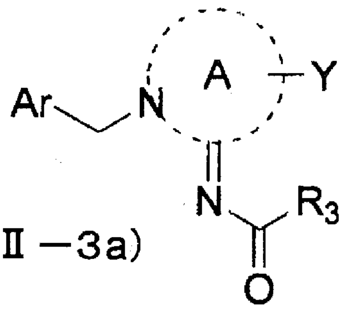

(II-3a)

oder der Verbindung mir der Formel (II-3c):

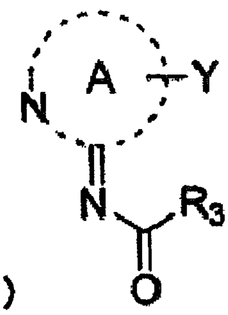

(II-3c)

in ein Schwefelatom umfasst, wobei
in diesen Formeln II-3a und II-3c der Ring mit der Formel:

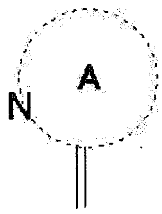

,

Y und $R_3$ dieselbe Bedeutung wie in Anspruch 1 für Formel (I) definiert haben.

## Revendications

1. Agent destiné au contrôle des nuisibles, contenant un dérivé hétérocyclique azoté comprenant un groupe 2-imino, qui est représenté par la formule (I) suivante, ou ses sels

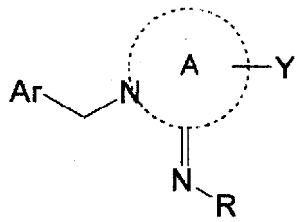

Formule (I)

dans la formule, Ar représente un groupe phényle qui peut être substitué par l'un quelconque parmi un atome d'halogène, un groupe alkyle en C1 à C4 qui peut être substitué par un atome d'halogène, un groupe alcoxy en C1 à C4 qui peut être substitué par un atome d'halogène, le groupe hydroxyle, le groupe cyano, le groupe nitro ; un hétérocycle de 5 ou 6 membres, qui peut être substitué par l'un quelconque parmi un atome d'halogène, un groupe alkyle en C1 à C4 qui peut être substitué par un atome d'halogène, un groupe alcoxy en C1 à C4 qui peut être substitué par un atome d'halogène, le groupe hydroxyle, le groupe cyano, le groupe nitro ; un groupe hétérocycloalkyle de 4 à 10 membres, le cycle représenté par la formule suivante :

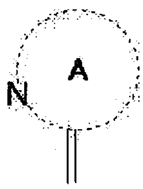

représente l'un quelconque des cycles représentés par les formules (A-1) à (A-40) :

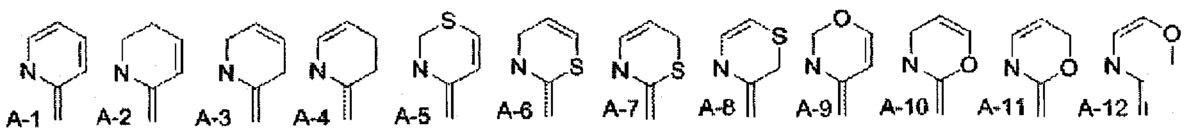

A-1 A-2 A-3 A-4 A-5 A-6 A-7 A-8 A-9 A-10 A-11 A-12

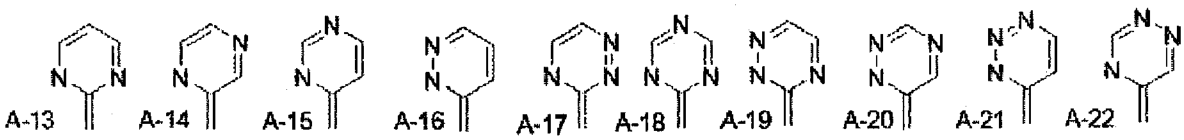

A-13 A-14 A-15 A-16 A-17 A-18 A-19 A-20 A-21 A-22

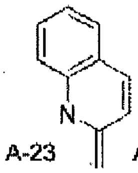 A-23 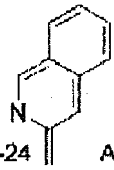 A-24 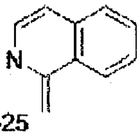 A-25 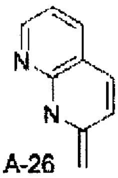 A-26 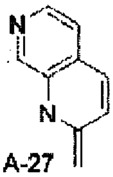 A-27 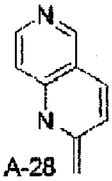 A-28 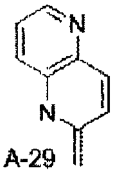 A-29 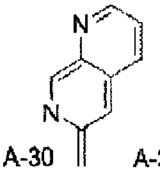 A-30 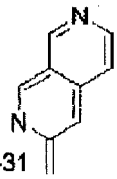 A-31

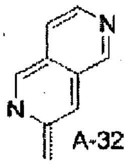 A-32 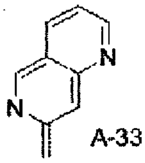 A-33 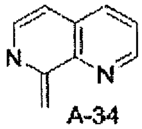 A-34 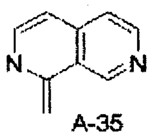 A-35 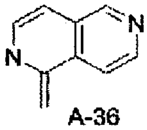 A-36 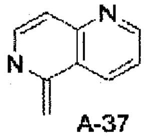 A-37 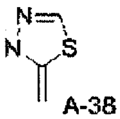 A-38

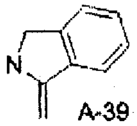 A-39 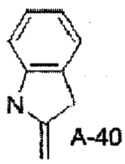 A-40 ,

Y représente l'atome d'hydrogène, un atome d'halogène, le groupe hydroxyle, un groupe alkyle en C1 à C6 qui peut être substitué par un atome d'halogène, un groupe alcoxy en C1 à C6 qui peut être substitué par un atome d'halogène, le groupe cyano ou le groupe nitro, et
R représente un groupe représenté par la formule (C) suivante :

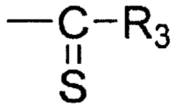 (c)

où R3 représente un groupe alkyle en C1 à C6 qui peut être substitué par un atome d'halogène.

2. Agent destiné au contrôle des nuisibles selon la revendication 1, où le cycle représenté par la formule suivante :

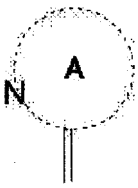

représente l'un des cycles représentés par les formules (A-1), (A-13) à (A-16), (A-23), (A-25), (A-38) et (A-39).

3. Agent destiné au contrôle des nuisibles selon la revendication 1 ou 2, où Ar dans la formule (I) est le groupe 6-chloro-3-pyridyle, le groupe 6-chloro-5-fluoro-3-pyridyle, le groupe 6-fluoro-3-pyridyle, le groupe 6-bromo-3-pyridyle, le groupe 2-chloro-5-pyrimidyle, le groupe 2-chloro-5-thiazolyle, ou le groupe 5-chloro-2-pyrazinyle.

4. Agent destiné au contrôle des nuisibles selon l'une quelconque des revendications 1 à 3, où le cycle représenté par la formule suivante :

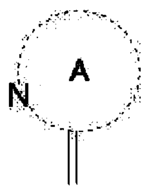

représente un cycle représenté par la formule (A-1) et Y représente l'atome d'hydrogène, un atome d'halogène ou le groupe cyano.

5. Agent destiné au contrôle des nuisibles selon la revendication 1, Ar représente le groupe 6-chloro-3-pyridyle, le groupe 2-chloro-5-thiazolyle, le groupe 6-chloro-5-fluoro-3-pyridyle, le groupe 6-fluoro-3-pyridyle, le groupe 6-bromo-3-pyridyle, le groupe 2-chloro-5-pyrimidyle ou le groupe 6-trifluorométhyl-3-pyridyle,
le cycle représenté par la formule suivante :

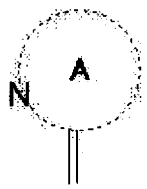

représente un cycle représenté par la formule (A-1),
Y représente l'atome d'hydrogène, et
R3 représente le groupe trifluorométhyle, le groupe difluorométhyle, le groupe chlorodifluorométhyle ou le groupe pentafluoroéthyle.

6. Agent destiné au contrôle des nuisibles selon la revendication 1, où le dérivé hétérocyclique azoté comprenant un groupe 2-imino est le N-[1-((6-chloropyridin-3-yl)méthyl)pyridin-2(1H)-ylidène]-2,2,2-trifluoroéthanethioamide.

7. Procédé pour le contrôle des nuisibles excepté pour le traitement thérapeutique d'animaux, par utilisation des agents destinés au contrôle des nuisibles selon l'une quelconque des revendications 1 à 6.

8. Procédé pour le contrôle des nuisibles selon la revendication 7, comprenant le traitement de graines, de racines, de tubercules, de bulbes et de rhizomes de plantes, de plantes germées, de pousses, du sol, d'une solution nutritive dans une culture de solution nutritive, ou d'un milieu solide en culture de solution nutritive avec l'agent destiné au contrôle des nuisibles, pour faire pénétrer et migrer le composé représenté par la formule (I) dans les plantes.

9. Procédé selon la revendication 7 ou 8, où le nuisible est un nuisible agricole et horticole.

10. Procédé selon la revendication 7, où le nuisible est un nuisible parasitaire animal.

11. Procédé selon l'une quelconque des revendications 7 à 10, où le nuisible est un nuisible qui présente une résistance à l'imidaclopride ou au fipronil.

12. Procédé de préparation d'un composé représenté par la formule (I-3) :

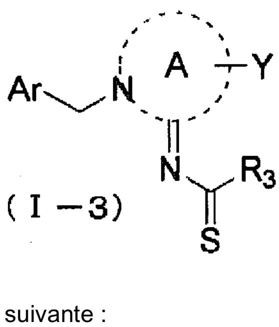

(I −3)

où Ar, le cycle représenté par la formule suivante :

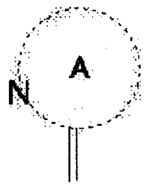

Y et R3 ont la même signification que celle définie pour la formule (I) à la revendication 1,
le procédé comprenant une réaction de conversion d'un atome d'oxygène dans le composé représenté par la formule (II-3a) :

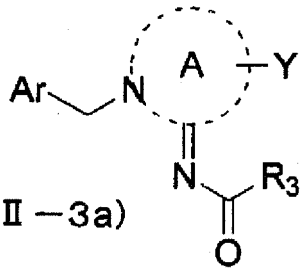

(II-3a) ,

ou dans le composé représenté par la formule (II-3c) :

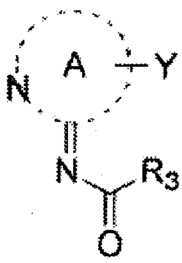

(II-3c) ,

en un atome de soufre,
dans lesdites formules II-3a et II-3c, le cycle représenté par la formule suivante :

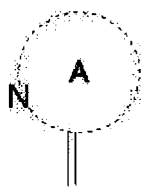

Y et R3 ont la même signification que celle définie pour la formule (I) à la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 432600 A
- EP 268915 A
- JP 5078323 A
- JP 2006051704 A
- EP 259738 A